# EUROPEAN PATENT APPLICATION

(11) **EP 4 279 515 A1**
(43) Date of publication of application: **22.11.2023**
(21) Application number: 22739064.8
(22) Date of filing: 12.01.2022
(51) Int. Cl.: C07K 19/00, A61K 35/17, C12N 5/10, C12N 15/62, A61P 35/02

(54) **FULLY HUMAN SINGLE-DOMAIN TANDEM CHIMERIC ANTIGEN RECEPTOR (CAR) TARGETING CD5, AND USE THEREOF**

(30) Priority: 12.01.2021 CN 202110032708
(71) Applicant: Nanjing Iaso Biotechnology Co., Ltd., Nanjing, Jiangsu 210000 (CN)
(72) Inventor: TAN, Taochao, Nanjing, Jiangsu 210000 (CN); DAI, Zhenyu, Nanjing, Jiangsu 210000 (CN); ZHOU, Jianfeng, Nanjing, Jiangsu 210000 (CN); ZHAO, Ya, Nanjing, Jiangsu 210000 (CN); WEI, Qiaoe, Nanjing, Jiangsu 210000 (CN); JIA, Xiangyin, Nanjing, Jiangsu 210000 (CN); LIU, Jianwei, Nanjing, Jiangsu 210000 (CN)
(74) Representative: Symbiosis IP Limited
(86) International application number: PCT/CN2022/071677
(87) International publication number: WO 2022/152186

(57) **Abstract**

Provided is a fully human single-domain tandem chimeric antigen receptor (CAR) capable of specifically binding to a CD5 protein, which comprises a CDS binding domain, a transmembrane domain, a co-stimulatory domain and an intracellular signaling domain. Also provided are engineered immune effector cells, such as T cells, comprising the chimeric antigen receptor, and use of the CAR and the engineered immune effector cells in the treatment of diseases or conditions associated with the expression of CD5.

## Description

### CROSS-REFERENCE TO RELATED APPLICATION

The present application claims priority to Chinese Patent Application No. 202110032708.5 filed with the China Patent Office on January 12, 2021, which is hereby incorporated by reference in its entirety.

### FIELD OF THE INVENTION

The present application relates to the field of biomedicine, in particular to a fully human single-domain tandem chimeric antigen receptor capable of specifically binding to CD5 protein and use thereof.

### BACKGROUND OF THE INVENTION

In recent years, chimeric antigen receptor T cell (CAR-T) technology has made breakthroughs, especially great success has been achieved in targets for B- cell malignancies, as represented by anti-CD19 CAR-T, and so far, three products, Kymriah, Yescarta and Tecartus have been approved by the FDA for marketing. Clinical trials have confirmed that CAR-T cells specifically targeting CD19 molecules can effectively treat B-cell malignancies, including relapsed/refractory B-ALL, CLL, and B-cell lymphoma¹⁻¹¹. According to literature reports, CD19 CAR-T has an effective rate of up to 90% in the treatment of relapsed/refractory ALL, and an effective rate of greater than 50% for CLL and some B- cell lymphomas. Although CAR-T therapy has achieved great success in the field of treatment of B-cell malignancies, it is very limited to study and apply it in T-cell malignancies.

T-cell malignancies include acute T-lymphoblastic leukemia (T-ALL) and T-cell lymphoma (TCL). T-ALL is a blood disease caused by abnormal proliferation of T lymphocytes, which is aggressive and progresses rapidly. T-cell lymphoma is a malignancy of T-cells that can develop in lymphoid tissues (such as lymph nodes and spleen) or outside lymphoid tissues (such as gastrointestinal tract, liver, nasal cavity, and skin), accounting for about 10%-15% of non-Hodgkin lymphoma, and an even higher proportion in China. CD5 is constitutively expressed on lymphocyte precursors, mature T-cells and some mature B-cells (Bl cells)^{12,13}. CD5 is highly expressed in about 85% of T-ALL and about 75% of peripheral T-cell lymphoma. In addition, CD5 is also frequently expressed in mantle cell lymphoma, chronic lymphocytic leukemia (B-CLL) and hairy cell leukemia cells (HCL). At present, T-cell malignancies have a high recurrence rate and poor prognosis after radiotherapy and chemotherapy. They are clinically intractable hematological malignancies, and there is an urgent need to develop cell therapy drugs for this disease. In normal cells, the expression of CD5 is limited to some subtypes of mature T cells and B cells, and the biological characteristics of CD5 antigen allow CD5 CAR T cells to produce effective anti-tumor activity against T-ALL and T lymphoma cells in vitro and in vivo. Therefore, CD5 can be used as a safe and reliable target for T-cell tumors.

Mamonkin M and other researchers developed a second-generation CD5 CAR using the scFv of the murine monoclonal antibody H65, and the results of clinical experiments showed that the CD5 CAR-T cells are safe and clinically effective in patients with r/r CD5+T-ALL and T-cell non-Hodgkin's lymphoma (T-NHL) treated with multiple lines in the past, and do not result in complete T-cell depletion^{14, 15}. More importantly, clearance of malignant T-cells by CD5 CAR-T cells may allow patients who were previously ineligible for transplantation to undergo HSCT (hematopoietic stem cell transplantation). However, according to the existing clinical follow-up data, it is known that the mouse-derived scFv CAR-T may suffer relapse of CD5+ malignant diseases after infusion. The limited duration of CAR-T in patients may be related to the production of anti-mouse antibodies, while the fully human scFv may be able to solve the problem of short duration of CD5 CAR-T in patients. The development of a fully human anti-CD5 antibody is of great significance for the development of the next generation of CAR-T products that last longer in vivo and have better long-term efficacy.

The present invention uses a fully human monoclonal antibody comprising only the heavy chain variable region. First of all, the fully human single-domain antibody simplifies the structure of CAR due to its small molecular weight, has lower immunogenicity than humanized murine antibodies, and has better potential in the application of antibody drugs or CAR-T. Second, the smaller size makes the fully human single-domain antibody more likely to access narrower or partially hidden antigenic epitopes, which has a spatial advantage over larger scFvs. In addition, the smaller size increases the viral titer of their gene therapy vectors and makes them easier to express on the surface of T-cells. Moreover, in the application of bispecific CAR, compared to the design of two scFvs in combination, the combination of two single-domain antibodies sdAbs capable of recognizing two antigens can simplify the structure of bispecific CAR, improve its expression efficiency and structural stability. After obtaining the antibody clone that specifically binds to the CD5 antigen and CD5 recombinant protein on the cell surface, we constructed it onto an IgG expression vector with human Fc segments to express the protein through CHOS cells, performed flow cytometric competition experiments, and obtained a fully human single-domain clone that binds to different epitopes of the CD5 antigen. Single-domain tandem CAR and a single single-domain clone may have the function of enhancing the efficacy of CAR-T, and may reduce the risk of ineffectiveness and recurrence due to antigen mutation. Fully human single-domain clones, tandem fully human single-domain clones that bind to different epitopes of the CD5 antigen and the control clone H65 are constructed on the second-generation CAR structure, and then packaged with lentivirus and transfected into T-cells. At the CAR-T cellular level, it is verified that the function of the tandem fully human single-domain CD5 antibody clone FHVH3 VH1 is stronger than that of the control clone H65 from the perspectives of target cell activation and cytotoxicity, target cell stimulation and proliferation.

In addition, literature reports that in most constructed tumor animal models, re-emerging tumor cells retain CD5 expression, which suggests that tumor recurrence is not due to the loss of antigen, and that failure to eradicate all xenografts is due to the poor persistence of CD5 CAR-T cells in mice. We used CRISPR/Cas9 technology to knock out the CD5 antigen on the surface of T cells, which minimizes the self-activation and suicide of CD5 CAR-T, and ensures its persistence and effectiveness in clinical verification.

In the development process, antibody screening/specificity identification at the phage level leads to quick and efficient screening of specific antibody clones, which is directly followed by CAR-T functional testing to select the best candidate antibodies, optimizing the antibody screening process for CAR-T development and improving the efficiency of research and development while ensuring the quality of research.

### SUMMARY OF THE INVENTION

The present application provides a fully human single-domain tandem chimeric antigen receptor that can specifically bind to CD5 and use thereof.

In one aspect, the present application includes a chimeric antigen receptor (CAR), wherein the CAR comprises a CD5 binding domain, a transmembrane domain, a co-stimulatory domain and an intracellular signaling domain, and the CD5 binding domain comprises one or more antibodies or fragments thereof specifically binding to CD5, wherein each antibody comprises a heavy chain complementarity determining region 1 (HCDR1), a heavy chain complementarity determining region 2 (HCDR2) and a heavy chain complementarity determining region 3 (HCDR3), the amino acid sequences of HCDR1, HCDR2, and HCDR3 of each antibody are selected from the following combinations: (1) HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40 ; (2) HCDR1 with an amino acid sequence as set forth in SEQ ID NO:41, HCDR2 with an amino acid sequence as set forth in SEQ ID NO:42, and HCDR3 with an amino acid sequence as set forth in SEQ ID NO:43; (3) HCDR1 with an amino acid sequence as set forth in SEQ ID NO:64, HCDR2 with an amino acid sequence as set forth in SEQ ID NO:65, and HCDR3 with an amino acid sequence as set forth in SEQ ID NO:66; (4) HCDR1 with an amino acid sequence as set forth in SEQ ID NO:67, HCDR2 with an amino acid sequence as set forth in SEQ ID NO:68, and HCDR3 with an amino acid sequence as set forth in SEQ ID NO:69.

In some embodiments, the CD5 binding domain comprises any two combinations of antibodies or fragments thereof selected from the following: (1) an antibody or a fragment thereof having HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40; (2) an antibody or a fragment thereof having HCDR1 with an amino acid sequence as set forth in SEQ ID NO:41, HCDR2 with an amino acid sequence as set forth in SEQ ID NO:42, and HCDR3 with an amino acid sequence as set forth in SEQ ID NO:43; (3) HCDR1 with an amino acid sequence as set forth in SEQ ID NO:64, HCDR2 with an amino acid sequence as set forth in SEQ ID NO:65, and HCDR3 with an amino acid sequence as set forth in SEQ ID NO:66; and (4) HCDR1 with an amino acid sequence as set forth in SEQ ID NO:67, HCDR2 with an amino acid sequence as set forth in SEQ ID NO:68, and HCDR3 with an amino acid sequence as set forth in SEQ ID NO:69.

In some embodiments, the CD5 binding domain comprises a first antibody or a fragment thereof and a second antibody or a fragment thereof that specifically bind to CD5, the HCDR1, HCDR2, and HCDR3 comprised in the first antibody or fragment thereof and the second antibody or fragment thereof are selected from any of the following combinations:
(1) the first antibody or fragment thereof comprises HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40; the second antibody or fragment thereof comprises HCDR1 with an amino acid sequence as set forth in SEQ ID NO:41, HCDR2 with an amino acid sequence as set forth in SEQ ID NO:42, and HCDR3 with an amino acid sequence as set forth in SEQ ID NO:43;
(2) the first antibody or fragment thereof comprises HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40; the second antibody or fragment thereof comprises HCDR1 with an amino acid sequence as set forth in SEQ ID NO:64, HCDR2 with an amino acid sequence as set forth in SEQ ID NO:65, and HCDR3 with an amino acid sequence as set forth in SEQ ID NO:66;
(3) the first antibody or fragment thereof comprises HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40; the second antibody or fragment thereof comprises HCDR1 with an amino acid sequence as set forth in SEQ ID NO:67, HCDR2 with an amino acid sequence as set forth in SEQ ID NO:68, and HCDR3 with an amino acid sequence as set forth in SEQ ID NO:69.

In some embodiments, the first antibody or fragment thereof is located at the N- or C-terminus of the second antibody or fragment thereof.

In some embodiments, the antibody comprises a heavy chain variable region, and the amino acid sequence of the heavy chain variable region is as set forth in 33, 35, 37, 47, 57, 59, 61 or63. In some embodiments, the antibody is a single-domain antibody. In some embodiments, the CD5 binding domain comprises a plurality of single-domain antibodies, and the plurality of single-domain antibodies can be linked by linkers, and the linker comprises fragment as set forth in SEQ ID NO:25.

In some embodiments, the transmembrane domain of the CAR comprises a polypeptide from a protein selected from: α, β or ζ chains of T cell receptors, CD28, CD3e, CD45, CD4, CD5, CD8α, CD9, CD16, CD22, CD33, CD37, CD64, CD80, CD86, CD134, CD137, and CD154. In some embodiments, the transmembrane domain comprises an amino acid sequence as set forth in SEQ ID No: 6 or a functional variant thereof.

In some embodiments, the co-stimulatory domain of the CAR comprises a polypeptide selected from the following proteins: CD28, 4-1BB, OX-40 and ICOS. In some embodiments, the co-stimulatory domain comprises an amino acid sequence as set forth in SEQ ID No: 8 or a functional variant thereof.

In some embodiments, the intracellular signaling domain of the CAR comprises a signaling domain derived from CD3ζ. In some embodiments, the intracellular signaling domain comprises an amino acid sequence as set forth in SEQ ID No: 10 or a functional variant thereof.

In some embodiments, the CAR further comprises a hinge region linking the CD5 binding domain and the transmembrane domain. In some embodiments, the hinge region comprises an amino acid sequence as set forth in SEQ ID No: 4 or a functional variant thereof.

In some embodiments, the CAR is further linked to a CD8α signal peptide. In some embodiments, the signal peptide comprises an amino acid sequence as set forth in SEQ ID No: 2 or a functional variant thereof.

In some embodiments, the CAR is further linked to a cleaving peptide. In some embodiments, the cleaving peptide comprises an amino acid sequence from a T2A peptide. In some embodiments, the cleaving peptide comprises an amino acid sequence as set forth in SEQ ID No: 12 or a functional variant thereof. In some embodiments, the CAR is further linked to a truncated EGFR molecule (tEGFR) by the cleaving peptide and the CSF2RA signal peptide. In some embodiments, the truncated EGFR molecule comprises an amino acid sequence as set forth in SEQ ID No: 16 or a functional variant thereof. The CSF2RA signal peptide comprises an amino acid sequence as set forth in SEQ ID No: 14 or a functional variant thereof.

In some embodiments, the CAR comprises an amino acid sequence selected from SEQ ID No: 27, 29, 31, 45, 49, 51, 53 or 55 or a functional variant thereof.

In another aspect, the present application further includes an isolated nucleic acid molecule encoding the CAR of the present application.

In another aspect, the present application further includes an isolated nucleic acid molecule encoding CAR, which comprises a nucleic acid sequence selected from SEQ ID No: 1, 3, 5, 7, 9, 11, 13, 15, 17-24, 26, 28, 30, 32, 34, 36, 44, 46, 48, 50, 52, 54, 56, 58, 60, or 62 or a functional variant thereof.

In another aspect, the present application further includes a vector comprising the nucleic acid molecule of the present application. In some embodiments, the vector is selected from plasmid, retroviral vector and lentiviral vector.

In another aspect, the present application further includes an immune effector cell comprising the CAR of the present application, the nucleic acid molecule of the present application, or the vector of the present application. In some embodiments, the immune effector cells are selected from T lymphocytes and natural killer (NK) cells. In some embodiments, CD5 is not expressed on the immune effector cells.

In another aspect, the present application further includes a method for preparing immune effector cells, which comprises knocking out the CD5 gene of the immune effector cells, and introducing the nucleic acid molecule or vector of the present application into the immune effector cells. In some embodiments, the knockout and/or introduction is carried out in vitro. In some embodiments, the knockout and/or introduction is achieved through an in vivo delivery system.

In another aspect, the present application further includes a pharmaceutical composition, which comprises the immune effector cells of the present application and a pharmaceutically acceptable adjuvant.

In another aspect, the present application further includes use of the CAR, the nucleic acid molecule, the vector, or the immune effector cells in the preparation of a drug, wherein the drug is used for treating a disease or condition associated with the expression of CD5. In some embodiments, the disease or condition associated with the expression of CD5 is a cancer or malignant tumor.

In another aspect, the present application further includes the CAR, the nucleic acid molecule, the vector, the immune effector cells or the pharmaceutical composition for use in the treatment of a disease or condition associated with the expression of CD5. In some embodiments, the disease or condition associated with the expression of CD5 is a cancer or malignant tumor. In another aspect, the present application further includes a method for treating a disease or condition associated with the expression of CD5, which comprises administering a therapeutically effective amount of the immune effector cells of the present application or the pharmaceutical composition of the present application to a patient in need thereof. In some embodiments, anti-EGFR antibodies such as cetuximab can be further administered to the patient in need thereof to inhibit the effect of the immune effector cells or the pharmaceutical composition, wherein the patient in need thereof include those patients who have serious adverse reactions to the immune effector cells or pharmaceutical compositions of the present application. In some embodiments, the disease or condition associated with the expression of CD5 is T lymphoblastic lymphoma or mantle cell lymphoma.

Those skilled in the art can easily perceive other aspects and advantages of the present application from the following detailed description. In the following detailed description, only exemplary embodiments of the present application are shown and described. As those skilled in the art will appreciate, the content of the present application enables those skilled in the art to make changes to the disclosed specific embodiments without departing from the spirit and scope of the invention to which the present application relates. Correspondingly, the drawings and descriptions in the specification of the present application are only exemplary rather than restrictive.

### DESCRIPTION OF DRAWINGS

Fig. 1 shows the schematic diagram of the principle of anti-CD5 VHs competitive binding FACS in the experiment (Fig. 1A) and the CD5 antigenic epitope binding identification results of CD5 KO CD5 CAR-T cells detected by flow cytometry (Fig. 1B).
Fig. 2 shows the CAR structure (Fig. 2A) used in the experiment and the CD5 and EGFRt expression detection of CAR-T cells after transfection (Fig. 2B).
Fig. 3 shows the surface expression of CD5 in T-ALL and T-lymphoma cell lines.
Fig. 4 shows the results of CD107a degranulation after co-incubation of different target cells with CAR-T cells.
Fig. 5 shows the cytotoxicity results of CAR-T cells on various target cells.
Fig. 6 shows the results of the basal apoptosis level of CAR-T/T cells.
Fig. 7 shows the proliferation of CAR-T cells after repeated stimulation of CD5 CAR-T cells by CD5-positive target cells.
Fig. 8 shows the results of treatment of the mouse T-cell tumor model by CAR-T/T cells and PBS.
Fig. 9 shows the results of the study on the binding of CT125A cells to CD5 antigen in Example 4.
Fig. 10 shows the results of degranulation activity of CT125A under stimulation by positive target cell in Example 5 (**, p<0.01; *, p<0.05).
Fig. 11 shows the results of cytotoxicity of CT125A on different types of positive target cells in Example 6.
Fig. 12 shows the results of the study on the CT125A cytokine release in Example 7.
Fig. 13 shows the results of the study on the elimination of CT125A cells by natural killer cells mediated by cetuximab in vitro.
Fig. 14 shows the survival rate curves of each group of animals during the experiment in Example 9.
Fig. 15 shows the change trend of body weight of animals in each group in Example 9.
Fig. 16 shows the change trend of the average tumor fluorescence signal intensity of animals in each group in Example 9.
Fig. 17 shows the trend of IL-2 in the peripheral blood of animals in each group in Example 9.
Fig. 18 shows the trend of IL-4 in the peripheral blood of animals in each group in Example 9.
Fig. 19 shows the trend of IL-6 in the peripheral blood of animals in each group in Example 9.
Fig. 20 shows the trend of IL-10 in the peripheral blood of animals in each group in Example 9.
Fig. 21 shows the trend of TNF-α in the peripheral blood of animals in each group in Example 9.
Fig. 22 shows the trend of IFN-γ in the peripheral blood of animals in each group in Example 9.
Fig. 23 shows the body weight changes (g) of animals in each group at each time point in Example 10.
Fig. 24 shows the tumor growth curves of animals in each group at each time point in Example 10.
Fig. 25 shows the results of the study on the binding of RD125 61-42-rFc single-domain antibody rabbit Fc fusion protein to CD5 positive cells.

### DETAILED DESCRIPTION OF THE INVENTION

The implementation of the invention of the present application will be described in the following specific examples, and those skilled in the art can easily understand other advantages and effects of the invention of the present application from the content disclosed in the specification. The CAR of the present application can specifically bind to CD5, the CAR-T cells made by using the CAR can stably express the CAR, and the CAR-T cells made by using the CAR have a higher CAR-positive rate. In addition, the CAR can promote the release of cytokines and can be used to treat diseases or conditions associated with the expression of CD5.

The implementation of the present application will employ, unless expressly indicated to the contrary, conventional methods of chemistry, biochemistry, organic chemistry, molecular biology, microbiology, recombinant DNA techniques, genetics, immunology and cell biology in the art. Descriptions of these methods can be found, for example, in Sambrook et al., Molecular Cloning: A Laboratory Manual (3rd Edition, 2001); Sambrook et al., Molecular Cloning: A Laboratory Manual (2nd Edition, 1989); Maniatis et al., Molecular Cloning: A Laboratory Manual (1982); Ausubel et al., Current Protocols in Molecular Biology (John Wiley and Sons, updated in July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I&II (IRL Press, Oxford, 1985); Anand, Techniques for the Analysis of Complex Genomes, (Academic Press, New York, 1992); Transcription and Translation (B. Hames & S. Higgins, Eds. , 1984); Perbal, A Practical Guide to Molecular Cloning (1984); Harlow and Lane, Antibodies, (Cold Spring Harbor Laboratory Press, Cold Spring Harbor, N.Y. , 1998) Current Protocols in Immunology QEColigan, A.M. Kruisbeek, D.H. MargµLies, E.M. Shevach and W. Strober, eds. , 1991); Annual Review of Immunology; and journal monographs such as Advances in Immunology.

Unless otherwise defined, all technical and scientific terms used herein have the meaning as commonly understood by one of ordinary skill in the art. For the purposes of the present application, the following terms are defined below.

In the present application, the term "chimeric antigen receptor" (CAR) is a fusion protein of the variable region of a single-chain antibody and a T cell signaling molecule. It allows T-cells to recognize specific antigens in a non-MHC-restricted manner and exert cytotoxicity. CAR is the core component of chimeric antigen receptor T cells (CAR-T), which can comprise a tumor-associated antigen (TAA) binding region, a transmembrane domain, a co-stimulatory domain and an intracellular signaling domain, and a hinge region may be further included between the tumor-associated antigen binding region and the transmembrane domain. In the present application, the CAR can be a genetically engineered chimeric protein capable of redirecting the cytotoxicity of immune effector cells to T-cells, which combines antibody-based antigen (such as CD5) specificity with T-cell receptor activation intracellular domains together. T-cells genetically modified to express CAR can specifically recognize and eliminate malignant cells expressing target antigens. For descriptions of CAR and CAR T-cells, see, for example, Sadelain M, Brentjens R, Rivi 'ere I. The basicprinciples of chimeric antigen receptor design. Cancer Discov. 2013;3(4):388-398; Turtle CJ, Hudecek M, Jensen MC, Riddell SR. Engineered T cells for anti-cancer therapy. Curr Opin Immunol. 2012;24(5):633-639; Dotti G, Gottschalk S, Savoldo B, Brenner MK. Design and development of therapies using chimeric antigen receptor-expressing T cells. Immunol Rev. 2014;257(1):107-126; and WO2013154760, WO2016014789.

In the present application, the term "CD5" is a type I transmembrane glycosylated protein that plays an important role in the negative regulation of T cell receptor signaling and promotes the survival of normal and malignant lymphocytes. CD5 is one of the characteristic surface markers of malignant T-cell tumors, and 80% of T-cell acute lymphoblastic leukemia (T-ALL) and peripheral T-cell lymphomas express CD5. In the present application, the CD5 may be human CD5 with GenBank accession number being NM_014207.4. CD5 proteins may also include fragments of CD5, such as the extracellular domain and fragments thereof.

In the present application, the term "CD5 binding domain" generally refers to the extracellular domain of CD5 CAR, which can specifically bind to an antigen. For example, the CD5 extracellular binding domain may comprise a chimeric antigen receptor capable of specifically binding to a CD5 polypeptide expressed on a human cell, an anti-CD5 antibody or an antigen-binding fragment thereof. The terms "binding domain", "extracellular domain", "extracellular binding domain", "antigen-specific binding domain" and "extracellular antigen-specific binding domain" are used interchangeably in the present application, and a CAR with the ability to specifically bind to a target antigen of interest (such asCD5) is provided. The CD5 binding domain may be of natural, synthetic, semi-synthetic or recombinant origin.

In the present application, the term "antibody" generally refers to a polypeptide molecule capable of specifically recognizing and/ or neutralizing a specific antigen. For example, an antibody may comprise an immunoglobulin composed of at least two heavy (H) chains and two light (L) chains inter-linked by disulfide bonds, and includes any molecule comprising an antigen-binding portion thereof. The term "antibody" includes monoclonal antibodies, antibody fragments or antibody derivatives, including but not limited to human antibodies, humanized antibodies, chimeric antibodies, single-domain antibodies (e.g., sdAb), single-chain antibodies (e.g., scFv). In the present application, the "fragment" of an antibody may refer to an antigen-binding fragment of an antibody, for example, Fab, Fab' and (Fab')₂ fragments and the like. The term "antibody" also includes all recombinant forms of antibodies, such as antibodies expressed in prokaryotic cells, nonglycosylated antibodies, and any antigen-binding antibody fragments and derivatives thereof. Each heavy chain can be composed of a heavy chain variable region (VH) and a heavy chain constant region. The VH regions can be further distinguished into hypervariable regions called complementarity determining regions (CDRs), which are interspersed in more conserved regions called framework regions (FRs). Each VH can be composed of three CDRs and four FR regions, which can be arranged in the following order from the amino terminus to the carboxyl terminus: FR1, CDR1, FR2, CDR2, FR3, CDR3 and FR4. The variable region of the heavy chain comprises a binding domain that interacts with the antigen. The constant regions of the antibodies may mediate the binding of the immunoglobulin to host tissues or factors, including various cells (e.g., effector cells) of the immune system and the first component (Clq) of the classical complement system.

In the present application, the term "single-domain antibody" may also be called sdAb, which refers to an antibody consisting of a single chain of heavy chain variable regions or heavy chain variable regions linked by a linker peptide.

In the present application, the term "transmembrane domain" generally refers to a domain in CAR that passes through the cell membrane, which is linked to the intracellular signaling domain and plays a role in transmitting signals. In the present invention, the transmembrane domain may be a CD8α transmembrane domain.

In the present application, the term "co-stimulatory domain" generally refers to an intracellular domain that can provide immune co-stimulatory molecules, which are cell surface molecules required for an effective response of lymphocytes to antigens. The co-stimulatory domain may include the co-stimulatory domain of CD28, and may also include the co-stimulatory domain of the TNF receptor family, such as the co-stimulatory domain of OX40 and 4-1BB.

In the present application, the term "hinge region" generally refers to the linking region between the antigen binding region and the immune cell Fc receptor (FcR) binding region. In the present invention, the hinge region may be a CD8α hinge region.

In the present application, the term "intracellular signaling domain" generally refers to the component of CAR located inside the cell for signal transduction, which comprises a signaling domain and a domain that specifically binds to the receptor component. For example, it may be selected from CD3ζ intracellular domain, CD28 intracellular domain, CD28 intracellular domain, 4-1BB intracellular domain and OX40 intracellular domain.

In the present application, the term "CD8α signal peptide" generally refers to a short (5-30 amino acids in length) peptide chain that guides the transfer of newly synthesized proteins to the secretory pathway.

In the present application, the term "cleaving peptide" refers to self-cleaving 2Apeptides, which can realize the function of cleaving proteins through ribosome jumping instead of proteolytic hydrolysis, and may include T2A, F2A and P2A, etc.

In the present application, the term "marker detection signal" generally refers to a gene, protein or other molecule with known function or sequence that can function as a specific marker and emit a detectable signal. The marker detection signal can be a fluorescent protein, such as: GFP, RFP, YFP. The marker detection signal can be EGFRt. The terms "EGFRt" and "tEGFR" are used interchangeably in the present invention and refer to a gene encoding a truncated human epidermal growth factor receptor polypeptide, which lacks the distal membrane EGF binding domain and cytoplasmic signaling tail, but retains extracellular epitopes recognized by anti-EGFR antibodies. EGFRt can be used as a non-immunogenic selection tool as well as a tracking marker capable of genetically modifying cells. In the present application, it can be used as a marker molecule for CAR-T cells to clear the ADCC pathway mediated by anti-EGFR antibodies (such as cetuximab) (cetuximab mediated ADCC pathway) of CAR-T cells in vivo when necessary (see US8802374B2), that is, it can be used as a safety switch in clinical transformation.

In the present invention, the term "CSF2RA signal peptide", that is, the colony stimulating factor 2 receptor subunit alpha signal peptide, is a peptide chain that can guide the expression of newly synthesized proteins on the surface of CAR-T cells.

In the present application, "anti-EGFR antibody" refers to antibodies that can trigger antibody-dependent cell-mediated cytotoxicity to allow immune cells to attack CAR-T cells with truncated epidermal growth factor receptor (EGFRt), to assist in the removal of CAR-T cells. The anti-EGFR antibody can be used when patients have severe adverse reactions after infusion of CAR-T or other situations that require removal of CAR-T cells. It can assist in the removal of CAR-T cells and alleviate symptoms related to CAR-T treatment. The anti-EGFR antibodies include, but are not limited to, cetuximab, panitumumab, necituzumab and nimotuzumab.

In the present application, "sequence identity" generally refers to the degree to which sequences are identical on a nucleotide-by-nucleotide or amino-acid-by-amino acid basis over a comparison window. "Percent sequence identity" can be calculated as follows: two optimally aligned sequences are compared over a comparison window to determine the number of positions with the same nucleic acid bases (e.g., A, T, C, G, I) or the same amino acid residues (e.g., Ala, Pro, Ser, Thr, Gly, Val, Leu, Ile, Phe, Tyr, Trp, Lys, Arg, His, Asp, Glu, Asn, Gln, Cys and Met) to obtain the number of matching positions, the number of matching positions is divided by the total number of positions in the comparison window (i.e., window size), and the result is multiplied by 100 to yield the percent sequence identity. Optimal alignment for purposes of determining percent sequence identity can be achieved in various ways known in the art, for example, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared or over a region of sequence of interest.

The term "functional variant" of a protein or polypeptide sequence in the present application refers to a sequence having amino acid changes compared to the parent sequence by one or more, such as 1-30, or 1-20 or 1-10, such as 1 or 2 or 3 or 4 or 5 amino acid substitutions, deletions and/or insertions. Functional variants substantially retain the biological properties of the protein or polypeptide sequence before the change. In one aspect, the present application encompasses variants of any protein or polypeptide sequence of the present application. In some embodiments, a functional variant of a protein or polypeptide sequence retains at least 60%, 70%, 80%, 90%, or 100% of the biological activity of the pre-altered parent. The functional variants described in the present invention may be functional variants of signal peptide, antibody, hinge region, transmembrane domain, co-stimulatory domain, intracellular signaling domain, cleaving peptide, CSF2RA signal peptide, and EGFRt. When referring to a functional variant of an antibody, it also includes functional variants of antibody variable regions (such as VH or VL), antibody constant regions (such as CH or CL), heavy chain CDR regions (HCDR1, HCDR2 or HCDR3), light chain CDR regions (LCDR1, LCDR2 or LCDR3) etc. Amino acid substitutions, deletions and/or insertions may occur in either the heavy chain CDR region or the light chain CDR region, or the heavy chain FR region or the light chain FR region, or the heavy chain constant region or the light chain constant region, wherein the variant substantially retains the biological properties of the antibody molecule before change. For antibodies, biological activities include, for example, antigen binding ability. In some embodiments, functional variants of antibodies comprise amino acid changes that do not result in the antibody variant losing binding to the antigen, but optionally confer properties such as increased affinity for the antigen and different effector functions. It is understood that the heavy chain variable region or light chain variable region, or each CDR region of the antibody can be altered individually or in combination. In some embodiments, there are no more than 1, 2, 3, 4, 5, 6, 7, 8, 9 or 10 amino acid changes in one or more or all three heavy chain CDRs. In some embodiments, the amino acid changes may be amino acid substitutions, such as conservative substitutions. In some embodiments, the functional variant has at least 80%, 85%, 90% or 95% or99% or higher amino acid identity to the parent. Similarly, a "functional variant" of a nucleic acid molecule in the present application refers to a nucleic acid molecule that encodes the same amino acid sequence as the parent nucleic acid molecule.

In the present application, the term "isolated" generally refers to an antibody that has been separated from components in its natural environment. In some embodiments, antibodies are purified to have a purity of greater than 95% or 99% as determined by, for example, electrophoresis (e.g., SDS-PAGE, isoelectric focusing (IEF), capillary electrophoresis) or chromatography (e.g., ion exchange or reversed-phase HPLC). For a review of methods for assessing antibody purity, see Flatman, S. et al., J.Chrom.B 848 (2007) 79-87.

In the present application, the term "nucleic acid molecule" generally refers to an isolated form of nucleotides, deoxyribonucleotides or ribonucleotides or analogs thereof of any length isolated from their natural environment or artificially synthesized. The nucleic acid molecules described in the present application can be isolated. For example, it may be produced or synthesized by the following methods: (i) it is amplified in vitro, such as by polymerase chain reaction (PCR) amplification, (ii) it is recombinantly produced by cloning, (iii) it is purified, for example, by enzymatic digestion and fractionation by gel electrophoresis, or (iv) it is synthesized, for example, by chemical synthesis. In some embodiments, the isolated nucleic acid is a nucleic acid molecule prepared by recombinant DNA techniques. In the present application, nucleic acids encoding the antibodies or antigen-binding fragments thereof can be prepared by various methods known in the art, including, but not limited to, overlap extension PCR operated using restriction fragments or using synthetic oligonucleotides, the specific operations of which can be found in Sambrook et al., Molecular Cloning, A Laboratory Manual, Cold Spring Harbor Laboratory Press, Cold Spring Harbor, N.Y. , 1989; and Ausube et al., Current Protocols in Molecular Biology, Greene Publishing and Wiley-Interscience, New York N.Y., 1993.

In the present application, the term "vector" generally refers to a nucleic acid molecule capable of self-replicating in a suitable host for transferring the inserted nucleic acid molecule into and/or between host cells. The vectors may include vectors mainly used for inserting DNA or RNA into cells, vectors mainly used for replicating DNA or RNA, and vectors mainly used for expression of transcription and/or translation of DNA or RNA. The vector also includes a vector having various functions as described above. The vector may be a polynucleotide capable of being transcribed and translated into a polypeptide when introduced into a suitable host cell. Generally, the vector can produce the desired expression product by culturing an appropriate host cell comprising the vector. In the present application, one or more nucleic acid molecules may be included in the vector. In addition, other genes may be included in the vector, such as marker genes that allow selection of the vector in appropriate host cells and under appropriate conditions. In addition, the vector may also comprise expression control elements that permit proper expression of the coding region in an appropriate host. Such control elements are well known to those skilled in the art, and may include, for example, promoters, ribosome binding sites, enhancers, and other control elements that regulate gene transcription or mRNA translation. In some embodiments, the expression control sequences are regulatable elements. The specific structure of the expression control sequence may vary depending on the species or the function of cell type, but generally includes 5' non-transcribed sequences and 5' and 3' non-translated sequences involved in the initiation of transcription and translation, respectively, such as TATA box, capping sequence, and CAAT sequence. For example, the 5' non-transcribed expression control sequence may comprise a promoter region which may comprise a promoter sequence for transcriptional control of the functionally linked nucleic acid. The vectors described herein may be selected from plasmids, retroviral vectors and lentiviral vectors. The plasmids, retroviral vectors and lentiviral vectors described in the present application may comprise CAR.

In the present application, the term "plasmid" generally refers to DNA molecules other than chromosomes or nucleoids in organisms such as bacteria and yeast, which exists in the cytoplasm and has the ability to replicate autonomously, enabling it to remain a constant number of copies in daughter cells and express the genetic information carried. Plasmids are used as vectors of genes in genetic engineering research.

In the present application, the term "retroviral vector" generally refers to a type of RNA virus whose genetic information is stored on ribonucleic acid, and most of these viruses have reverse transcriptase. Retroviruses comprises at least three genes: *gag,* a gene for the proteins that make up the center and structure of the virus; *pol,* a gene for reverse transcriptases; and *env,* a gene for the virus coat. Through retroviral transfection, the retroviral vector can randomly and stably integrate its own genome and the foreign genes it carries into the host cell genome, for example, it can integrate CAR molecules into the host cell.

In the present application, the term "lentiviral vector" generally refers to a type of diploid RNA viral vector belonging to retroviruses. The lentiviral vector is based on the genome of the lentivirus, and it is a vector prepared by removing a plurality of sequence structures related to viral activity to make it biologically safe, and then introducing the sequence and expression structure of a target gene required for the experiment into the genome backbone. Compared to other retroviruses, lentiviral vectors have a wider range of hosts, and have the ability to infect both dividing and non-dividing cells. For some cells that are difficult to transfect, such as primary cells, stem cells, and undifferentiated cells, it can greatly improve the transduction efficiency of the target gene (see Chen Chen and Wan Haisu, Lentiviral vectors and their research progress, Chinese Journal of Lung Cancer 17.12 (2014): 870-876. PMC). Through lentiviral vector transfection, the retroviral vector can randomly and stably integrate its own genome and the foreign genes it carries into the host cell genome, for example, it can integrate CAR molecules into the host cell.

In the present application, the term "transposon" generally refers to a discrete segment of DNA comprising a transposase gene, flanked by terminal inverted repeats (TIRs) comprising transposase binding sites. Transposases can bind to TIRs and transfer the transposon to a new site. The transposon of the present application is a two-component system consisting of a plasmid carrying CAR (transposon) and another plasmid carrying a transposase. The transposon can be introduced into target cells by means of electrotransduction and the like. For example, first, the two components are electroporated into peripheral blood mononuclear cells (PBMCs), and the expressed transposase acts on the terminal inverted repeats (TIRs) on both sides of CAR to cleave the CAR (transposon), which then integrated into the TA dinucleotide sequence in the genome of the target cell (e.g., T-cell). After transposition and stable genome integration are completed, the CAR protein can be expressed on the surface of the target cell (see Cheng Zhang, Jun Liu, Jiang F Zhong, et al. Engineering CAR-T cells. Biomarker Research. 2017, 5:22).

In the present application, the term "gene editing" generally refers to the technology of site-directed modification of the genome, which may include technologies based on zinc finger nucleases (ZFNs), transcription activator like effector nucleases (TALENs), clustered regularly interspaced short palindromic repeats/CRISPR-associated protein (Cas9) (CRISPR/Cas9) and other technologies. It enables highly efficient targeted modification of the genome by adding, removing or changing genetic material at specific locations in the genome. The gene editing described in the present application may include introducing CAR molecules into the genome of recipient cells through gene editing techniques (such as CRISPR-Cas9).

In the present application, the term "immune effector cell" generally refers to immune cells involved in clearing foreign antigens and performing effector functions in an immune response. It includes, for example, plasma cells, cytotoxic T-cells, NK cells, APSC pluripotent cells, mast cells, etc.

In the present application, the term "pharmaceutically acceptable adjuvant" generally refers to a pharmaceutically acceptable formulation carrier, solution or additive that enhances the properties of the formulation. Such additives are well known to those skilled in the art.

In the present application, the term "cancer" generally refers to or describes a physiological condition in mammals that is typically characterized by dysregulation of cell proliferation or survival. In the present application, hyperproliferative diseases referred to as cancer include, but are not limited to, solid tumors such as cancers occurring in the breast, respiratory tract, brain, reproductive organs, digestive tract, urinary tract, eye, liver, skin, head and neck, thyroid, parathyroid, and their distant metastases. Such diseases also include lymphomas, sarcomas, and leukemias. Examples of breast cancer include, but are not limited to, invasive ductal carcinoma, invasive lobular carcinoma, ductal carcinoma in situ, and lobular carcinoma in situ. Examples of cancers of the respiratory tract include, but are not limited to, small cell lung cancer and non-small cell lung cancer, as well as bronchial adenoma and pleuropulmonary blastoma. Examples of brain cancers include, but are not limited to, brainstem and hypothalamus keratomas, cerebellar and cerebral astrocytomas, medulloblastomas, ependymomas, and neuroectodermal and pineal tumors. Tumors of the male genitalia include, but are not limited to, prostate and testicular cancers. Tumors of the female genitalia include, but are not limited to, cancers of the endometrium, cervix, ovary, vagina, and vulva, and uterine tumors. Tumors of the respiratory tract include, but are not limited to, cancers of the anus, colon, colorectum, esophagus, gallbladder, stomach, pancreas, rectum, small intestine, and salivary glands. Tumors of the urethra include, but are not limited to, cancers of the bladder, penis, kidney, renal pelvis, ureter, and urethra. Eye cancers include, but are not limited to, intraocular melanoma and retinoblastoma. Examples of liver cancer include, but are not limited to, hepatocellular carcinoma (hepatocellular carcinoma with or without the fibrolamellar variation), cholangiocarcinoma (intrahepatic cholangiocarcinoma), and mixed hepatocellular cholangiocarcinoma. Skin cancers include, but are not limited to, squamous cell carcinoma, Kaposi's sarcoma, malignant melanoma, Merkel cell skin cancer, and non-melanoma skin cancer. Head and neck cancers include, but are not limited to, cancers of the larynx/hypopharynx/nasopharynx/oropharynx, and cancers of the lips and mouth. Lymphatic cancers include, but are not limited to, AIDS-related lymphoma, non-Hodgkin's lymphoma, cutaneous T-cell lymphoma, Hodgkin's disease, and central nervous system lymphoma. Sarcomas include, but are not limited to, soft tissue sarcomas, osteosarcomas, malignant fibrous histiocytomas, lymphosarcomas, and rhabdomyosarcomas. Leukemias include, but are not limited to, acute myeloid leukemia, acute lymphoblastic leukemia, chronic lymphocytic leukemia, chronic myeloid leukemia, and hairy cell leukemia.

The term "and/or" should be understood as meaning any one of the alternatives or both of the alternatives.

As used in the present application, the term "comprising" or "including" means including stated elements, integers or steps, but not excluding any other elements, integers or steps. In the present application, when the term "comprising" or "including" is used, unless otherwise specified, situations consisting of the mentioned elements, integers or steps are also covered. For example, when referring to "comprising" the antibody variable region of a particular sequence, it is also intended to encompass an antibody variable region that consists of that particular sequence.

In the present application, the term "about" usually refers to a change in the range of 0.5%-10% above or below the specified value, such as a change in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### Chimeric antigen receptor

In the present application, the CAR may comprise an extracellular domain specifically binding to CD5, a transmembrane domain, an intracellular co-stimulatory signaling domain, and an intracellular signaling domain. In the present application, the extracellular domain of the CAR may comprise a single-domain antibody (VHH), two or more single-domain antibodies in tandem (2×VHH) of the present invention. For example, the single-domain antibody can be linked to the transmembrane domain via a hinge region, such as the CD8α hinge. In the present application, the CAR can be used to transduce immune effector cells (such as T-cells) and expressed on the cell surface. Therefore, the present application can also provide T-cells expressing the chimeric antigen receptor, and the use of the T-cells and/or the CAR in the preparation of drugs for treating CD5-related diseases.

In the present application, the chimeric antigen receptor (CAR) may comprise a CD5 binding domain, a transmembrane domain, a co-stimulatory domain and an intracellular signaling domain. In the present application, the CD5 binding domain may comprise an antibody or fragment thereof specifically binding to CD5, and the antibody may comprise a heavy chain complementarity determining region 1 (HCDR1), a heavy chain complementarity determining region 2 (HCDR2) and a heavy chain complementarity determining region 3 (HCDR3), the amino acid sequences of HCDR1-3 are as set forth in SEQ ID NOs: 38-43, 64-69. In the present application, the antibody comprises a heavy chain variable region, and the amino acid sequence of the heavy chain variable region is as set forth in SEQ ID No: 33, 35, 37, 47, 57, 59, 61 or 63.

In the present application, the antibody may be a single-domain antibody. In some embodiments, the antibody may comprise an amino acid sequence as set forth in SEQ ID No: 33, 35, 37, 47, 57, 59, 61 or 63 or a functional variant thereof. For example, the single-domain antibody may include FHVH1 sdAb, the sequence of which is as set forth in SEQ ID No: 33; the single-domain antibody may include FHVH3 sdAb, the sequence of which is as set forth in SEQ ID No: 35; the single-domain antibody may include FHVH3VH1 sdAb, the sequence of which is as set forth in SEQ ID No: 37; the single-domain antibody may include FHVH1 VH3 sdAb, the sequence of which is as set forth in SEQ ID No: 47; the single-domain antibody may include FHVH2 sdAb, the sequence of which is as set forth in SEQ ID No: 57; the single-domain antibody may include FHVH4 sdAb, the sequence of which is as set forth in SEQ ID No: 59; the single-domain antibody may include FHVH2VH1 sdAb, the sequence of which is as set forth in SEQ ID No: 61; the single-domain antibody may include FHVH4VH1 sdAb, the sequence of which is as set forth in SEQ ID No: 63.

For example, the single-domain antibody described in the present application can be FHVH1 sdAb, the sequence of which is as set forth in SEQ ID No: 33, and the amino acid sequences of HCDR1-3 of the single-domain antibody FHVH1 are respectively as set forth in SEQ ID No: 38, SEQ ID No: 39 and SEQ ID No: 40; the single-domain antibody may include FHVH3 sdAb, the sequence of which is as set forth in SEQ ID No: 35, and the amino acid sequences of HCDR1-3 of the single-domain antibody FHVH3 are respectively as set forth in SEQ ID No: 41, SEQ ID No: 42 and SEQ ID No: 43; the single-domain antibody may include FHVH1 VH3 sdAb, the sequence of which is as set forth in SEQ ID No: 37, and the amino acid sequences of HCDR1-3 of the single-domain antibody FHVH1VH3 are respectively as set forth in SEQ ID No: 38, SEQ ID No: 39, SEQ ID No: 40, SEQ ID No: 41, SEQ ID No: 42 and SEQ ID No: 43. The single-domain antibody may include FHVH3VH1 sdAb, the sequence of which is as set forth in SEQ ID No: 37, and the amino acid sequences of HCDR1-3 of the single-domain antibody FHVH3VH1 are respectively as set forth in SEQ ID No: 38, SEQ ID No: 39, SEQ ID No: 40, SEQ ID No: 41, SEQ ID No: 42 and SEQ ID No: 43.

For example, the single-domain antibody described in the present application can be FHVH2 sdAb, the sequence of which is as set forth in SEQ ID No: 57, and the amino acid sequences of HCDR1-3 of the single-domain antibody FHVH2 are respectively as set forth in SEQ ID No: 64, SEQ ID No: 65 and SEQ ID No: 66; the single-domain antibody may include FHVH4 sdAb, the sequence of which is as set forth in SEQ ID No: 59, and the amino acid sequences of HCDR1-3 of the single-domain antibody FHVH4 are as respectively set forth in SEQ ID No: 67, SEQ ID No: 68 and SEQ ID No: 69; the single-domain antibody may include FHVH2VH1 sdAb, the sequence of which is as set forth in SEQ ID No: 61, and the amino acid sequences of HCDR1-3 of the single-domain antibody FHVH2VH1 are respectively as set forth in SEQ ID No: 64, SEQ ID No: 65, SEQ ID No: 66, SEQ ID No: 38, SEQ ID No: 39 and SEQ ID No: 40. The single-domain antibody may include FHVH4VH1 sdAb, the sequence of which is as set forth in SEQ ID No: 63, and the amino acid sequences of HCDR1-3 of the single-domain antibody FHVH4VH1 are respectively as set forth in SEQ ID No: 67, SEQ ID No: 68, SEQ ID No: 69, SEQ ID No: 38, SEQ ID No: 39 and SEQ ID No: 40.

The CAR described in the present application may comprise a transmembrane domain, and the transmembrane domain may comprise a polypeptide from a protein selected from the group consisting of: α, β or ζ chains of T cell receptors, CD28, CD3e, CD45, CD4, CD5, CD8α, CD9, CD16, CD22, CD33, CD37, CD64, CD80, CD86, CD134, CD137, and CD154. In the present application, the transmembrane domain may comprise an amino acid sequence as set forth in SEQ ID No: 6 or a functional variant thereof. For example, the transmembrane domain of the present application may include CD8α, the sequence of which is as set forth in SEQ ID No: 6.

In the present application, the co-stimulatory domain may comprise a polypeptide from a protein selected from the group consisting of: CD28, 4-1BB, OX40 and ICOS. In the present application, the co-stimulatory domain may comprise an amino acid sequence as set forth in SEQ ID No: 8 or a functional variant thereof.

The CAR described in the present application can include an intracellular signaling domain, which can comprise a signaling domain derived from CD3ζ. In the present application, the intracellular signaling domain may comprise an amino acid sequence as set forth in SEQ ID No: 10 or a functional variant thereof.

The CAR of the present application may include a hinge region, and the hinge region may link the antibody and the transmembrane domain. In the present application, the hinge region may comprise an amino acid sequence as set forth in SEQ ID No: 4 or a functional variant thereof.

The CAR of the present application may include a signal peptide, for example, the signal peptide may be located at the N-terminus of the extracellular domain that specifically binds to CD5. The signal peptide may comprise an amino acid sequence as set forth in SEQ ID No: 2 or a functional variant thereof. For example, the signal peptide can be a CD8α signal peptide, the sequence of which is as set forth in SEQ ID No: 2.

In the present application, the CAR can also be linked to a cleaving peptide. In the present application, the cleaving peptide may comprise an amino acid sequence derived from a T2A peptide. In the present application, the cleaving peptide may comprise an amino acid sequence as set forth in SEQ ID No: 12 or a functional variant thereof. For example, the cleaving peptide can be T2A, the sequence of which is as set forth in SEQ ID No: 12.

In the present application, the CAR can also be linked to an EGFRt fragment, and the EGFRt fragment can be used for signal detection, or used as a molecular switch for CAR-T cells.

In the present application, the CAR may comprise an amino acid sequence as set forth in SEQ ID No: 27, 29, 31, 45, 49, 51, 53 or 55, or a functional variant thereof. For example, the CAR can be selected from FHVH1 CAR, the sequence of which is as set forth in SEQ ID No: 27. For another example, the CAR can be selected from FHVH3 CAR, the sequence of which is as set forth in SEQ ID No: 29; the CAR can be selected fromFHVH3VH1 CAR, the sequence of which is as set forth in SEQ ID No: 31. The CAR can be selected from FHVH1VH3 CAR, the sequence of which is as set forth in SEQ ID No: 45. The CAR can be selected from FHVH2 CAR, the sequence of which is as set forth in SEQ ID No: 49; the CAR can be selected from FHVH4 CAR, the sequence of which is as set forth in SEQ ID No: 51; the CAR can be selected from FHVH2VH1 CAR, the sequence of which is as set forth in SEQ ID No: 53; and the CAR can be selected from FHVH4VH1 CAR, the sequence of which is as set forth in SEQ ID No: 55.

In some embodiments, the CAR of the present application may sequentially comprise a CD5 binding domain, a transmembrane domain, a co-stimulatory domain, and an intracellular signaling domain from the N-terminus. Among others, the CAR may include a CD5 binding domain, and the sequence of the CD5 binding domain is as set forth in SEQ ID No: 33, 35. Among others, the CD5 binding domain may include HCDR1-3, the sequences of which are sequentially as set forth in SEQ ID No: 38-40; and the CD5 binding domain may include another group of HCDR1-3, the sequences of which are sequentially as set forth in SEQ ID No: 41-43. The CAR may include FHVH3VH1 CAR or the CAR of the present application having the same two sets of HCDR1-3. The CD5 binding domain includes a tandem heavy chain variable region, the sequence of which is as set forth in SEQ ID No: 37 or 47, wherein the heavy chain variable region may include HCDR1-3, the sequences of which are sequentially set forth in SEQ ID No: 38-40, and the CD5 binding domain may include another group of HCDR1-3, the sequence of which are sequentially as set forth in SEQ ID No: 41-43.

In some embodiments, the CAR of the present application may sequentially comprise a CD5 binding domain, a transmembrane domain, a co-stimulatory domain, and an intracellular signaling domain from the N-terminus. Among others, the CAR may include a CD5 binding domain, and the sequence of the CD5 binding domain is as set forth in SEQ ID No: 57, 59. Among others, the CD5 binding domain may include HCDR1-3, the sequences of which are sequentially as set forth in SEQ ID No: 64-66; and the CD5 binding domain may include another group of HCDR1-3, the sequences of which are sequentially as set forth in SEQ ID No: 67-69. The CD5 binding domain includes a tandem heavy chain variable region, the sequence of which is as set forth in SEQ ID No: 61 or 63, wherein the heavy chain variable region may include HCDR1-3, the sequences of which are sequentially as set forth in SEQ ID No: 64-66, 38-40, and the CD5 binding domain may include another group of HCDR1-3, the sequence of which are sequentially as set forth in SEQ ID No: 67-69, 38-40.

A linker peptide may also be included between the heavy chain variable regions, the sequence of which is as set forth in SEQ ID No: 25. For example, the CAR may include FHVH3VH1 CAR, FHVH1VH3 CAR, FHVH2VH1 CAR, FHVH4VH1 CAR or the CAR of the present application having the same linker peptide. The transmembrane domain may include a transmembrane domain derived from CD8α, and its sequence may be as set forth in SEQ ID No: 6. For example, the CAR may include FHVH3VH1 CAR, FHVH1VH3 CAR, FHVH2VH1 CAR, FHVH4VH1 CAR or the CAR of the present application having the same transmembrane domain. The co-stimulatory domain may comprise a co-stimulatory structure from 4-1BB, and its sequence may be as set forth in SEQ ID No: 8. For example, the CAR may include FHVH3VH1 CAR, FHVH1VH3 CAR, FHVH2VH1 CAR, FHVH4VH1 CAR or the CAR of the present application having the same co-stimulatory domain. The intracellular signaling domain may comprise a signaling domain derived from CD3ζ, the sequence of which is as set forth in SEQ ID No: 10. For example, the CAR may include FHVH3VH1 CAR, FHVH1 VH3 CAR, FHVH2VH1 CAR, FHVH4VH1 CAR or the CAR of the present application having the same intracellular signaling domain. The CAR may also comprise a hinge region, which may be located at the C-terminus of the CD5 binding domain and at the N-terminus of the transmembrane domain, and its sequence may be as set forth in SEQ ID No: 4, for example. For example, the CAR may include FHVH3VH1 CAR, FHVH1VH3 CAR, FHVH2VH1 CAR, FHVH4VH1 CAR or the CAR of the present application having the same hinge region. The CAR can also be linked to a signal peptide, which can be located at the N-terminal of the CAR, and its sequence can be as set forth in SEQ ID No: 2.

The CAR can also be linked to a cleaving peptide, for example: T2A. The cleaving peptide may be located at the C- terminus of the intracellular signaling domain, and its sequence may be as set forth in SEQ ID No: 12. The CAR can also be linked to the CSF2RA signal peptide, which can be located before EGFRt, and its sequence can be as set forth in SEQ ID No: 14, for example. The CAR can also be linked to a marker detection signal, which can be located at the C-terminus of the CAR (or, the cleaving peptide). The marker detection signal can be selected from the group consisting of: GFP, RFP, YFP or EGFRt, and the sequence of EGFRt can be as set forth in SEQ ID No: 16, for example.

For example, the CAR of the present application can be FHVH3VH1 CAR, the amino acid sequences of its VHH (FHVH1) HCDR1-3 are respectively as set forth in SEQ ID No: 38, SEQ ID No: 39 and SEQ ID No: 40; the amino acid sequence of VHH (FHVH1) is as set forth in SEQ ID No:33; the amino acid sequences of VHH(FHVH3)HCDR1-3 are as set forth in SEQ ID No: 41, SEQ ID No: 42 and SEQ ID No: 43 respectively; the amino acid of VHH(FHVH3) is as set forth in SEQ ID No:35. The sequence of the linker peptide between VHH(FHVH3) and VHH (FHVH1) is as set forth in SEQ ID No: 25; its hinge region is as set forth in SEQ ID No: 4; its transmembrane domain is as set forth in SEQ ID No: 6; its co-stimulatory domain is 4-1BB co-stimulatory domain, as set forth in SEQ ID No: 8; its CD3ζ intracellular signaling domain is as set forth in SEQ ID No: 10. The FHVH3VH1 CAR also comprises a cleaving peptide as set forth in SEQ ID No: 12, a CSF2RA signal peptide as set forth in SEQ ID No: 14, and EGFRt as set forth in SEQ ID No: 16; a CD8α signal peptide as set forth in SEQ ID No:2.

CD8α For example, the CAR of the present application can be FHVH1 CAR, the amino acid sequences of its VHH(FHVH1)HCDR1-3 are respectively as set forth in SEQ ID No: 38, SEQ ID No: 39 and SEQ ID No: 40; the amino acid sequence of VHH(FHVH1) is as set forth in SEQ ID No:33; its hinge region is as set forth in SEQ ID No: 4; its transmembrane domain is as set forth in SEQ ID No: 6; its co-stimulatory domain is 4-1BB co-stimulatory domain, as set forth in SEQ ID No: 8; its CD3ζ intracellular signaling domain is as set forth in SEQ ID No: 10; the FHVH1 CAR also comprises a cleaving peptide as set forth in SEQ ID No: 12, a CSF2RA signal peptide as set forth in SEQ ID No: 14, and EGFRt as set forth in SEQ ID No: 16; and a CD8α signal peptide as set forth in SEQ ID No: 2.

For example, the CAR of the present application can be FHVH3 CAR, the amino acid sequences of its VHH(FHVH3)HCDR1-3 are respectively as set forth in SEQ ID No: 41, SEQ ID No: 42 and SEQ ID No: 43; the amino acid sequence of VHH(FHVH3) is as set forth in SEQ ID No:35; its hinge region is as set forth in SEQ ID No: 4; its transmembrane domain is as set forth in SEQ ID No: 6; its co-stimulatory domain is 4-1BB co-stimulatory domain, as set forth in SEQ ID No: 8; its CD3ζ intracellular signaling domain is as set forth in SEQ ID No: 10; the FHVH1 CAR also comprises a cleaving peptide as set forth in SEQ ID No: 12, a CSF2RA signal peptide as set forth in SEQ ID No: 14, and EGFRt as set forth in SEQ ID No: 16; and a CD8α signal peptide as set forth in SEQ ID No: 2.

### Nucleic acid, vector, cell, preparation method and composition

In another aspect, the present application provides an isolated nucleic acid molecule which can encode the CAR of the present application. The isolated nucleic acid molecule encoding CAR described in the present application may comprise a nucleic acid sequence selected from the group consisting of SEQ ID No: 1, 3, 5, 7, 9, 11, 13, 15, 17-24, 26, 28, 30, 32, 34, 36, 44, 46, 48, 50, 52, 54, 56, 58, 60, or 62 or a functional variant thereof. The nucleic acid molecules described in the present application can be isolated. For example, it may be produced or synthesized by the following methods: (i) it is amplified in vitro, such as by polymerase chain reaction (PCR) amplification, (ii) it is recombinantly produced by cloning, (iii) it is purified, for example, by enzymatic digestion and fractionation by gel electrophoresis, or (iv) it is synthesized, for example, by chemical synthesis. In some embodiments, the isolated nucleic acid is a nucleic acid molecule prepared by recombinant DNA techniques.

In another aspect, the present application provides a vector which may comprise the nucleic acid molecule. In the present application, the vector may be selected from one or more of plasmids, retroviral vectors and lentiviral vectors. The lentiviral vector of the present application may comprise CAR. For example, the lentiviral vector described in the present application may comprise a nucleic acid sequence selected from the group consisting of SEQ ID No: 1, 3, 5, 7, 9, 11, 13, 15, 17-24, 26, 28, 30, 32, 34, 36, 44, 46, 48, 50, 52, 54, 56, 58, 60, or 62 or a functional variant thereof. In addition, other genes may be included in the vector, such as marker genes that allow selection of the vector in appropriate host cells and under appropriate conditions. In addition, the vector may also comprise expression control elements that permit proper expression of the coding region in an appropriate host. Such control elements are well known to those skilled in the art, and may include, for example, promoters, ribosome binding sites, enhancers, and other control elements that regulate gene transcription or mRNA translation. In some embodiments, the expression control sequences are regulatable elements. The specific structure of the expression control sequence may vary depending on the species or the function of cell type, but generally includes 5' non-transcribed sequences and 5' and 3' non-translated sequences involved in the initiation of transcription and translation, respectively, such as TATA box, capping sequence, and CAAT sequence. For example, the 5' non-transcribed expression control sequence may comprise a promoter region which may comprise a promoter sequence for transcriptional control of the functionally linked nucleic acid. One or more nucleic acid molecules described in the present application can be operably linked to the expression control element. Such vectors may include, for example, plasmids, cosmids, viruses, phages, or other vectors commonly used in, for example, genetic engineering. For example, the vector is an expression vector, including vector sdAb plasmid and/or CAR plasmid.

In another aspect, the present application provides an immune effector cell which may comprise the CAR, the nucleic acid molecule, or the vector of the present application. In the present application, the immune effector cells may be mammalian cells. In the present application, the immune effector cells may be selected from T lymphocytes and natural killer (NK) cells.

In another aspect, the present application provides a method for preparing immune effector cells, which comprises knocking out the CD5 gene of the immune effector cells, and introducing the vectors of the present application into the immune effector cells. For example, the vectors described herein can be introduced into the immune effector cells, such as T lymphocytes or natural killer (NK) cells. In some embodiments, each type of cell or each cell may comprise one or more of the vectors described in the present application. In some embodiments, each type of cell or each cell may comprise a plurality of (e.g., 2 or more) or a plurality of types of (e.g., 2 types or more) vectors of the present application. In the present application, for introducing the vector into immune effector cells, the vector of the present application can be introduced into the cells by methods known in the art. For example, retroviral vectors can be used to transfect immune effector cells to integrate the viral genome with CAR molecules into the host genome to ensure long-term and stable expression of the target gene. For another example, transposons are introduced into target cells through plasmids carrying CAR (transposon) and plasmids carrying transposase. As another example, CAR molecules can be added to the genome through gene editing (such as CRISPR/Cas9). In the present application, the vector carrying the CAR molecule of the present application can be introduced into the cells by methods known in the art, such as electroporation, liposome transfection (lipofectamine 3000, Invitrogen).

In another aspect, the present application provides a pharmaceutical composition, which may comprise the immune effector cells and a pharmaceutically acceptable adjuvant. The pharmaceutically acceptable adjuvants may include buffers, antioxidants, preservatives, low molecular weight polypeptides, proteins, hydrophilic polymers, amino acids, sugars, chelating agents, counterions, metal complexes and/or non-ionic surfactants, etc. In the present application, the pharmaceutical composition can be formulated for administration orally, intravenously (for example, intravenous injection, I.V.), intramuscularly (e.g., intramuscular injection, I.M.), in situ at the tumor site, by inhalation, rectally, vaginally, transdermally or via a subcutaneous depot.

### Pharmaceutical use

In another aspect, the present application provides the use of the CAR, the nucleic acid molecule, the vector, or the immune effector cells in the preparation of a drug, wherein the drug is used to treat a disease or condition associated with the expression of CD5. In the present application, the disease or condition associated with the expression of CD5 is a cancer or malignant tumor. In some embodiments, the cancer or malignancy may be selected from a malignant T-cell tumor or a malignant B-cell tumor. Among them, the malignant T-cell tumor can be selected from T-cell acute lymphoblastic leukemia (T-ALL), T-cell lymphoma (TCL) (such as peripheral T-cell lymphoma, cutaneous T-cell lymphoma (CTCL), T-cell non- Hodgkin's lymphoma (T-NHL)); the malignant B-cell neoplasm may be selected from chronic lymphocytic leukemia (B-CLL) (e.g., hairy cell leukemia (HCL)), mantle cell lymphoma (B-MCL), diffuse large B lymphoma (DLBCL).

In another aspect, the present application provides the use of the CAR, the nucleic acid molecule, the vector, or the immune effector cells in the treatment of a disease or condition associated with the expression of CD5.

In another aspect, the present application provides a method for treating a disease or condition associated with the expression of CD5, comprising administering to a patient the CAR, the nucleic acid molecule, the vector, or the immune effector cells.

Without being limited by any theory, the following examples are only to illustrate the working mode of the chimeric antigen receptor, vector, cell, and composition of the present application, and are not intended to limit the scope of the present invention.

### Study overview

The researchers use fully human phages for antibody screening to directly obtain fully human monoclonal antibodies. Compared to traditional hybridoma technology, it omits the difficult step of humanizing murine antibody. Moreover, the fully human antibody has lower immunogenicity than humanized murine antibody, and has better potential in the development of CAR-T. After obtaining antibody clones that specifically bind to the cell surface CD5 antigen and CD5 recombinant protein, the applicant conducted in-depth research, constructed these clones and the control clone H65 onto the second-generation CAR structure, and then packaged them with lentivirus and transfected into T cells. At the level of CAR-T cells, fully human CD5 antibody clones and candidate CAR-T molecules with stronger functions than the control clone H65 were screened from the perspectives of target cell activation and cytotoxicity, and target cell stimulation and proliferation.

In addition, literature reports that in most constructed tumor animal models, re-emerging tumor cells retain CD5 expression, which suggests that tumor recurrence is not due to the loss of antigen, and that failure to eradicate all xenografts is due to the poor persistence of CD5 CAR-T cells in mice¹⁴. The experimental results show that CD5 knockout CAR-T cells can expand normally, and maintain cytotoxicity to CD5-positive target cells, while minimizing the self-activation and suicide of CD5 CAR-T, ensuring its persistence and effectiveness in clinical validation.

### Examples

### Example 1. CAR-T cell antigen binding assay and epitope competition experiment

### Experimental purpose and principle:

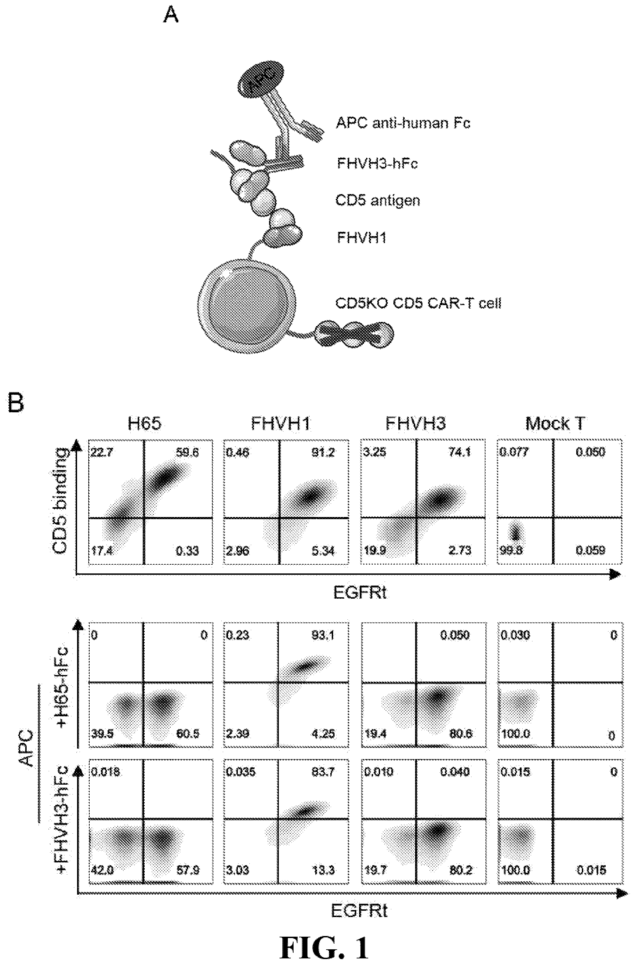

As shown in Fig. 1A, in order to detect whether there are clones that bind to different epitopes of the CD5 antigen among the obtained fully human single-domain clones, the clones to be tested are constructed on IgG vectors with human Fc tags and expressed in CHOS cells. If the clone IgG antibody to be tested and the CD5 KO T cells expressing the CAR to be tested bind to different epitopes of the CD5 antigen, then the clone IgG antibody to be tested and the CD5 KO T cells expressing the CAR to be tested can simultaneously bind to the same CD5 antigen, and then bind to APC-human Fc antibody, showing APC-positive in flow cytometry. Otherwise, it is APC-negative.

### Brief experimental steps of cell antigen binding assay:

1) Take CD5 KO CAR-T transfected with clones H65, FHVH1, and FHVH3 and MOCK T, each 1 × 10^6, centrifuge at 600g for 5 min at room temperature. After washing twice with PBS, mix with 0.2 µg of biotinylated CD5 antigen, and incubate at 4°C in the dark for 30 min.
2) Wash twice with PBS, add 0.2 µL/test APC-streptavidin and 5 µL of PE-EGFR antibody, wash twice with PBS, resuspend with 100 µL of PBS, and detect by flow cytometry.

### Brief experimental steps of epitope competition experiment:

1) Take CD5 KO CAR-T transfected with clones H65, FHVH1, and FHVH3 and MOCK T, each 1 × 10^6, centrifuge at 600g for 5 min at room temperature. Wash twice with PBS for later use;
2) Pre-mix 0.5 µg each of H65 antibody, FHVH3 antibody or FHVH1 antibody with human Fc tag and 0.2 µg of CD5 antigen, then add to CD5 KO T cells expressing the CAR to be tested respectively, and incubate at 4°C in the dark for 30 min.
3) Wash twice with PBS.
4) Add 2 µL of APC-human Fc and 5 µL of PE-EGFR antibody to each well, and incubate at 4°C in the dark for 30 min. After washing with PBS, resuspend in 100 µL of PBS, and detect by flow cytometry.

### Main materials and reagents:

APC anti-human IgG, Fcγ fragment specific, Jackson ImmunoResearch, Cat. 109-136-170;
PE anti-human EGFR Antibody, Clone AY13, BioLegend, Cat. No.352904;

### Experimental results:

As shown in the schematic diagram of Fig. 1A, after the FHVH3-IgG antibody with hFc was bound to the CD5 antigen, it could bind to the CD5 KO CAR-T of clone FHVH1, and then bind to the APC-human Fc antibody. The CD5 KO CAR-T transfected with H65, FHVH1, and FHVH3 could effectively bind to CD5 antigen, while MOCK T did not bind to CD5 antigen (Fig. 1B, upper part). H65-hFc and FHVH3-hFc antibodies did not affect the binding of FHVH1 CAR-T cells to CD5 antigen, indicating that FHVH1 binds to different CD5 epitopes than H65 and FHVH3, while FHVH3 and H65 recognize overlapping CD5 antigenic epitopes (Fig. 1B, lower part). After proving that FHVH1 and FHVH3 bind to different CD5 antigenic epitopes, we speculate that the tandem use of FHVH1 and FHVH3 can improve the efficacy and reduce the risk of tumor escape caused by antigen mutations. Therefore, FHVH1 and FHVH3 linked in tandem in different orders can be used as two candidate clones of anti-CD5 bi-epitope antibody and anti-CD5 bi-epitope CAR.

### Example 2. Preparation and detection of CD5 KO CAR-T cells

### Experimental purpose and principle:

The present application uses lentiviral transfection to make the knockout T cells express CAR. For the CAR-T preparation process, please refer to the patent (Zhou J, Liu J, Hu G, et al. Chimeric antigen receptor (car) binding to bcma, and uses thereof: US Patent Application 16/650,580[P]. 2020-8-6.). The lentiviral vector is based on the genome of the lentivirus, and it is a vector prepared by removing a plurality of sequence structures related to viral activity to make it biologically safe, and then introducing the sequence and expression structure of a target gene required for the experiment into the genome backbone. Compared to other retroviruses, lentiviral vectors have a wider range of hosts, and have the ability to infect both dividing and non-dividing cells. For some cells that are difficult to transfect, such as primary cells, stem cells, and undifferentiated cells, it can greatly improve the transduction efficiency of the target gene (see Chen Chen and Wan Haisu, Lentiviral vectors and their research progress, Chinese Journal of Lung Cancer 17.12 (2014): 870-876. PMC). Through lentiviral vector transfection, CAR molecules can be integrated into host cells.

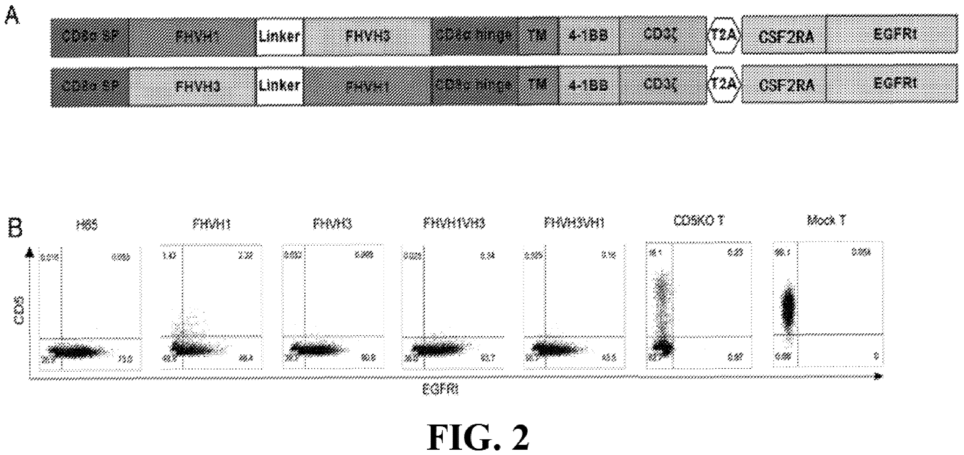

T cells were knocked out of CD5 and transfected with a single single-domain clone CAR lentivirus, tandem fully human single-domain clone CAR lentivirus (the structure as shown in Fig. 2A), and mouse-derived control H65 CAR lentivirus, detected after 5-7 days for CD5, EGFRt expression and CD5 antigen expression (Fig. 2B). The CAR structure comprises CD8α signal peptide, sdAb, CD8α hinge region, CD8α transmembrane region, 4-1BB co-stimulatory molecule and CD3ζ and is linked to a truncated EFGR molecule (EGFRt) with T2A. EGFRt can be used as a safety switch during clinical transformation, and because EGFRt is co-expressed with CAR molecules, it can be used as an indirect detection index for the distribution of CAR molecules on the surface of T cells without affecting the structure and function of CAR.

The sequences of exemplary CAR molecules and antigen recognition portions thereof mentioned herein are shown in Table 1 below.

**Table 1. Exemplary CAR molecules and antigen recognition portions thereof**

| FHVH1 CAR: | | | | | |
|---|---|---|---|---|---|
| CD8α signal peptide-FHVH1 sdAb--CD8α hinge/TM--4-1BB co-stimulatory molecule-CD3ζ--T2A--CSF2RA signal-tEGFR | | | | | |
| CDRs of FHVH1 sdAb: | | | | | |
| SEQ ID NO: 38 | | HCDR1 | | GFTFSHSA | |
| SEQ ID NO: 39 | | HCDR2 | | IYARGGYT | |
| SEQ ID NO: 40 | | HCDR3 | | ARGYHLEYMVSQDV | |

| FHVH3 CAR: | | | | | |
|---|---|---|---|---|---|
| CD8α signal peptide-FHVH3 sdAb--CD8α hinge/TM--4-1BB co-stimulatory molecule-CD3ζ--T2A--CSF2RA signal-tEGFR | | | | | |
| CDRs of FHVH3 sdAb: | | | | | |
| SEQ ID NO: 41 | | HCDR1 | | GGTFSNYA | |
| SEQ ID NO: 42 | | HCDR2 | | ISAYNGDT | |
| SEQ ID NO: 43 | | HCDR3 | | ARYESMSGQDI | |

| FHVH3VH1 CAR: | | | | | |
|---|---|---|---|---|---|
| CD8α signal peptide-FHVH3VH1 sdAb--CD8α hinge/TM--4-1BB co-stimulatory molecule--CD3ζ--T2A-CSF2RA signal-tEGFR | | | | | |
| CDRs of FHVH3VH1 sdAb: | | | | | |
| SEQ ID | HCDR1 | GGTFSNYA | SEQ ID | HCDR1 | GFTFSHSA |
| NO: 41 | | | NO: 38 | | |
| SEQ ID NO: 42 | HCDR2 | ISAYNGDT | SEQ ID NO: 39 | HCDR2 | IYARGGYT |
| SEQ ID NO: 43 | HCDR3 | ARYESMSGQDI | SEQ ID NO: 40 | HCDR3 | ARGYHLEYMVSQD V |

| FHVH1VH3 CAR: | | | | | |
|---|---|---|---|---|---|
| CD8α signal peptide-FHVH1 VH3 sdAb--CD8α hinge/TM --4-1BB co-stimulatory molecule--CD3ζ--T2A--CSF2RA signal—tEGFR | | | | | |
| CDRs of FHVH1VH3 sdAb: | | | | | |
| SEQ ID NO: 38 | HCDR1 | GFTFSHSA | SEQ ID NO: 41 | HCDR1 | GGTFSNYA |
| SEQ ID NO: 39 | HCDR2 | IYARGGYT | SEQ ID NO: 42 | HCDR2 | ISAYNGDT |
| SEQ ID NO: 40 | HCDR3 | ARGYHLEYMVS QDV | SEQ ID NO: 43 | HCDR3 | ARYESMSGQDI |

| FHVH2 CAR: | | | | | |
|---|---|---|---|---|---|
| CD8α signal peptide-FHVH2 sdAb--CD8α hinge/TM--4-1BB co-stimulatory molecule-CD3ζ--T2A--CSF2RA signal—tEGFR | | | | | |
| CDRs of FHVH2 sdAb: | | | | | |
| SEQ ID NO: 64 | | HCDR1 | | GFTFSSYE | |
| SEQ ID NO: 65 | | HCDR2 | | ISSSGSTI | |
| SEQ ID NO: 66 | | HCDR3 | | ARVAQREGDV | |

| FHVH4 CAR: | | | | | |
|---|---|---|---|---|---|
| CD8α signal peptide-FHVH4 sdAb--CD8α hinge/TM--4-1BB co-stimulatory molecule-CD3ζ--T2A--CSF2RA signal—tEGFR | | | | | |
| CDRs of FHVH4 sdAb: | | | | | |
| SEQ ID NO: 67 | | HCDR1 | | GYSFSNHW | |
| SEQ ID NO: 68 | | HCDR2 | | VYPGDSDT | |
| SEQ ID NO: 69 | | HCDR3 | | ARGGTIDGDYGGRQDF | |

| FHVH2VH1 CAR: | | | | | |
|---|---|---|---|---|---|
| CD8α signal peptide-FHVH2VH1 sdAb--CD8α hinge/TM--4-1BB co-stimulatory molecule--CD3ζ--T2A-CSF2RA signal-tEGFR | | | | | |
| CDRs of FHVH2VH1 sdAb: | | | | | |
| SEQ ID NO: 64 | HCDR1 | GFTFSSYE | SEQ ID NO: 38 | HCDR1 | GFTFSHSA |
| SEQ ID NO: 65 | HCDR2 | ISSSGSTI | SEQ ID NO: 39 | HCDR2 | IYARGGYT |
| SEQ ID NO: 66 | HCDR3 | ARVAQREGDV | SEQ ID NO: 40 | HCDR3 | ARGYHLEYMVSQ DV |

| FHVH4VH1 CAR: | | | | | |
|---|---|---|---|---|---|
| CD8α signal peptide-FHVH4VH1 sdAb--CD8α hinge/TM--4-1BB co-stimulatory molecule--CD3ζ--T2A--CSF2RA signal—tEGFR | | | | | |
| CDRs of FHVH4VH1 sdAb: | | | | | |
| SEQ ID NO: 67 | HCDR1 | GYSFSNHW | SEQ ID NO: 38 | HCDR1 | GFTFSHSA |
| SEQ ID | HCDR2 | VYPGDSDT | SEQ ID | HCDR2 | IYARGGYT |
| NO: 68 | | | NO: 39 | | |
| SEQ ID NO: 69 | HCDR3 | ARGGTIDGDYG GRQDF | SEQ ID NO: 40 | HCDR3 | ARGYHLEYMVSQ DV |

### Experimental purpose and principle:

All mature T cells express the CD5 antigen on the surface, while the CD5 CAR-T developed by Mamonkin M and other researchers without CD5 knockout has been reported to have a certain degree of suicide¹⁴ and has a limited duration in the patient's body, which greatly limits the potential of the patient disease remission time and application of the CAR-T product. In order to solve this problem, the present application uses CRISPR/Cas9 technology to knock out the CD5 antigen on the surface of T cells to minimize the self-activation and suicide of CD5 CAR-T, and ensure its persistence and effectiveness.

The brief experimental steps of CD5 KO T cell preparation process are as follows:
1) Prepare the sgRNA/Cas9 RNP mixture: calculate the required amount of sgRNA and Cas9 protein according to the amount of cells (1 × 10^6 T cells need to use 30 µg of Cas9 protein and 20 µg of sgRNA), mix the sgRNA and Cas9 protein gently, and co-incubate at room temperature for 15 min.
2) Gently mix the incubated sgRNA/Cas9 RNP mixture with T cells and co-incubate at room temperature for 10 min.
3) Knock off the CD5 antigen of T cells using the voltage and shock time recommended for the electroporator.
4) Place CD5 KO T cells in fresh rewarmed T cell culture medium, put them back in a 5% carbon dioxide incubator at 37°C, perform lentiviral transfection after 24 h, and detect knockout efficiency and CAR transfection efficiency from the 5th day to the 7th day after the cell state recovers.

The brief experimental steps of CD5 antigen expression detection and EGFRt expression detection of CAR-T/T cells are as follows:
1) Take 1 × 10⁶ CAR-T/T cells per well, add PBS to wash the cells once, centrifuge at 300g for 5 min, and discard the supernatant.
2) Resuspend the cell pellet in 100 µL of PBS, add 5 µL of APC-CD5 antibody and 5 µL of PE-EGFR antibody respectively, and incubate at 4°C for 15 min in the dark.
3) Wash twice with PBS and centrifuge at 300g for 5 min.
4) Resuspend with 200 µL of PBS, and detect by flow cytometry.

### Main materials and reagents:

Chemically synthesized EasyEdit sgRNA, Nanjing GenScript Biotech Co., Ltd.;
sgRNA: gctgtagaactccaccacgc (SEQ ID NO: 70);
TrueCut^{™} Cas9 Protein v2, thermo, Cat. No.A36498;
APC Mouse Anti-Human CD5 antibody, Clone UCHT2, BD Pharmingen, Cat. No.555355;
PE anti-human EGFR Antibody, Clone AY13, BioLegend, Cat. No.352904;
Fetal bovine serum (FBS), Gibco, Cat. No. 10099141.

### Experimental results:

The CAR-T studied in the present application targets the CD5 target. If the CAR-T cells function well, the CAR-T cells transfected with lentivirus can kill CAR-T/T cells that still express the CD5 antigen. In this experiment, CAR-T cells could clear the cells without CD5 knockout. In order to reduce the suicide of CD5 CAR-T cells in subsequent experiments, CD5 was knocked out by CRISPR/Cas9 technology.

As shown in Fig. 2B, the x-axis is the expression of EGFRt (that is, the indirectly represented expression of CAR), and the y-axis is the expression of CD5. Compared to MOCK T (untransfected T cells), the positive expression rate of CD5 in T cells with CD5 knockout was only 16.1%, indicating that the efficiency of CD5 knockout by CRISPR/Cas9 technology can reach more than 80%, which helps to reduce the suicide of CD5 CAR-T cells in subsequent experiments. Among them, the T cells that was not completely knocked out of CD5 and still expressed the CD5 antigen were gradually eliminated by the CD5KO CAR-T cells during the culture process, so the CD5 antigen in the CD5KO CAR-T cells was basically undetectable, proving that the CD5KO CAR-T cells have a good function in eliminating CD5+ cells.

### Example 3. In vitro function verification of CAR-T cells

### Experimental purpose and principle:

Since fully human single-domain clones and tandem clones that bind to CD5 antigen may not have good activation function after being constructed into the CAR structure, its function on CAR-T cells needs to be further confirmed, and the most active CAR molecules are to be screened. To this end, we prepared lentiviral vectors of these CAR molecular clones, and transduced T cells to prepare CAR-T cells. Then, the in vitro biological efficacy of the CAR-T cells was evaluated by CD107a degranulation assay and in vitro cytotoxicity assay. Through the functional verification of these CAR-T levels, CAR molecules with ideal efficacy and safety were finally screened for the development of downstream CAR-T product.

### CD107a degranulation assay

CD107a is a marker for intracellular microvesicles, and CD107a on the cell membrane increases after granzyme-loaded microvesicles fuse with the cell membrane, and when its recovery is blocked by monesin (purchased from BioLegend), it can quantitatively reflect the intensity of microvesicle release. When CAR-T is stimulated by the target antigen on the target cell, it will cause the release of granzymes, and the activation of T cells can be judged by the increase of CD107a by flow cytometry.

### Brief experimental steps of CD107a degranulation:

1) Verification of the CD5 expression of target cells: take 1 × 10⁶ target cells JURKAT, CCRF-CEM, CCRF-CD5 KO, MOLT-4, SUP-T1, K562, RAJI per well, add PBS to wash once, and centrifuge at 300g for 5 min, and discard the supernatant. Resuspend the cell pellet in 100 µL of PBS, add 5 µL of APC-CD5 antibody, and incubate at 4°C for 15 min in the dark. Wash twice with PBS and centrifuge at 300g for 5 min. Resuspend in 100 µL of PBS, and detect with flow cytometry.
2) Centrifuge the CAR-T cells to be tested and the target cells at 300g for 5 min at room temperature, respectively, discard the supernatant, resuspend in 1640 medium +10%FBS to 4×10⁶ cells/mL;
3) In a 96- well plate, add 100 µL of CAR-T cells to be tested and 100 µL of the target cells, and mix well;
4) Add 5 µL of PE/Cy7 mouse anti-human CD107a antibody and 0.2 µl of monensin to each well of cells, and then put them in a cell incubator (37°C, 5% CO₂) to incubate for 4 h;
5) After completion of incubation, centrifuge at 4°C and 600g for 5 min, discard the supernatant, and wash the cells twice with 200 µL of PBS;
6) Resuspend the cells with 100 µL of PBS, and add 5 µL of APC anti-human EGFR and 5 µL of BV421 anti-human CD8α antibody respectively, mix well and incubate on ice in the dark for 20 min;
7) After completion of incubation, wash the cells 3 times with 200 µL of PBS; re-suspend with 100 µL of PBS and then detect by flow cytometry.

### Main samples and reagents:

Target cells JURKAT, CCRF-CEM, CCRF-CD5 KO, MOLT-4, SUP-T1, K562, RAJI;
APC Mouse Anti-Human CD5 antibody, Clone UCHT2, BD Pharmingen, Cat. No.555355;
Fetal bovine serum, Gibco, Cat. No.10099141;
Monensin, BioLegend, Cat. No. 420701;
PE/Cy7 mouse anti-human CD107a, BD Pharmingen, Cat. No. 561348;
BV421 anti-human CD8α, Biolegend, Cat. No. 301036;
APC anti-human EGFR, BioLegend, Cat. No. 352906.

### Experimental results:

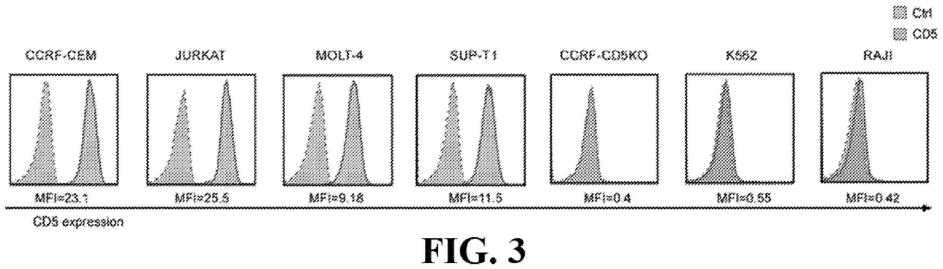

As shown in Fig. 3, the CD5 antigen expression intensity of each target cell was judged by detecting the average fluorescence intensity of target cells CCRF-CEM, JURKAT, MOLT-4, SUP-T1, CCRF-CD5 KO, K562, RAJI APC Anti-Human CD5. JURKAT, CCRF-CEM were CD5 antigen high expression cell lines, MOLT-4, SUP-T1 were CD5 antigen medium expression cell lines, andCCRF-CD5 KO, K562, RAJI were negative cell lines. CCRF-CD5 KO is obtained by knockout of CD5 from a CCRF-CEM cell line by CRISPR/Cas9 technology (the method is the same as the CD5 knockout of T cells). The knocked-out cells were diluted to the limit and then coated onto monoclonal plates. When the monoclonal cells were expanded to a detectable number, 2*10^5 CCRF-CD5 KO monoclonal cells were taken for flow cytometry, which confirmed that their CD5 expression was negative. Then the monoclonal cells could be used in experiments.

The CAR-T cells were obtained by lentiviral transduction, and CD107a degranulation assay was performed after the CAR-T cells were cultured in vitro for 9 days. The CAR-T cells to be detected were incubated with target cells, monensin and CD 107a antibody for 4 h, and the cell density of both the CAR-T cells and target cells was 4×10⁵ cells/mL. Then, after the samples were labeled with CD8 antibody and EGFR antibody, flow cytometric detection was performed. The cells were analyzed in Flowjo software, living cell gate (P1) was selected in the scatter plot, and cell debris was removed. For the cells in the P1 gate, a single scattered cell gate (P2) was selected after analysis. Then, CD8 positive cells were further selected in the P2 gate (P3). Finally, in the P3 gate, the CD107a positive proportion in the cells that were positive in EGFR antibody staining (i.e., CAR positive cells) was analyzed.

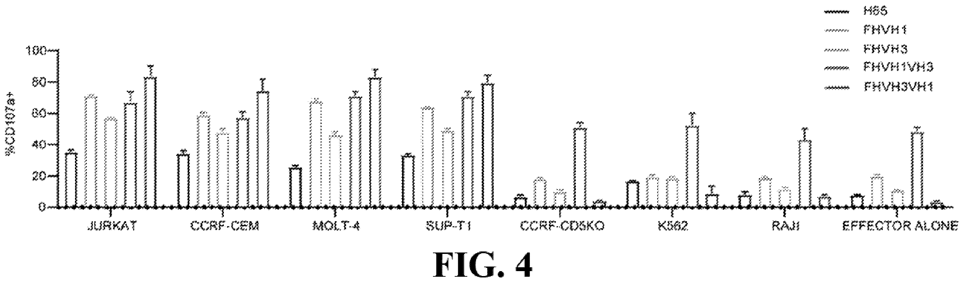

The analysis results are shown in Fig. 4, and the results show that all CAR-T cells except FHVH1VH3 could be specifically activated by CD5-positive target cells but not by CD5-negative target cells, showing good specificity. FHVH1, FHVH3, and FHVH3VH1 CAR-T cells had stronger CD107a degranulation function than control CAR-T cells (H65 CAR-T cells), whileFHVH1 VH3 CAR-T cells themselves had non-specific activation.

### In vitro cytotoxicity assay

The purpose and principle of the experiment: In vitro cytotoxicity assay uses CCRF-CEM, JURKAT, MOLT4 andSUP-T1 as CD5 positive target cells, CCRF-CD5 KO, K562 and RAJI cells as CD5 negative target cells to evaluate the antigen-specific cytotoxicity of CD5 CAR-T cells. Among them, the above cells are respectively transduced by lentivirus to obtain target cells stably expressing firefly luciferase, so the activity of luciferase in the sample can reflect the number of target cells. The CAR-T cells and target cells are co-incubated and cultured. When target cells are killed by CAR-T cells, luciferase is released and quickly inactivated (firefly luciferase has a half-life of about 0.5 h). If the target cells are not killed or inhibited by CAR-T cells, more luciferases will be produced as the target cells proliferate and continue to express luciferase. Therefore, the cytotoxicity of CAR-T on target cells can be detected by the activity of luciferase.

### Brief experimental steps of in vitro cytotoxicity assay

1) Centrifuge the above cells at room temperature and at 300g for 5 min respectively, discard the supernatant, and then re-suspended the cells in 1640+10%FBS culture medium to 2×10⁵ cells/mL; add 100 µL of target cells to each well of 96-well plates respectively;
2) According to the CAR positive rate and effector-target ratio of the CAR-T sample to be tested, add the corresponding CAR-T cells to each well of the 96-well plate respectively, well mix with the target cells, put the mixed cells in a 37°C 5% CO₂ incubator to incubate for 24 h;
3) detect the luciferase activity of the sample in each well using a luciferase detection kit.

### Main samples and reagents:

Target cells CCRF-CEM, JURKAT, MOLT4, SUP-T1, CCRF-CD5 KO, K562 and RAJI;
Steady-Glo Luciferase Assay System, Promega, Cat. No. E2520.

### Experimental results:

CAR-T cell samples and a fixed number of target cells (2×10⁴) were mixed according to different effector-target ratios (E:T), co-incubated for 24 h, and then the luciferase activity (RLU) in the samples was detected. Since the luciferase activity can reflect the number of target cells in the sample, the cytotoxicity/inhibiting ability of CAR-T cells on target cells can be obtained through the change of luciferase activity in the sample. The lower the luciferase activity readout (RLU), the more target cells are killed.

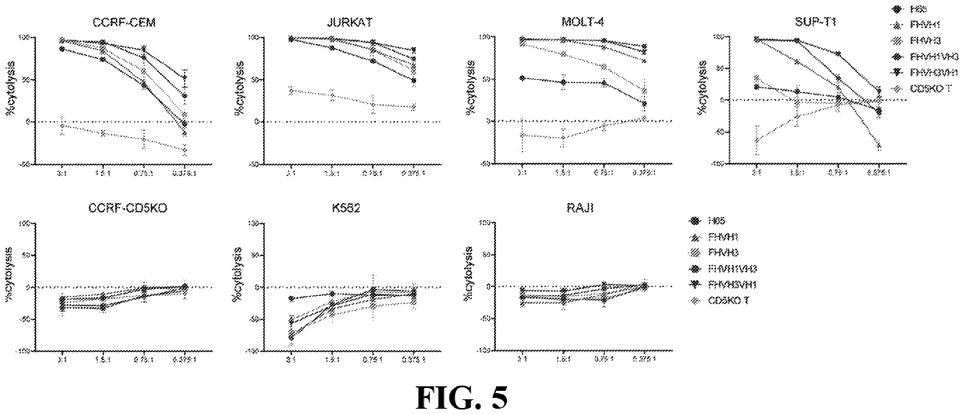

As shown in Fig. 5, the y-axis in the figure is the proportion of target cells killed, and the x-axis is the effector: target cells ratio (E:T). For all CAR-T cell samples, the cytotoxicity on positive target cells was stronger than that of control H65 CAR-T cells, and there was no obvious cytotoxicity when co-incubated with negative target cells. Therefore, CAR-T samples of FHVH1, FHVH3, FHVH1VH3, and FHVH3VH1 could specifically kill CD5-positive target cells, and had no non-specific cytotoxicity on CD5-negative target cells. With a lower effector-target ratio, especially when co-incubated with the target cells MOLT-4 and SUP-T1 with medium expression of CD5, the cytotoxicity of FHVH1VH3 and FHVH3VH1 on the target cells was stronger than that of FHVH1 and FHVH3.

### CAR-T/T cell apoptosis detection

1) Take the CAR-T cells cultured to Day 10-12, CD5 KO T and MOCK T cells, each 1 * 10^6, centrifuge at 600g at room temperature for 5 min, and wash twice with PBS.
2) Add 100 µL of binding buffer to each well for resuspension, add 10 µL of annexin V (FITC) and 5 µL of PI(PE), incubate at room temperature for 15 min, add 200 µL of binding buffer, and carry out flow cytometry.

### Main samples and reagents:

H65, FHVH1, FHVH3, FHVH1VH3, FHVH3VH1 CAR-T cells, CD5 KO T, MOCK T cells;
FITC Annexin V Apoptosis Detection Kit with PI, Biolegend, Cat. No. 640914.

### Experimental results:

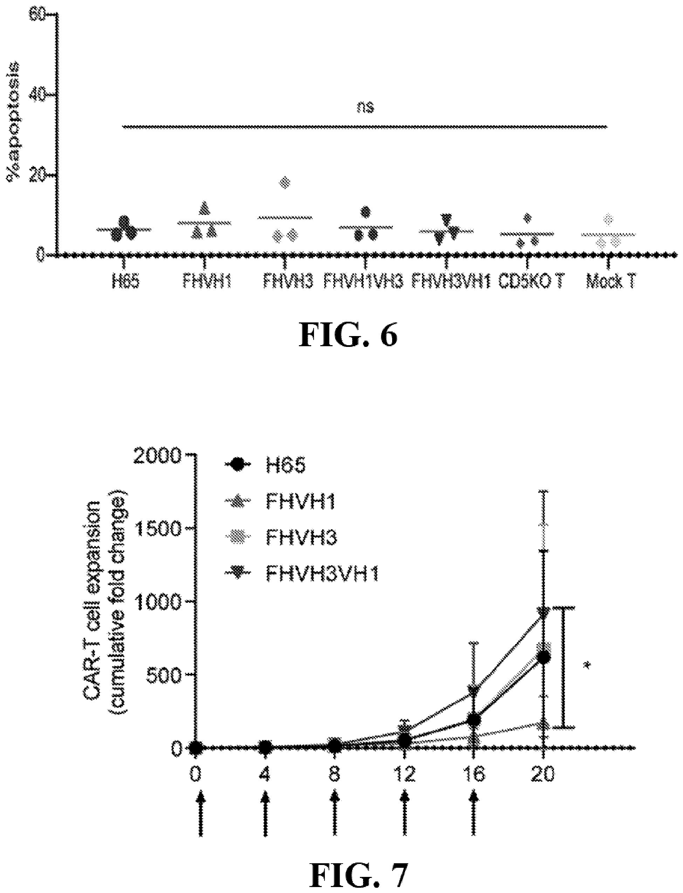

As shown in Fig. 6, there was no statistical difference in the apoptosis levels of H65, FHVH1, FHVH3, FHVH1VH3, FHVH3VH1 CAR-T cells, CD5 KO T, and MOCK T cells (3independent repeated experiments), indicating that after CD5 knockout, after 10-12 days of culture, CD5 CAR-T cells did not have obvious cell apoptosis caused by self-activation and "suicide", indicating that they may not lose function due to self-activation and "suicide", and can stably exist in the body of the patient and exert a tumor-killing effect.

### Repeated stimulation proliferation experiment

### Experimental purpose and principle:

Mitomycin C-treated target cells (CCRF-CEM) were mixed with different groups of CD5 KO CAR-T cells for multiple stimulations, and then the CAR-T cells and target cells were co-incubated to determine the ability of different CAR-T to proliferate after being repeatedly stimulated by target cells.

Brief experimental steps of repeated stimulation experiment:
1) Take CCRF-CEM cells at 7×10⁶ cells, and centrifuge at 300g at room temperature for 5 min;
2) Adjust the density to 0.2×10⁶ cells/mL with a complete medium, add 5 µL of Mitomycin C stock solution (1 µg/µL) and mix well, then incubate at 37°C and 5% CO₂ for 24 h before use.
3) Take the CCRF-CEM-Mitomycin C cells treated with Mitomycin C for24 h, centrifuge at 300g to change the medium, wash 6 times with PBS, resuspend the CCRF-CEM-Mitomycin C cells in CTS medium, count and adjust the density to 6× 10⁶ cells/mL before use.
4) Take 3×10⁵ CD5KO CAR-T cells respectively, and transfer them into 24-well plates. Add 50 µL of CCRF-CEM-Mitomycin cells to each well to make the effector-to-target ratio E: T = 1: 1. Replenish with CTS complete medium until the final culture volume reaches 500 µL, mix well, culture at 37°C, 5% CO₂ for 72 h and count, treat the target cells (CCRF-CEM) with Mitomycin again, and repeat the repeated stimulation in the above 1-4 steps and plot the amplification curve.

### Main samples and reagents:

FHVH1, FHVH3, FHVH3VH1, H65 CAR-T cells, CCRF-CEM cell lines;
Mitomycin C, STEMCELL Technologies, Cat. No. 73274;
CTS^{™} OpTmizer^{™} T Cell Expansion SFM, Gibco, Cat. No. A1048501;
Fetal bovine serum (FBS), Gibco, Cat. No. 10099141.

### Experimental results:

As shown in Fig. 7, after repeated stimulation of the 4 groups of CAR-T cell samples, their proliferation ability was ordered as follows: FHVH3VH1 CAR-T>FHVH3 CAR-T>H65 CAR-T>FHVH1 CAR-T, after 5 times of target cell stimulation, FHVH3VH1, FHVH3 andH65 CAR-T cells could still be effectively expanded. The proliferation ability of CAR-T cells stimulated by target cells is closely related to the long-term prognosis of patients. Therefore, FHVH3VH1 CAR-T and FHVH3 CAR-T can be considered to have the potential of long-term proliferation and elimination of tumor cells in vivo.

### Example 3. In vivo functional verification of CAR-T cells in mouse tumor model

### Experimental purpose and principle:

The fully human single-domain clone and tandem clone bound with CD5 antigen that are constructed into the CAR structure had been proved in the in vitro functional verification to have good functions of activating and killing tumor cells, and the ability to kill CD5+ target cells and the ability to expand upon stimulation with target cells of the fully human single-domain tandem clone FHVH3VH1 was enhanced relative to single single-domain clones. To this end, we performed an in vivo biological potency assessment of CAR-T cells. The human acute T-cell leukemia cell line CCRF-CEM-ffLuc was used to establish a tumor model, and the functional verification of CAR-T was carried out in mice to prove the effectiveness and safety of CAR-T.

### Brief experimental steps:

1) Six-week-old female NCG mice were inoculated with tumors by tail vein injection of 1 × 10⁶ CCRF-CEM-ffLuc cells on day 0.
2) On day 4 and day 7, 2 × 10⁶ and 1 × 10⁶ CD5KO CAR+ T cells, CD5KO T cells or PBS (n = 5) were infused via tail vein injection, respectively.
3) Tumor burden was assessed weekly using bioluminescent imaging, and mouse survival curves were monitored.

### Main samples and reagents:

FHVH1, FHVH3, FHVH3VH1 and H65 CAR-T cells, and human acute T-cell leukemia cell line CCRF-CEM-ffLuc cell line;
NCG severe immunodeficiency mice, GemPharmatech Co., Ltd. (Jiangsu).

### Experimental results:

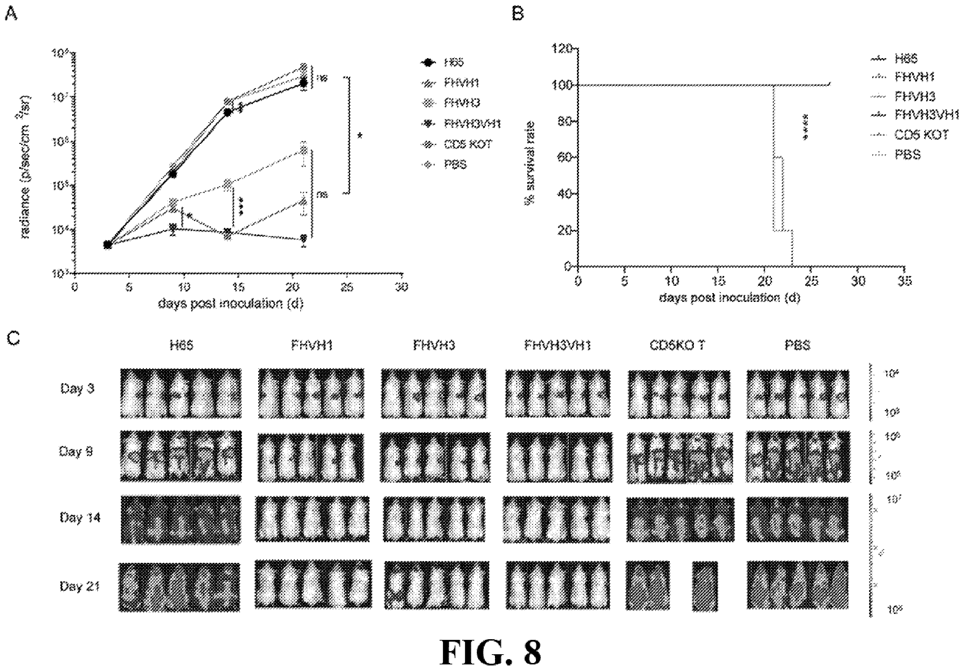

As shown in Fig. 8, FHVH1, FHVH3 and FHVH3VH1 CAR-T in the 4 groups of CAR-T cell samples could eliminate tumor cells in tumor-bearing mice, while the same dose of H65 CAR-T in mice only weakly inhibited tumor; the anti-tumor effect of FHVH1 CAR-T cells, FHVH3 CAR-T cells and FHVH3VH1 CAR-T cells in vivo was better than that of H65 CAR-T cells, while the anti-tumor effect of FHVH3VH1 CAR-T cells was stronger than that of FHVH1 and FHVH3 CAR-T cells. As shown in Fig. 8B, by Day 23, H65, FHVH1, FHVH3 and FHVH3VH1 CAR-T cells could effectively prolong the survival time of tumor-bearing mice, statistically different from that of CD5KO T cells or PBS treatment group (P< 0.0001, n = 5).

In order to further study the function of FHVH3VH1 CAR-T (hereinafter referred to as CT125A) cells, the following studies were carried out.

### Example 4 Study on binding of CT125A cells to target antigen CD5

Study purpose: To investigate the ability of CT125A cells to bind to the target antigen CD5.

Study method: After incubation of CT125A cells (2×10⁵ cells/well) with different concentrations of fluorescent dye-labeled human CD5 protein, the cells were detected by FCM (flow cytometry). The relationship between the percentage of the fluorescence-labeled CAR⁺ positive cells and the concentration of CD5 protein was fitted by Graphpad Prism software to calculate the affinity EC50 constant. This experiment was completed by Shanghai IASO Biotherapeutics Co., Ltd., and a total of three independent experiments were repeated.

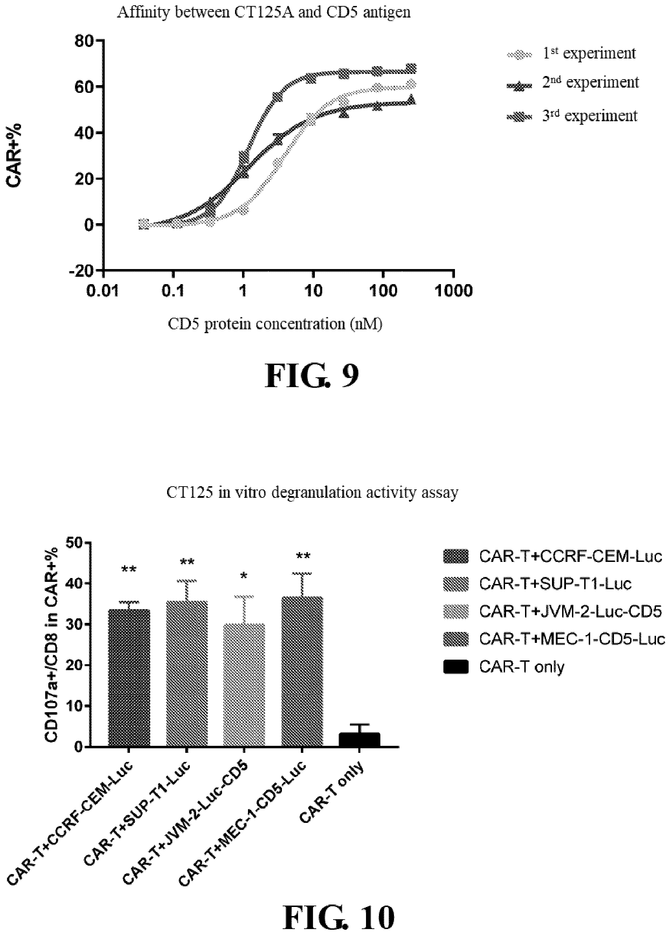

Study results: In the 3 validation batches, the apparent affinity EC50 between CT125A and human CD5 antigen was 2.07±1.03nM, and the results are shown in Table 2 and Fig. 9.

**Table 2 Summary of detection results of affinity between CT125A and human CD5 antigen**

| **Cell name** | **Group** | **Number of times of experiments** | **Apparent affinity EC₅₀ (nM)** | **Average (nM)** | **Standard deviation** |
|---|---|---|---|---|---|
| CT125A injection | CD5 Protein | 1st time | 3.82 | 2.07 | 1.23 |
| | | 2nd time | 1.22 | | |
| | | 3rd time | 1.18 | | |

Study conclusion: CT125A cells have stable, good and specific binding ability to human CD5 antigen.

### Example 5 Study on degranulation activity of CT125A in vitro

Study purpose: To evaluate the degranulation activity of CT125A under specific stimulation by positive target cells.

Study protocol: Flow cytometry was used to detect the translocation of CD 107a molecules on the membrane surface of 3 batches of CAR-T cells. The cytoplasm of CAR-T cells contains a high concentration of cytotoxic granules in the form of vesicles. When CAR-T cells are co-incubated with target cells, the toxic granules will reach the serosal surface and fuse with the cell membrane, causing the release of the granule contents, eventually leading to the death of the target cells. With the occurrence of degranulation, CD107a molecules are transported to the surface of the cell membrane and can bind to CD107a antibody. The expression of CD107a was quantitatively analyzed by the FCM method to evaluate the degranulation function of CT125A. This experiment was completed by Shanghai IASO Biotherapeutics Co., Ltd., and a total of three independent experiments were repeated.

Study results: After incubation of CT125A with 4 lines of CD5-positive target cells, the degranulation reaction was significantly increased, and the positive rates of CD107a were 33.22%±3.18% (CCRF-CEM-Luc), 35.43±7.33nM (SUP-T1-Luc); 29.71±10.01nM (JVM-2-Luc-CD5); 36.38±8.56nM (MEC-1-CD5-Luc), respectively; in the group without the target cell, the positive rate of CD107a was only 3.07%±3.34%. The data are detailed in Table 3 and Fig. 10.

**Table 3 The degranulation activity of CT125A stimulated by different kinds of positive target cells**

| **Name** | **Cell group** | **Number of times of experiments** | **Proportion of CD107a⁺ positive in CD8⁺CAR⁺ population (%)** | **Average (%)** | **Standard deviation** |
|---|---|---|---|---|---|
| CD107a | CAR-T+CCRF-CEM-Luc | 1st time | 32.14 | 33.22 | 3.18 |
| | | 2nd time | 37.54 | | |
| | | 3rd time | 29.99 | | |
| | CAR-T+SUP-T1-Luc | 1st time | 36.54 | 35.43 | 7.33 |
| | | 2nd time | 43.80 | | |
| | | 3rd time | 25.95 | | |
| | CAR-T+JVM-2-Luc-CD5 | 1st time | 21.88 | 29.71 | 10.01 |
| | | 2nd time | 43.84 | | |
| | | 3rd time | 23.41 | | |
| | CAR-T+MEC-1-CD5-Luc | 1st time | 30.59 | 36.38 | 8.56 |
| | | 2nd time | 48.47 | | |
| | | 3rd time | 30.07 | | |
| | CAR-T only | 1st time | 0.93 | 3.07 | 3.34 |
| | | 2nd time | 0.50 | | |
| | | 3rd time | 7.78 | | |

Study conclusion: After co-incubating CT125A cells with the 4 positive target cell lines, the positive rate of CD107a was significantly increased compared to CT125A cells themselves. This result indicates that CT125A cells had stable, specific and good degranulation activity.

### Example 6 Study on CT125A cytotoxicity in vitro

Study purpose: To evaluate the cytotoxicity of CT125 injection on positive target cells in vitro.

Study method: CAR-T cells and target cells were co-incubated and cultured according to different effector-to-target ratios (from 10:1 to 0.5:1, 2-fold dilution). When target cells are killed by CAR-T cells, luciferase is released and quickly inactivated. If the target cells are not killed or inhibited by CAR-T cells, more luciferases will be produced as the target cells proliferate and continue to express luciferase. Therefore, the cytotoxicity of CAR-T on target cells can be detected by the activity of luciferase.

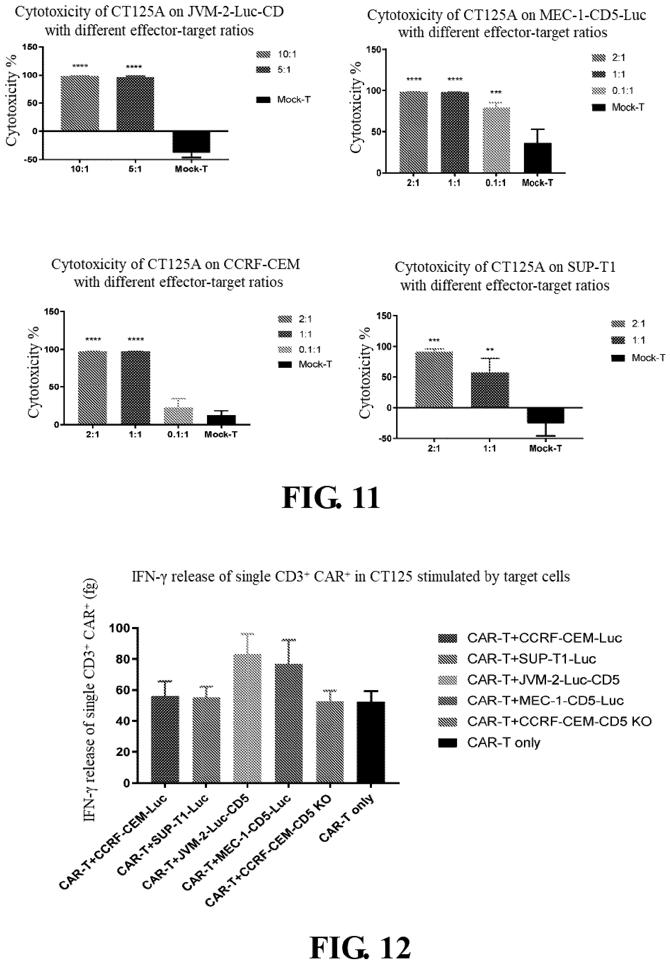

Study results: Compared to the MOCK-T group, CT125A had significant cytotoxicity on the 4 positive target cell lines for the first two effector-target ratios in the three effector-target ratios (high, medium, low) measured, which had a dose-effect relationship: for CCRF-CEM-Luc cells, the cytotoxicity when the effector-target ratio was 2:1 and 1:1 was respectively 96.91% and 95.58%; for SUP-T1-Luc cells, when the effector-target ratio was 2:1 and 1:1, the cytotoxicity was respectively 91.04% and 56.94%; for JVM-2-Luc-CDS cells, the cytotoxicity when the effector-to-target ratio was 10:1 and 5:1 was respectively 98.51% and 96.40%; and for MEC-1- CD5-Luc cells, when the effector-to-target ratio was 2:1 and 1:1, the cytotoxicity was 98.25% and 97.71%, respectively. See Table 4 and Fig. 11 for specific data.

**Table 4 Cytotoxicity of CT125A on different kinds of positive target cells**

| **Cell group** | **Number of times of experiments** | **CT125A cytotoxicity (%)** | | | **Mock-T** |
|---|---|---|---|---|---|
| | | **2 (or 10)** | **1 (or 5)** | **0.1 (or 0.5)** | **2 (or 10)** |
| CAR-T+CCRF-CEM- Luc | 1st time | 96.84 | 95.33 | 18.69 | 11.70 |
| | 2nd time | 96.80 | 95.16 | 12.47 | 6.46 |
| | 3rd time | 97.08 | 96.26 | 36.32 | 18.76 |
| CAR-T+SUP-T1-Luc | 1st time | 85.51 | 33.96 | -702.95 | -13.83 |
| | 2nd time | 92.33 | 56.33 | -509.90 | -12.36 |
| | 3rd time | 95.29 | 80.52 | -345.02 | -48.32 |
| CAR-T+JVM-2-Luc- CD5 | 1st time | 98.40 | 94.01 | -139.47 | -42.54 |
| | 2nd time | 98.61 | 97.37 | -71.61 | -42.15 |
| | 3rd time | 98.52 | 97.81 | -47.83 | -27.56 |
| CAR-T+MEC-1-CD5- Luc 3rd | 1st time | 98.13 | 97.46 | 74.18 | 26.40 |
| | 2nd time | 98.07 | 97.53 | 76.83 | 27.00 |
| | time | 98.56 | 98.13 | 85.98 | 55.22 |

Study conclusions: CT125A cells had stable, specific and good cytotoxicity on the 4 positive target cell lines assayed in vitro.

### Example 7 Study on the expression of γ interferon of CT125A injection

Study purpose: To study the expression level of Interferon-γ (IFN-γ) secreted by CT125A injection after co-incubation with target cells, so as to understand the cytotoxicity of this injection on tumor cells.

Study method: The CBA (Cytometric Bead Array, flow microsphere chip technology) method was used to detect the secretion level of IFN-γ expression in the supernatant after co-incubation of different types of CD5 positive target cells and CT125 injection.

Study results: Compared to only CAR-T cell group: under the stimulation of positive target cells CCRF-CEM-Luc and SUP-T1-Luc, the release of IFN-γ was slightly increased; under the stimulation of positive target cells JVM-2-Luc-CDS and MEC-1-CDS-Luc, the release of IFN-γ was significantly increased; and the release of IFN-γ was not significantly changed under the stimulation of negative target cells CCRF-CEM-CDS KO. See Fig. 12 for specific experimental results.

Study conclusion: For CT125A, the average release of IFN-γ tended to increase under the stimulation of the 4 positive target cell lines, but without statistically significant difference. The release of IFN-γ did not change significantly under the stimulation of one negative target cell line.

### Example 8 Study on cetuximab-mediated elimination of CT125A cells by natural killer cells in vitro

Study purpose: To investigate whether EGFRt, a molecular switch on the surface of CT125A cells, can mediate the elimination of CT125A cells by natural killer cells under the action of cetuximab.

Study method: The survival rate of CAR⁺ cells under the action of NK with different concentrations of cetuximab was mainly investigated.

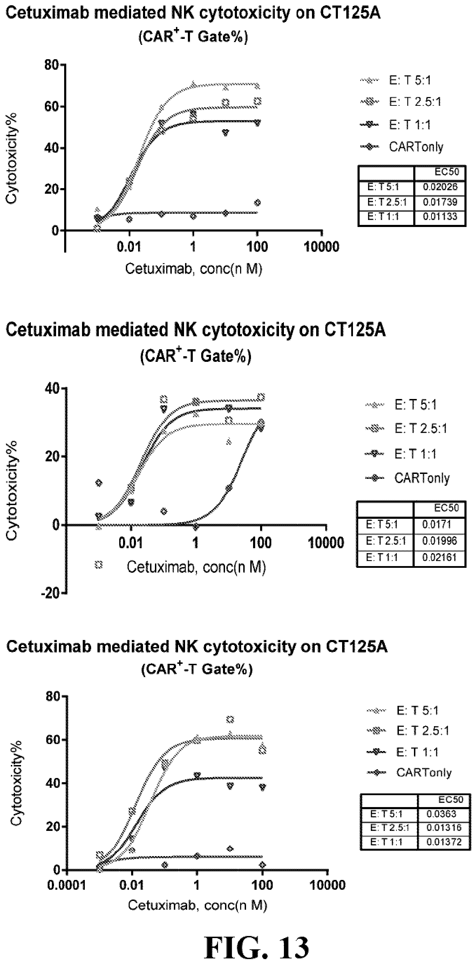

Study results: The cytotoxicity data of NK from three test subjects showed that cetuximab could effectively kill CT125A. The three tested effector-target ratios were all similarly effective, and the cytotoxicity was dose-dependent with the antibody. The EC50 was between 0.01-0.03 nM, and the maximum cytotoxicity was 30%-70%. See Fig. 13 for specific experimental results.

Research conclusion: Cetuximab can mediate the cytolysis and elimination of CT125A by NK cells in vitro, which proves that the molecular switch mechanism of EGFRt-CART is effective. It is consistent with the reported mechanism of action of cetuximab: antibody-dependent cell-mediated cytotoxicity (ADCC), antibody-dependent cellular phagocytosis (ADCP), and antibody-dependent complement-mediated cytotoxicity (CDC). Some test subjects showed that CT125A had suicide phenomenon at high concentrations of cetuximab, which may be related to the presence of a small amount of CAR-NK and CAR-NKT in CT125A preparations (some NKTs had low CD16 expression).

### Example 9 Study on the effect of CT125A on immunodeficient mice bearing SUP-T1-Luci transplanted tumors

Study purpose: In this experiment, the test product CT125A injection was administered intravenously to NOG mice transplanted with human T-lymphoblastomas cells (SUP-T1-Luci) (NOD-Cg.Prkdc^{SCID} IL-2rg^{tm1sug}/JicCrl mice, purchased from Beijing Vital River Laboratory Animal Technology Co., Ltd.) to evaluate its inhibitory effect on tumor cell proliferation.

Study method: 65 female NOG mice were inoculated with 0.2 mL of human T-lymphoblastoma cell (SUP-T1-Luci) suspension in the tail vein under sterile conditions (cell density 5.33×10⁶ cells/mL, cell viability 100.00%). On Day 4 after inoculation, 50 animals with suitable tumor signals and similar body weight were selected and randomly divided into 5 groups: cytoprotective solution group (10 animals), Mock-T group (10 animals, 9.87×10⁶ T-cells/animal), the low-dose group of the test product (10 animals, 0.3×10⁶ CAR-T cells/animal), the medium-dose group of the test product (10 animals, 1.0×10⁶ CAR-T cells/animal) and the high-dose group of the test product (10 animals, 3.0×10⁶ CAR-T cells/animal). All animals were administered with a single injection through the tail vein, the day of administration was regarded as D1, the second day after administration was regarded as D2, and so on.

General clinical observations were carried out twice a day after the first administration. Before grouping, the animals were weighed once on each of D3, D7, D11, D14, D18, D21 and D23. Before grouping, on D7, D14, D21 and D23, for all the animals, chemiluminescence signals were captured by a small animal in vivo imager. On each of D2, D3, D5, D7 and D23, lymphocyte subsets (CD45⁺, CD45⁺CD3⁺ and CD45⁺CD3⁺CD5⁺), and peripheral blood cytokines (IL-2, IL-4, IL-6, IL-10, TNF-α and IFN-γ) were detected once.

### Study results: 1) General clinical observation and survival rate

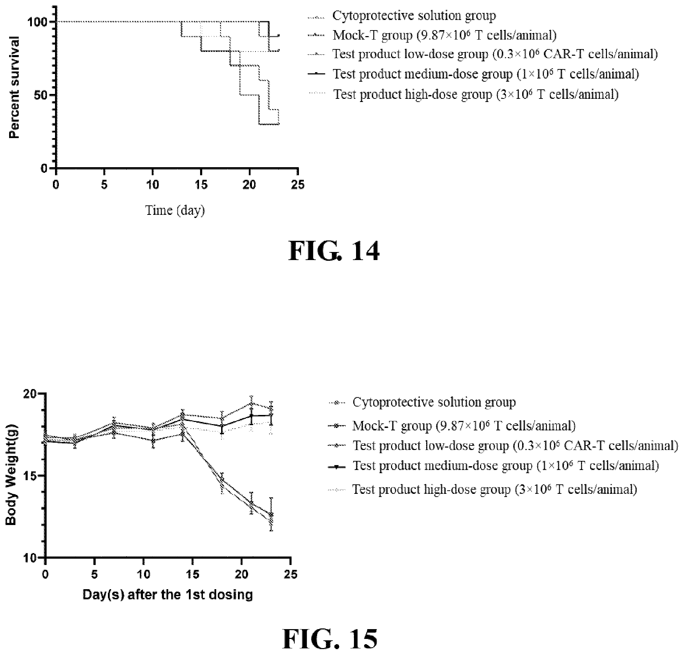

During the experiment, animals in the cytoprotective solution group began to die on D17, and the clinical manifestations before death included bowed back and/or weakness of hind limbs, and 3 animals survived on D23. Animals in the Mock-T group began to die on D13. The clinical manifestations before death included bowed back, weakness of hind limbs, tilted head and lack of energy. On D23, 3 animals survived. Animals in the low, medium and high dose groups of the test product began to die on D21, D15 and D18 respectively, and 8, 9 and 8 animals survived on D23 respectively. The remaining animals were euthanized on D23. See Fig. 14 for specific results.

### 2) Body weight

After dosing, each group of the test product showed a steady upward trend. The body weight of the animals in the cytoprotective solution group and the Mock-T group first increased and then decreased. Compared to the cytoprotective solution group, the body weight of the animals in the Mock-T group had no significant difference during the experiment, and the body weight in the low, medium and high dose groups of the test product was significantly higher than in the cytoprotective solution group from D18 (P≤0.05). The results are shown in Fig. 15.

### 3) Tumor cell proliferation (tumor cell bioluminescent intensity):

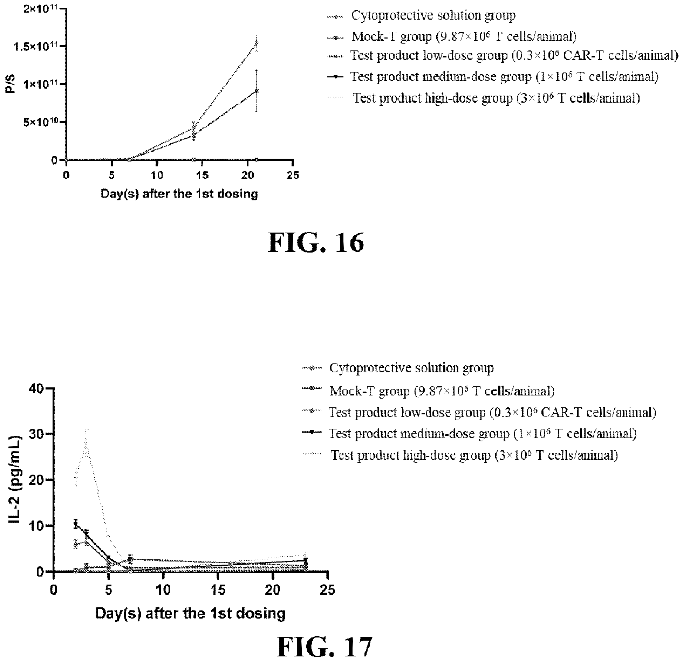

After dosing, the tumor signal intensity in the cytoprotective solution group and the Mock-T group continued to increase. The tumor signal intensity in the low, medium and high dose groups of the test product continued to decrease. Compared to the cytoprotective solution group, there was no significant difference in tumor signal intensity in the Mock-T group during the experiment. The tumor signal intensity in each group of the test product decreased significantly from D7 (*P*≤0.05), and the weakening of the tumor signal intensity decreased with the increase of the test product dose, and there was an obvious dose-dependent relationship. The results are shown in Fig. 16.

### 4) Flow cytometry

During the experiment, the ratio of peripheral blood CD45⁺ lymphocytes in the low-dose group of the test product was basically stable, and it was 0.4±0.3% on D23. In the cytoprotective solution group, Mock-T group, test product medium and high dose groups, it fluctuated slightly during D2-D7, and increased by D23. The ratio of peripheral blood CD45⁺ lymphocytes in each group of the test product showed a significant dose-dependent increase during the experiment.

During the experiment, the ratio of peripheral blood CD45⁺CD3⁺ lymphocytes in the cytoprotective solution group decreased in a fluctuating manner. The Mock-T group showed a stable trend. The low, medium and high dose groups of the test product all showed a trend of decreasing first and then increasing.

During the experiment, the ratio of peripheral blood CD45⁺CD3⁺CD5⁺ lymphocytes in the cytoprotective solution group showed an overall upward trend. The Mock-T group showed a fluctuating upward trend. The low, medium and high dose groups of the test product all showed a trend of increasing first and then decreasing.

### 5) Cytokine detection

In the cytoprotective solution group, the peripheral blood IL-2 content was basically stable, and rose slightly to 0.36±0.09 pg/mL by D23. The change of peripheral blood IL-2 content was basically stable in the Mock-T group. The low, medium and high dose groups of the test product all showed an overall trend of decreasing. The results are shown in Fig. 17.

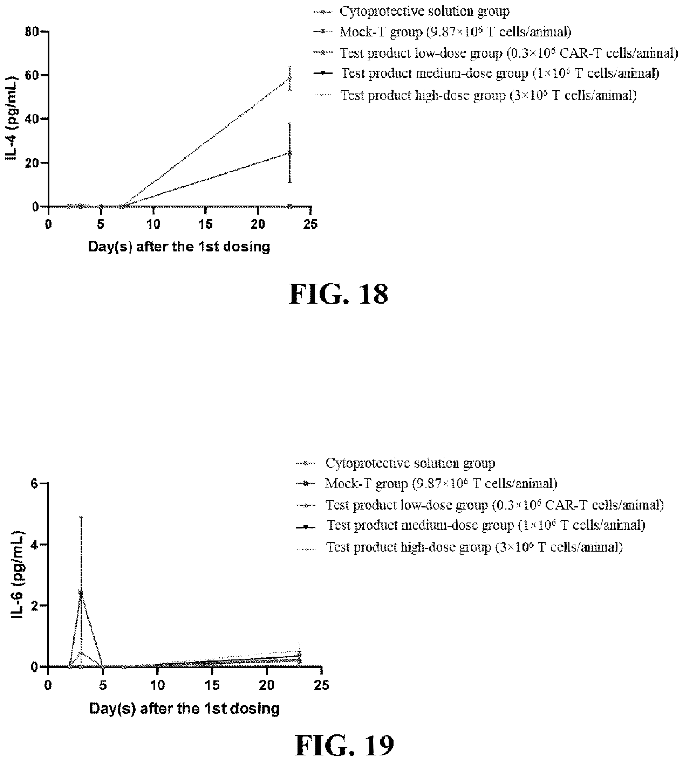

During the experiment, the peripheral blood IL-4 content in the cytoprotective solution group increased from 0.00±0.00 pg/mL on D2 to 58.55±9.42 pg/mL on D23. The peripheral blood IL-4 content in the other groups basically remained unchanged and remained stable at around the baseline of 0.00±0.00 pg/mL. The IL-6 content in the peripheral blood of animals in all groups was basically stable around the baseline of 0.00±0.00 pg/mL. The results are shown in Figs. 18-19.

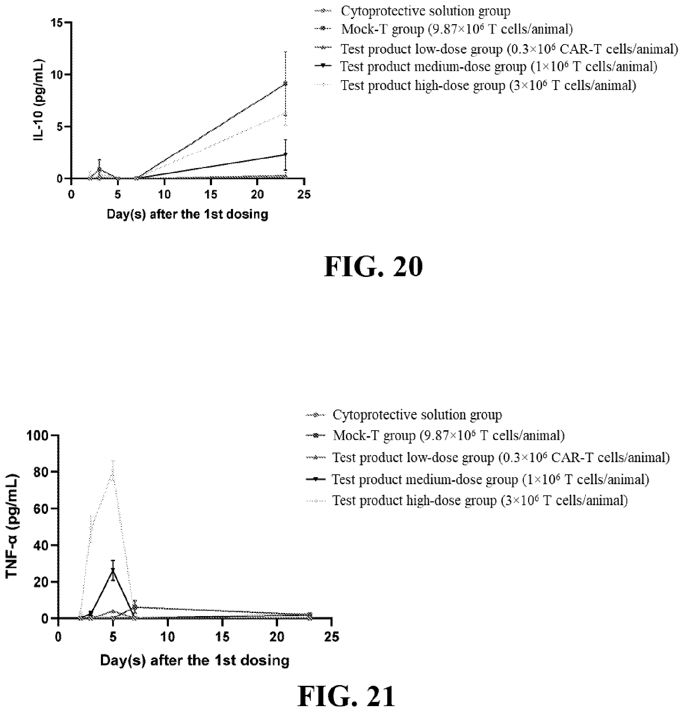

During the experiment, the levels of peripheral blood IL-10 in the cytoprotective solution group and the Mock-T group fluctuated slightly, and were basically stable near the baseline. In the high dose group of the test product, it increased slightly from 0.35±6.29 pg/mL onD2 to 6.29±3.38 pg/mL on D28. The peripheral blood IL-10 content in the other groups was basically stable around the baseline, with no obvious trend of change. The results are shown in Fig. 20.

During the experiment, the peripheral blood TNF-α content in the cytoprotective solution group rose slightly to 0.29±0.26 pg/mL on D23. In the Mock-T group, it increased first and then decreased. In the low, medium and high dose groups of the test product, it increased until D5, and then decreased, all decreased to 0.00±0.00 pg/mL on D7, and then slightly increased on D23, showing a fluctuating change. The peripheral blood TNF-α content in each group of the test product had an obvious dose-dependent increase from D2 to D5 and on D23, suggesting that the changes of peripheral blood TNF-α content in each group of the test product may be dose-dependent. The results are shown in Fig. 21.

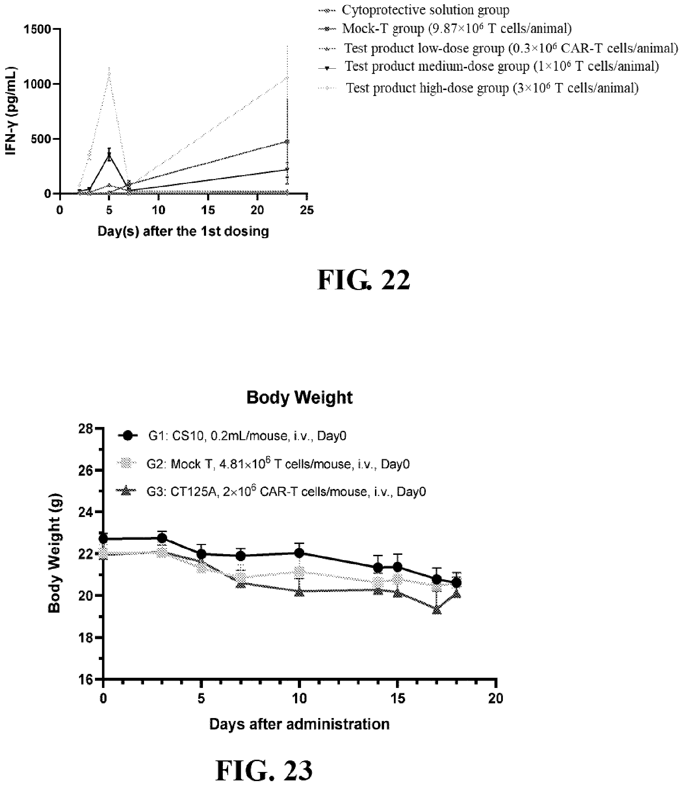

During the experiment, the peripheral blood IFN-γ content in the cytoprotective solution group was basically stable at 0.00±0.00 pg/mL. The Mock-T group showed a continuous rising trend. In the test product low dose group, it increased on D5, then dropped until D23. In the medium and high dose groups of the test product, it rose until D5, then dropped until D7, and then rose until D23. The peripheral blood IFN-γ content in each group of the test product had an obvious dose-dependent increase at every time point, suggesting that the changes of peripheral blood IFN-γ content in each group of the test product may be dose-dependent. The results are shown in Fig. 22.

**Conclusion:** This experiment successfully established the NOG mouse vein xenograft tumor model of human T-lymphoblastoma cells (SUP-T1-Luci). Under the conditions of this experiment, a single intravenous injection of the test product CT125A injection at doses of 0.3×10⁶, 1.0×10⁶ and 3.0×10⁶ CAR-T cells/animal had inhibitory and clearing effects on the growth of NOG mouse vein transplanted tumors of human T lymphoblastoma SUP -T1-Luci cells, and the tumor inhibiting and clearing effects were enhanced with the increase of the dose of the test product, suggesting an obvious dose-dependent relationship.

### Example10 Pharmacodynamic experiment of CT125A on NPG mouse systemic xenograft tumor model of human mantle cell lymphoma JVM-2-Luc-CD5 cell line

Study purpose: To study the anti-tumor efficacy of CD5-targeting CAR-T cell injection CT125A on NPG mice systemic xenografts of CD5-positive human mantle cell lymphoma JVM-2-Luc-CDS cells.

### Study method:

### 1) Cell culture

The human mantle cell lymphoma JVM-2-Luc-CDS cell suspension used in this experiment was self-made by the inventor. Commercially available JVM-2 cells were utilized to overexpress Luc to construct the JVM-2-Luc cell line, and then CD5 was expressed on the JVM-2-Luc cell line to obtain JVM-2-Luc-CDS cells. Cells were collected and counted, resuspended in PBS, and inoculated into the tail vein of 50 NPG mice. Each mouse was inoculated with 3×10⁶ JVM-2-Luc-CD5 cells. The cells were resuspended in PBS and inoculated into the mice by tail vein injection with an inoculation volume of 200 µL.

### 2) Animal grouping and dosing regimen

On Day 4 after inoculation, the mice will be randomly divided into 3 groups with 10 mice in each group according to body weight and tumor size (fluorescence intensity) using E-workbook. The grouping and dosing regimen are shown in Table 5. The injection of CAR-T cells was performed on the day of grouping, and the day of grouping and administration was defined as Day 0.

**Table 5. Grouping and dosing regimen**

| **Group** | JVM-2-Luc | **N** | **Drug** | **Dosage (CAR⁺ cells/mouse)** | **Dosing regimen** |
|---|---|---|---|---|---|
| 1 | 3×10⁶ | 10 | CS10 | - | i.v., Day 0 |
| 2 | | 10 | Mock T | The total cell number is the same as CT125A | i.v., Day 0 |
| 3 | | 10 | CT125A | 2×10⁶ | i.v., Day 0 |

| | | | | | |
|---|---|---|---|---|---|
| Note: 1. N represents the number of animals in each group; 2. Administration volume: The administration volume was adjusted according to 200 µL/animal. 3. CT125A cells are as mentioned above, CS10 is commercially available. | | | | | |

### 3) Drug formulation method

Drug formulation method: Four tubes of CT125A were taken out from the cryopreservation place, quickly placed in a 37°C water bath, shaken gently until the cells were just completely thawed, and then cells were aseptically taken out in a biological safety cabinet. The CT125A cells were transferred to a suitable sterile container, mixed upside down and then an appropriate amount of cells were taken to count the cell density and viability. CT125A viable cell density was 2.58×10⁷/mL (viability rate 85.52%), CAR+ (41.59%) viable cells were 1.07×10⁷/mL, a total of 4 mL, then CS10 was added to dilute to the required density and volume to 4.28 mL. The same method was used to adjust the Mock T cell density to be consistent with the CT125A total T-cell density. The prepared cell suspension was placed on wet (crushed) ice and passed into the animal room for administration to the animal, and the administration was completed within 2 h after resuscitation.

### 4) Evaluation method

**Cage side observation:** The appearance and behavior of each mouse were observed every day, starting from the beginning of grouping to the end of the experiment. All abnormal appearances and behaviors were recorded in the clinical observation form of the PharmaLegacy lab.

**In vivo imaging:** before grouping (Day 0), and Day 5, Day 10, Day 15 and Day 18 after grouping, a total of 4 times of imaging was performed. Before imaging, the mice were maintained under anesthesia with 3 - 4% isoflurane.

**Animal body weight:** After grouping, the body weight of the mice was measured and recorded twice a week until the end of the experiment.

**Clinical observation of animals:** observations were made once a day during the experiment, and the observations included but not limited to the mental state, diet of the animals, and the like. All abnormal appearances and behaviors were recorded in the clinical observation form of the PharmaLegacy lab and fed back to the client in time. Whether there was an allergic reaction after dosing, whether ascites occurred, etc. should be observed after dosing. If there was any abnormality or death, it needed to be recorded and reported.

**Sample collection:** On Day 7 after grouping, about 100 µL of whole blood was collected from each mouse and stored at -80°C; at the end of the experiment on Day 18, about 300 µL of whole blood was collected from each mouse and stored at -80°C.

### 5) Euthanasia

During the experiment, if any one or more of the following situations occur, the animal should be euthanized:
1) Health status: Severe emaciation and body score (according to SOP PL-ONC023) less than 2.
2) Impaired animal function: Nodules interfere with normal animal function (such as eating, drinking, or ambulating).
3) Other disease symptoms.

Animals displaying the above symptoms would be recorded as "abnormal".

At the end of the in vivo experiment on Day 18, all mice were asphyxiated with CO₂ and then sacrificed by dislocation of the cervical spine. Before the end of the in vivo experiment, dead animals would not be subject to sample collection.

### 6) Statistical analysis

Results would be presented as mean ± S.E.M. The body weight and tumor fluorescence intensity of the mice in each group were plotted and statistically analyzed using the statistical software GraphPad Prism8 for Windows (serial number: GPS-1766552-EDSH-0204F). After performing Log transformation on the tumor fluorescence intensity (if there is zero value data, it is taken as 1 and then subjected to Log transformation), Two-way ANOVA (mixed model) and Turkey test were used to determine whether there was a significant difference in the fluorescence signal value between the groups, and P<0.05 was considered significant difference.

### Study results:

### 1) Animal weight and status

During the experiment, some animals in the CS10 control group and the CT125A group lost more than 15% of their body weight or died. Of the 10 mice in the CS10 group, 2 mice died, and in the CT125A group, 1 mouse died. On Day 18, the average weight loss rate of the CS10 control group was 8.96%, and the average body weight loss rate of the CT125A group was 8.64%, indicating that the model itself could cause moderate weight loss of animals, while CT125A had no effect on animal body weight, without obvious toxicity. See Fig. 23 for the body weight changes of the animals at each time point in each group, and Table 6 for the body weight statistical data at each time point in each group.

**Table 6. Body weight statistical table of each group at each time point (g)**

| Group | | Days after grouping | | | | | | | | |
|---|---|---|---|---|---|---|---|---|---|---|
| | | 0 | 3 | 5 | 7 | 10 | 14 | 15 | 17 | 18 |
| G1: CS10, 0.2 mL/mouse, i.v., Day 0 | Mean | 22.72 | 22.75 | 21.98 | 21.89 | 22.03 | 21.35 | 21.38 | 20.78 | 20.62 |
| | SEM | 0.26 | 0.33 | 0.47 | 0.36 | 0.48 | 0.57 | 0.60 | 0.54 | 0.50 |
| G2: Mock T, 4.81×10⁶ T cells/mouse, i.v., Day 0 | Mean | 22.03 | 22.04 | 21.32 | 20.86 | 21.16 | 20.63 | 20.80 | 20.47 | 20.61 |
| | SEM | 0.40 | 0.50 | 0.51 | 0.61 | 0.92 | 0.69 | 0.52 | 0.41 | 0.43 |
| G3: CT125A, 2×10⁶ CAR-T cells/mouse, i.v., Day0 | Mean | 21.95 | 22.07 | 21.62 | 20.61 | 20.21 | 20.29 | 20.15 | 19.35 | 20.12 |
| | SEM | 0.30 | 0.35 | 0.35 | 0.61 | 0.61 | 0.79 | 0.84 | 0.87 | 0.76 |

### 2) Tumor growth

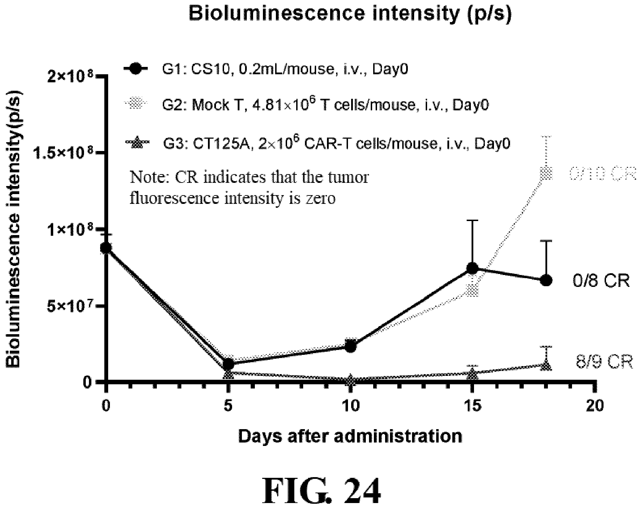

Four days after inoculation of human mantle cell lymphoma JVM-2-Luc-CDS cell line, dosing was performed in groups. The day of dosing was regarded as Day 0. Fluorescence imaging was performed on Day 5, Day 10, Day 15 and Day 18 during the experiment, and statistical analysis was performed on the tumor growth in animals in each group. See Fig. 24 and Table 7 for the tumor growth and statistical data of animals in each group at each time point. Fluorescent imaging photos of the animals are in the appendix.

During the experiment, the tumor fluorescence intensity of mice in the vehicle CS10 group (G1) decreased first and then increased; the tumor fluorescence intensity was 8.77×10⁷±0.88×10⁷ p/s on Day 0 of grouping; the tumor fluorescence intensity decreased to 1.19*×10⁷±0.17×*10⁷ p/s on Day 5; after that, the tumor fluorescence intensity maintained an upward trend; by Day 18, the fluorescence intensity of the control group animals was 6.65×10⁷±2.57×10⁷ p/s. The results showed that this study successfully established a systemic xenograft model of human mantle cell lymphoma JVM-2-Luc-CDS cells in NPG mice.

Animals in the G2 group were given a single dose of Mock T cells at 4.81×10⁶ T-cells/mouse on Day 0, and the tumor fluorescence intensity was regularly detected. The data showed that the trend of the tumor fluorescence intensity of animals in the Mock T group (G2) was similar to that of the control group, and the tumor fluorescence intensity was 1.36×10⁸±2.44×10⁷ p/s by Day 18; the fluorescence intensity of tumor at each time point was not significantly different from that of the vehicle control group CS10.

Animals in group G3 were given a single dose of CT125A at 2×10⁶ CAR+ cells/mouse on Day 0, and then the tumor fluorescence intensity was regularly detected. The data showed that CT125A group (G3) had significantly lower tumor fluorescence intensity at each time point after dosing compared to the day of grouping, and the tumor fluorescence intensity on Day 0 was 8.77×10⁷±9.08×10⁶ p/s, and the tumor fluorescence intensity was 1.15×10⁷±1.15 × 10⁷ p/s by Day 18. The tumor fluorescence intensity at each time point in the CT125A group was significantly lower than that in the CS10 control group or Mock T group during the same period (P< 0.01, see Table7). And at the end of the experiment, 8 of 9 surviving mice in the CT125A group had zero tumor fluorescence intensity, while none of the surviving animals in the CS10 control group and Mock T group had tumor fluorescence Intensity of zero.

**Table 7. Tumor growth in each group at each time point (×10⁷, Bioluminescence intensity, p/s)**

| Group | | Days after grouping | | | | |
|---|---|---|---|---|---|---|
| | | **0** | **5** | **10** | **15** | **18** |
| G1: CS10, 0.2 mL/mouse, i.v., DayO | Mean | 8.77 | 1.19 | 2.32 | 7.46 | 6.65 |
| | SEM | 0.88 | 0.17 | 0.40 | 3.13 | 2.57 |
| G2: Mock T, 4.81×10⁶ T cells/mouse, i.v., DayO | Mean | 8.75 | 1.42 | 2.49 | 5.98 | 13.63 |
| | SEM | 0.92 | 0.15 | 0.33 | 0.98 | 2.44 |
| | *P Value (v.s.* G1) | >*0.9999* | *0.5050* | *0.9137* | *0.9012* | *0.3666* |
| | *P Value (v.s.* G3) | *0.9997* | ***0.0035*** | ***0.0014*** | ***0.0013*** | ***<0.0001*** |
| G3: CT125A, 2×10⁶ CAR-T cells/mouse, i.v., DayO | Mean | 8.76 | 0.60 | 0.17 | 0.58 | 1.15 |
| | SEM | 0.91 | 0.11 | 0.10 | 0.51 | 1.15 |
| | *P Value (v.s.* G1*)* | 0.9997 | **0.0234** | **0.0014** | **0.0014** | **0.0001** |
| | *P Value (v.s.* G2) | *0.9997* | ***0.0035*** | ***0.0014*** | ***0.0013*** | ***<0.0001*** |

### Conclusions

In summary, under the conditions of this experiment, a single intravenous injection of CT125A injection at a dose of 2×10⁶ CAR-T cells/mouse had a strong inhibitory and clearing effect on the growth of NPG mouse systemic xenograft tumors of human mantle cell lymphoma JVM-2-Luc-CD5 cells, with good animal tolerance.

### Example11 Human membrane protein array study

Study purpose: To investigate the non-specific cross-reactivity of CT125A tandem single-domain antibody rabbit FC fusion protein RD125 61-42 rFc (constructed by fusing the above FHVH3VH1 with rFc) with human membrane proteins

Study method: A membrane protein array platform (Membrane Proteome Array, MPA) composed of about 6,000 different human membrane protein clones from Integral Molecular Company of the United States was used to detect the binding between CT125A single-chain antibody rabbit FC fusion protein RD125 61-42 rFc and cells expressing human membrane protein clones, positive bound cells were screened out, and the results were confirmed for the second time.

Study results: In the screening test, at a concentration of 5 µg/mL, RD125 61-42 rFc had a strong signal of binding with cells expressing human CD5 clones, and weakly bound to a small number of cells expressing human membrane proteins. In the second concentration gradient confirmation test, it was confirmed that RD125 61-42 rFc had dose-dependent strong binding to human CD5 membrane expressing cells, and weakly binding to CAMK1G calmodulin kinase membrane expressing cells; the fluorescence value for binding to CD5 membrane expressing cells was about 40-100 times higher than that for binding to CAMK1G membrane expressing cells.

Study conclusions: Human membrane protein array detection results showed that CT125A single-chain antibody rabbit FC fusion protein RD125 61-42 rFc only had weak non-specific binding to CAMK1G calmodulin kinase, indicating that CT125A single-domain antibody has better specificity.

### Example 12 Determination of the affinity of anti-CD5 sdAbs

### Experimental purpose and principle:

The affinity between CD5 sdAbs and the antigen may have an important impact on the cytotoxicity and persistence of CAR-T in patients. In order to determine this important property, we used ForteBio's Octet molecular interaction technology to measure it. The biomembrane interferometry technology used in the Octet system is a label-free technology that provides high-throughput biomolecular interaction information in real time. The instrument emits white light to the surface of a sensor and collects the reflected light. The reflected light spectra of different frequencies is affected by the thickness of the optical film layer of the biosensor. Some frequencies of reflected light forms constructive interference (blue), while others are affected by destructive interference (red). These interferences are detected by a spectrometer to form an interference spectrum, which is displayed as the phase shift intensity (nm) of the interference spectrum. Therefore, once the number of molecules bound to the sensor surface increases or decreases, the spectrometer will detect the shift of the interference spectrum in real time, and this shift directly reflects the thickness of the biofilm on the sensor surface, from which high-quality data of biomolecular interaction can be obtained, so as to determine the kinetic parameters of biomolecular interactions (Kon, Kdis and KD), providing important information for the research and development process.

### Brief experimental steps:

1) Dilute anti-CDS IgG (constructed by fusing the VHH sequence of CD5 with human IgG4 Fc) to 20 µg/mL with loading buffer (1×PBS, pH 7.4, 0.01% BSA and 0.02% Tween 20), and load it onto the biosensor at approximately 0.8 nM.
2) After a 60s equilibration period, monitor the binding kinetics of the CD5 antigen (Aero, CD5-H52H5) at various antigen concentrations (100 to 1.563 nM). Carry out 160 s association and 300 s dissociation at each concentration, respectively.
3) Wash 3 times with 10mMGlycine-HCl, pH1.5 to regenerate the chip.
4) Analyze the binding constant by using a 1:1 binding site model (Biacore X-100 evaluation software).

### Experimental results:

Affinity refers to the strength of the binding of a single molecule to its ligand and is usually measured and reported by the equilibrium dissociation constant (KD), which is used to assess and rank the intensity of interaction between two molecules. The binding of an antibody to its antigen is a reversible process, and the rate of the binding reaction is directly proportional to the concentration of the reactants. The smaller the KD value, the greater the affinity of the antibody for its target. As shown in Table 8, FHVH1, FHVH3, FHVH3VH1 (FHVH3 and FHVH1 linked by linker (GGGGSGGGGSGGGGS) (SEQ ID NO: 21)), FHVH4 and FHVH2 could all bind to CD5 antigen, and the affinity of FHVH3VH1 was slightly higher than that of FHVH1, FHVH3, FHVH4 and FHVH2.

**Table 8 Anti-CD5 IgG affinity determination**

| Analyte | KD (M) | kon(1/Ms) | kdis(1/s) |
|---|---|---|---|
| FHVH1 (#42 IgG) | 2.90E-09 | 4.80E+04 | 1.39E-04 |
| FHVH3 (#61 IgG) | 3.96E-09 | 7.28E+04 | 2.88E-04 |
| FHVH3VH1 (#61-42 IgG) | 1.67E-09 | 1.13E+05 | 1.89E-04 |
| FHVH4 (#62 IgG) | 2.74E-08 | 1.25E+04 | 3.09E-04 |
| FHVH2 (#60 IgG) | 8.32E-09 | 2.55E+04 | 2.12E-04 |

### Example 13 Study on binding of tandem single-domain antibody to CD5⁺ target cells

Study purpose: To investigate the binding ability of CD5 single-domain antibody to CD5⁺ target cells.

Study method: Different concentrations of CD5 tandem single-domain antibody rabbit Fc fusion protein 61-42-rFc (i.e., a fusion protein constructed by linking #61 clone (FHVH3) with #42 clone (FHVH1) by linker (GGGGSGGGGSGGGGS) and then linking them to rabbit Fc) were incubated with CD5⁺ target cells respectively, the cells were washed twice with PBS, and then fluorescent dye-labeled rabbit Fc antibody was added to mark the positive cells, and detected by FCM (flow cytometry). The relationship between the percentage of fluorescence-labeled positive CD5⁺ target cells and different concentrations of CD5 tandem single-domain antibody was analyzed by fitting using Graphpad Prism software to calculate the EC50 constant. A total of three independent repetitions were performed in this experiment.

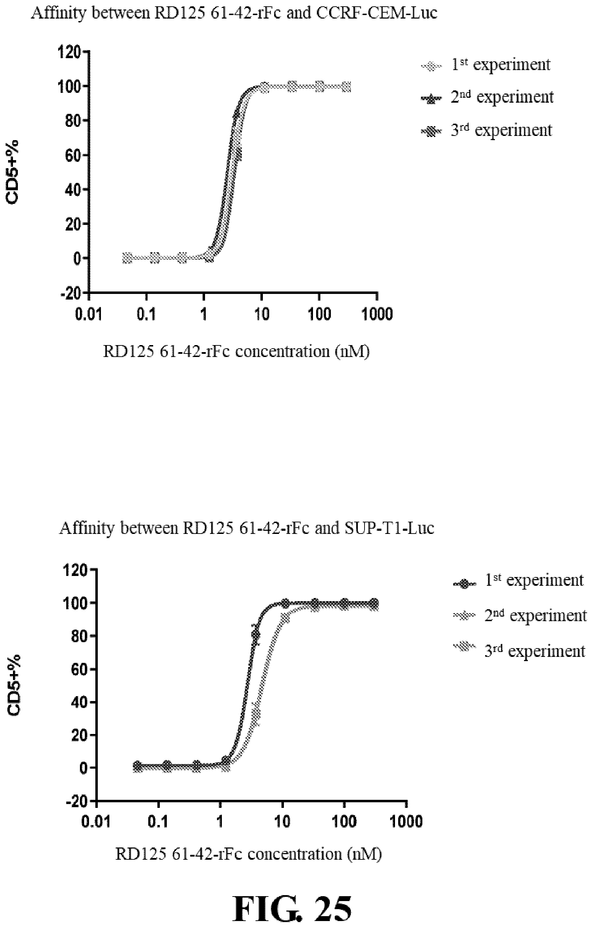

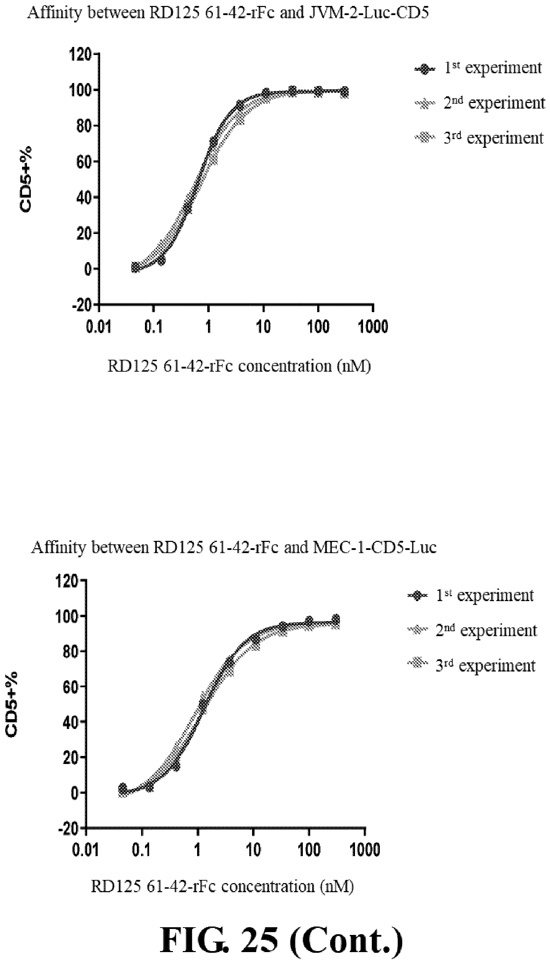

Study results: The CD5 tandem single-domain antibody rabbit FC fusion protein 61-42 rFc had high affinity with each of the 4 lines of CD5 positive cells, with the Kd being: 2.99±0.35nM (CCRF-CEM-Luc); 4.02 ±0.92nM (SUP-T1-Luc); 0.64±0.07nM (JVM-2-Luc-CDS); 1.14±0.16nM (MEC-1-CDS-Luc), respectively. The specific results are shown in Table 9 and Fig. 25.

**Table 9 Summary of affinity between 61-42-rFc single-domain antibody and each CD5 positive cell**

| **Name** | **Cell name** | **Number of times** | **Apparent** | **Average** | **Standard** |
|---|---|---|---|---|---|
| 61-42-rFc | CCRF-CEM-Luc | 1st time | 3.04 | 2.99 | 0.35 |
| | | 2nd time | 2.54 | | |
| | | 3rd time | 3.39 | | |
| | SUP-T1-Luc | 1st time | 2.72 | 4.02 | 0.92 |
| | | 2nd time | 4.58 | | |
| | | 3rd time | 4.75 | | |
| | JVM-2-Luc-CD5 | 1st time | 0.64 | 0.64 | 0.07 |
| | | 2nd time | 0.56 | | |
| | | 3rd time | 0.72 | | |
| | MEC-1-CD5-Luc | 1st time | 1.30 | 1.14 | 0.16 |
| | | 2nd time | 0.92 | | |
| | | 3rd time | 1.20 | | |

Study conclusions: The CD5 tandem single-domain antibody rabbit Fc fusion protein 61-42-rFc had stable, good and specific binding ability to the 4 CD5 positive cell lines tested, with EC50 values of 1-5 nM.

### References:

1. Castella, M., et al., Development of a Novel Anti-CD19 Chimeric Antigen Receptor: A Paradigm for an Affordable CAR T Cell Production at Academic Institutions. MolecµLar Therapy - Methods & Clinical Development, 2019. 12: p. 134-144.
2. Castella, M., et al., Point-Of-Care CAR T-Cell Production (ARI-0001) Using a Closed Semi-automatic Bioreactor: Experience From an Academic Phase I Clinical Trial. Frontiers in immunology, 2020. 11: p. 482.
3. Ortíz-Maldonado, V., et al., CART19-BE-01: A MµLticenter Trial of ARI-0001 Cell Therapy in Patients with CD19(+) Relapsed/Refractory Malignancies. Mol Ther, 2020.
4. Gill, S., et al., CD19 CAR-T cells combined with ibrutinib to induce complete remission in CLL. Journal of Clinical Oncology, 2017. 35(15_suppl): p. 7509-7509.
5. Neelapu, S.S., et al., Axicabtagene Ciloleucel CAR T-Cell Therapy in Refractory Large B-Cell Lymphoma. New England Journal of Medicine, 2017. 377(26): p. 2531-2544.
6. Maude, S.L., et al., CD19-targeted chimeric antigen receptor T-cell therapy for acute lymphoblastic leukemia. Blood, 2015. 125(26): p. 4017.
7. Jacobson, C.A., CD19 Chimeric Antigen Receptor Therapy for Refractory Aggressive B-Cell Lymphoma. Journal of Clinical Oncology, 2018. 37(4): p. 328-335.
8. Kochenderfer, J., et al., Anti-CD19 chimeric antigen receptor T cells preceded by low-dose chemotherapy to induce remissions of advanced lymphoma. Journal of Clinical Oncology, 2016. 34(18_suppl): p. LBA3010-LBA3010.
9. Grupp, S.A., et al., Chimeric Antigen Receptor Modified T Cells for Acute Lymphoid Leukemia. New England Journal of Medicine, 2013. 368(16): p. 1509-1518.
10. Hirayama, A.V., et al., The response to lymphodepletion impacts PFS in aggressive non-Hodgkin lymphoma patients treated with CD19 CAR-T cells. Blood, 2019: p. blood-2018-11-887067.
11. Davila, M.L., et al., Efficacy and toxicity management of 19-28z CAR T cell therapy in B cell acute lymphoblastic leukemia. Sci Transl Med, 2014. 6(224): p. 224ra25.
12. Jones, N.H., et al., Isolation of complementary DNA clones encoding the human lymphocyte glycoprotein T1/Leu-1. Nature, 1986. 323(6086): p. 346-349.
13. Huang, H.J., et al., MolecµLar cloning of Ly-1, a membrane glycoprotein of mouse T lymphocytes and a subset of B cells: molecµLar homology to its human counterpart Leu-1/T1 (CD5). Proceedings of the National Academy of Sciences of the United States of America, 1987. 84(1): p. 204-208.
14. Mamonkin, M., et al., A T-cell-directed chimeric antigen receptor for the selective treatment of T-cell malignancies. Blood, 2015. 126(8): p. 983-992.
15. Hill, L.C., et al., Safety and Anti-Tumor Activity of CD5 CAR T-Cells in Patients with Relapsed/Refractory T-Cell Malignancies. Blood, 2019. 134(Supplement_1): p. 199-199.

The specific sequences of the amino acids and nucleotides mentioned in the examples and other parts of the present application are as follows:
CD8α signal peptide nucleic acid sequence of FHVH1, FHVH2, FHVH3 and FHVH4, 63bp (SEQ ID NO: 1):
   ATGGCCCTGCCTGTGACAGCTCTGCTCCTCCCTCTGGCCCTGCTGCTCCATGCCGCCAGACCC
CD8α signal peptide protein sequence of FHVH1, FHVH2, FHVH3 and FHVH4, 21 aa (SEQ ID NO:2): MALPVTALLLPLALLLHAARP
CD8α hinge region nucleic acid sequence of FHVH1, FHVH2, FHVH3 and FHVH4: 165bp (SEQ ID NO: 3) 
TTCGTGCCCGTGTTCCTGCCCGCCAAACCTACTACTACCCCTGCACCTAGGCCTCCCACCCCAGC
CCCAACAATCGCCAGCCAGCCTCTGTCTCTGCGGCCCGAAGCCTGTAGACCTGCTGCCGGCGGA
GCCGTGCACACCAGAGGCCTGGACTTCGCCTGCGAC
CD8α hinge region protein sequence of FHVH1, FHVH2, FHVH3 and FHVH4, 55aa (SEQ ID NO: 4)
   FVPVFLPAKPTITPAPRPPTPAPTJASQPLSLRPEACRPAAGGAVHTRGLDFACD
CD8α transmembrane region nucleic acid sequence of FHVH1, FHVH2, FHVH3 and FHVH4, 84bp (SEQ ID NO: 5): 
ATCTACATCTGGGCCCCTCTGGCCGGCACCTGTGGCGTGCTGCTGCTGAGCCTGGTGATCACCCT
GTACTGCAACCACCGGAAC
CD8α transmembrane region protein sequence of FHVH1, FHVH2, FHVH3 and FHVH4, 28aa (SEQ ID NO: 6)
   IYIWAPLAGTCGVLLLSLVITLYCNHRN
4-1BB co-stimulatory domain nucleic acid sequence of FHVH1, FHVH2, FHVH3 and FHVH4, 126bp, (SEQ ID NO: 7): 
AAACGGGGCAGAAAGAAACTCCTGTATATATTCAAACAACCATTTATGAGACCAGTACAAACTAC
TCAAGAGGAAGATGGCTGTAGCTGCCGATTTCCAGAAGAAGAAGGAGGATGTGAACTG
4-1BB co-stimulatory domain protein sequence of FHVH1, FHVH2, FHVH3 and FHVH4, 42aa (SEQ ID NO:8): KRGRKKLLYIFKQPFMRPVQTTQEEDGCSCRFPEEEEGGCEL
CD3z intracellular signaling domain nucleic acid sequence of FHVH1, FHVH2, FHVH3 and FHVH4, 336bp (SEQ ID NO: 9): 
AGAGTGAAGTTCAGCAGATCCGCCGACGCCCCTGCCTACCAGCAGGGACAGAACCAGCTGTAC
AACGAGCTGAACCTGGGCAGACGGGAAGAGTACGACGTGCTGGACAAGCGGAGAGGCCGGGA
CCCCGAGATGGGCGGAAAGCCCAGACGGAAGAACCCCCAGGAAGGCCTGTATAACGAACTGCA
GAAAGACAAGATGGCCGAGGCCTACAGCGAGATCGGCATGAAGGGCGAGCGGAGGCGCGGCA
AGGGCCACGATGGCCTGTACCAGGGCCTGAGCACCGCCACCAAGGACACCTACGACGCCCTGC
ACATGCAGGCCCTGCCCCCCAGA
CD3z intracellular signaling domain protein sequence of FHVH1, FHVH2, FHVH3 and FHVH4, 112aa (SEQ ID NO: 10): 
RVKFSRSADAPAYQQGQNQLYNELNLGRREEYDVLDKRRGRDPEMGGKPRRKNPQEGLYNELQKD
KMAEAYSEIGMKGERRRGKGHDGLYQGLSTATKDTYDALHMQALPPR;
Cleaving peptide T2A nucleic acid sequence of FHVH1, FHVH2, FHVH3 and FHVH4, 54bp (SEQ ID NO: 11):
   GAGGGAAGGGGCAGCTTATTAACATGTGGCGATGTGGAAGAGAACCCCGGTCCC;
Cleaving peptide T2A protein sequence of FHVH1, FHVH2, FHVH3 and FHVH4, 18aa, (SEQ ID NO: 12): EGRGSLLTCGDVEENPGP;
CSF2RA signal nucleic acid sequence of FHVH1, FHVH2, FHVH3 and FHVH4, 66bp, (SEQ ID NO: 13):
   ATGCTGCTGCTCGTGACCTCTTTACTGTTATGTGAGCTGCCCCACCCCGCTTTTTTACTGATCCCT
CSF2RA signal protein sequence of FHVH1, FHVH2, FHVH3 and FHVH4, 22aa, (SEQ ID NO: 14): MLLLVTSLLLCELPHPAFLLIP
tEGFR nucleic acid sequence of FHVH1, FHVH2, FHVH3 and FHVH4: 1005bp (SEQ ID NO: 15) 
CGTAAGGTGTGTAACGGAATCGGCATTGGCGAGTTCAAGGACTCTTTAAGCATCAACGCCACA
AACATCAAGCACTTCAAGAATTGTACCTCCATCAGCGGCGATTTACACATTCTCCCCGTGGCTT
TTCGTGGCGATTCCTTCACCCACACCCCCCCTCTGGACCCCCAAGAGCTGGACATTTTAAAAAC
CGTGAAGGAGATCACCGGCTTTCTGCTGATCCAAGCTTGGCCCGAGAATCGTACCGACCTCCAC
GCCTTCGAGAATTTAGAGATTATTCGTGGAAGGACCAAGCAGCACGGCCAGTTCTCTTTAGCCG
TCGTGTCTTTAAACATTACCAGCCTCGGTTTAAGGTCTTTAAAGGAGATTAGCGACGGCGACGT
GATCATCTCCGGCAACAAGAACCTCTGCTACGCCAACACCATCAACTGGAAGAAGCTGTTCGG
AACCAGCGGCCAAAAGACCAAGATCATCAGCAATCGTGGAGAGAACTCTTGTAAGGCCACTGG
TCAAGTTTGCCACGCCCTCTGTAGCCCCGAAGGATGTTGGGGCCCCGAGCCTAGGGACTGTGTT
AGCTGCAGAAACGTGAGCAGAGGCAGAGAGTGTGTGGACAAATGCAATTTACTGGAAGGAGA
GCCTAGGGAGTTCGTGGAGAACAGCGAATGTATCCAGTGCCACCCCGAGTGTTTACCTCAAGCC
ATGAACATCACTTGTACCGGAAGGGGCCCCGATAACTGCATCCAATGCGCCCACTACATCGAC

GGACCCCACTGCGTGAAAACTTGTCCCGCCGGAGTGATGGGAGAGAATAACACTTTAGTGTGG
AAGTACGCCGACGCTGGCCACGTCTGCCATCTGTGCCACCCCAACTGTACCTACGGCTGCACTG
GTCCCGGTTTAGAGGGATGTCCTACCAACGGCCCCAAGATCCCCTCCATCGCCACCGGAATGGT
GGGCGCTCTGTTATTACTGCTGGTGGTGGCTCTGGGCATCGGTTTATTCATG
tEGFR protein sequence of FHVH1, FHVH2, FHVH3 and FHVH4, 335aa (SEQ ID NO: 16): 
RKVCNGIGIGEFKDSLSINATNIKHFKNCTSISGDLHILPVAFRGDSFTHTPPLDPQELDILKTVKEITG
FLLIQAWPENRTDLHAFENLEIIRGRTKQHGQFSLAVVSLNITSLGLRSLKEISDGDVIISGNKNLCYA
NTINWKKLFGTSGQKTK
IISNRGENSCKATGQVCHALCSPEGCWGPEPRDCVSCRNVSRGRECVDKCNLLEGEPREFVENSECI
QCHPECLPQAMNITCTGRGPDNCIQCAHYIDGPHCVKTCPAGVMGENNTLVWKYADAGHVCHLC
HPNCTYGCTGPGLEGCPTNGPKIPSIATGMVGALLLLLVVALGIGLFM
FHVH3VH1, FWVH2VH1 and FWVH4VH1CD8α signal peptide nucleic acid sequence, 63bp (SEQ ID NO: 17) ATGGCCCTACCTGTGACAGCCCTACTGTTACCCCTGGCCCTCCTTCTGCATGCTGCTAGACCT
FHVH3VH1, FWVH2VH1 and FWVH4VH1CD8α hinge region nucleic acid sequence: 249 bp (SEQ ID NO: 18) 
TTTGTGCCTGTATTTCTGCCTGCCAAGCCCACCACAACACCTGCCCCTAGACCACCCACCCCTGC
CCCCACCATTGCTTCTCAGCCCCTTAGCTTAAGACCTGAAGCCTGTAGACCTGCTGCTGGGGGG
GCTGTGCACACAAGAGGCCTGGACTTTGCCTGTGACATCTACATCTGGGCCCCCCTGGCTGGCA
CCTGTGGGGTGCTGCTGCTGAGCCTGGTGATCACCCTGTACTGCAACCACAGAAAC
FHVH3VH1, FWVH2VH1 and FWVH4VH14-1BB intracellular co-stimulatory domain nucleic acid sequence, 126bp, (SEQ ID NO: 19) 
AAGAGAGGCAGAAAGAAGCTGCTGTACATCTTCAAGCAGCCCTTCATGAGACCTGTGCAGACC
ACCCAAGAGGAGGATGGCTGCAGCTGCAGATTCCCTGAGGAGGAGGAGGGGGGCTGTGAGCT
G
FHVH3VH1, FWVH2VH1 and FWVH4VH1CD3z intracellular signaling domain nucleic acid sequence, 336bp (SEQ ID NO: 20) 
AGAGTGAAGTTCAGCAGATCTGCTGATGCCCCTGCCTATCAGCAAGGGCAGAATCAGCTGTAC
AATGAGCTGAATCTGGGCAGAAGAGAGGAGTATGATGTGCTGGACAAGAGAAGAGGCAGAGA
CCCTGAGATGGGGGGCAAGCCTAGAAGAAAGAACCCCCAAGAGGGCCTGTATAATGAGCTGCA
GAAGGACAAGATGGCTGAGGCCTACTCTGAGATTGGCATGAAGGGGGAGAGAAGAAGAGGCA
AGGGCCATGATGGCCTGTACCAAGGCCTGAGCACAGCCACCAAGGACACCTATGATGCCCTAC
ACATGCAAGCTCTGCCTCCTAGA
FHVH3VH1, FWVH2VH1 and FWVH4VH1 cleaving peptide T2A nucleic acid sequence, 54bp (SEQ ID NO: 21):
   GAAGGAAGGGGCAGCCTACTGACCTGTGGGGATGTGGAGGAGAACCCTGGCCCC
FHVH3VH1, FWVH2VH1 and FWVH4VH1CSF2RA signal nucleic acid sequence, 66bp, (SEQ ID NO:22):
   ATGTTGCTATTAGTAACCAGCCTGCTGCTGTGTGAGCTGCCCCACCCTGCCTTCCTGTTAATCCCA
FHVH3VH1, FWVH2VH1 and FWVH4VH1tEGFR nucleic acid sequence: 1005bp (SEQ ID NO: 23)
   CGAAAGGTATGTAATGGCATTGGCATFGGGGAGTTTAAGGACAGCCTGAGCATCAATGCCACC 
AACATCAAGCACTTCAAGAACTGCACAAGCATCAGTGGGGACTTGCACATCCTGCCTGTGGCCT
TCAGAGGGGACAGCTTCACCCACACCCCCCCCCTGGACCCCCAAGAGCTGGACATCCTGAAGA
CAGTGAAGGAGATCACTGGCTTCTTGCTGATCCAAGCCTGGCCTGAGAACAGAACAGACCTGC
ATGCCTTTGAGAACCTGGAGATCATCAGAGGCAGAACCAAGCAGCATGGGCAGTTCAGCCTGG
CTGTGGTGAGCCTGAACATCACAAGCCTGGGCCTGAGAAGCTTAAAGGAGATCTCTGATGGGG
ATGTGATCATCTCTGGCAACAAGAACCTGTGCTATGCCAACACCATCAACTGGAAGAAGCTGTT
TGGCACCTCTGGGCAGAAGACCAAGATCATCAGCAACAGAGGGGAGAACTCCTGTAAGGCCAC
TGGCCAAGTGTGTCATGCCCTATGCAGCCCTGAGGGGTGCTGGGGCCCTGAGCCTAGAGACTGT
GTGAGCTGCAGAAATGTGAGCAGAGGCAGAGAGTGTGTGGACAAGTGCAACCTGCTGGAGGG
GGAGCCTAGAGAGTTTGTGGAGAACTCTGAGTGTATTCAGTGTCATCCTGAGTGCCTGCCCCAA
GCCATGAACATCACCTGCACTGGCAGAGGCCCTGACAACTGCATTCAGTGTGCCCACTACATTG
ATGGCCCCCACTGTGTGAAGACCTGCCCTGCTGGGGTGATGGGGGAGAACAACACCCTGGTGT
GGAAGTATGCTGATGCTGGCCATGTGTGTCACCTGTGCCATCCCAACTGCACCTATGGCTGCAC
TGGCCCTGGCCTGGAGGGCTGCCCCACCAATGGTCCCAAGATTCCTAGCATTGCCACTGGCATG
GTGGGGGCCCTGCTCCTACTTCTGGTGGTTGCCCTGGGCATTGGCCTGTTCATG
FHVH3VH1, FWVH2VH1 and FWVH4VH1 Linker nucleic acid sequence, 45bp (SEQ ID NO: 24)
   GGGGGGGGGGGCTCTGGGGGGGGTGGCTCAGGTGGCGGTGGCTCT
FHVH3VH1, FWVH2VH1 and FWVH4VH1 Linker protein sequence, 15aa (SEQ ID NO:25)
   GGGGSGGGGSGGGGS
FHVH1: CAR nucleic acid sequence, 2271bp (SEQ ID NO: 26): 
ATGGCCCTGCCTGTGACAGCTCTGCTCCTCCCTCTGGCCCTGCTGCTCCATGCCGCCAGACCCGA
AGTTCAGCTGCTGGAAAGCGGTGGTGGTCTGGTTCAGCCTGGTGGTAGCCTGCGTCTGAGCTGT
GCAGCAAGCGGTTTTACCTTTAGCCATAGCGCCATGGGTTGGGTTCGTCAGGCACCTGGTAAAG
GTCTGGAATGGGTTAGCAGCATCTATGCCCGCGGCGGCTATACCTATTATGCAGATAGCGTTAA
AGGTCGTTTTACCATTAGCCGTGATAACAGCAAAAATACCCTGTACCTGCAGATGAATAGTCTG
CGTGCAGAGGATACCGCAGTGTATTATTGTGCGCGCGGTTACCATCTGGAATACATGGTTTCTC
AGGATGTTTGGGGTCAAGGTACTCTGGTGACCGTCTCCTCATTCGTGCCCGTGTTCCTGCCCGCC
AAACCTACTACTACCCCTGCACCTAGGCCTCCCACCCCAGCCCCAACAATCGCCAGCCAGCCTC
TGTCTCTGCGGCCCGAAGCCTGTAGACCTGCTGCCGGCGGAGCCGTGCACACCAGAGGCCTGG
ACTTCGCCTGCGACATCTACATCTGGGCCCCTCTGGCCGGCACCTGTGGCGTGCTGCTGCTGAG
CCTGGTGATCACCCTGTACTGCAACCACCGGAACAAACGGGGCAGAAAGAAACTCCTGTATAT
ATTCAAACAACCATTTATGAGACCAGTACAAACTACTCAAGAGGAAGATGGCTGTAGCTGCCG
ATTTCCAGAAGAAGAAGAAGGAGGATGTGAACTGAGAGTGAAGTTCAGCAGATCCGCCGACGC
CCCTGCCTACCAGCAGGGACAGAACCAGCTGTACAACGAGCTGAACCTGGGCAGACGGGAAGA

GTACGACGTGCTGGACAAGCGGAGAGGCCGGGACCCCGAGATGGGCGGAAAGCCCAGACGGA
AGAACCCCCAGGAAGGCCTGTATAACGAACTGCAGAAAGACAAGATGGCCGAGGCCTACAGC
GAGATCGGCATGAAGGGCGAGCGGAGGCGCGGCAAGGGCCACGATGGCCTGTACCAGGGCCT
GAGCACCGCCACCAAGGACACCTACGACGCCCTGCACATGCAGGCCCTGCCCCCCAGAGGATC
CGGAGAGGGAAGGGGCAGCTTATTAACATGTGGCGATGTGGAAGAGAACCCCGGTCCCATGCT
GCTGCTCGTGACCTCTTTACTGTTATGTGAGCTGCCCCACCCCGCTTTTTTACTGATCCCTCGTA
AGGTGTGTAACGGAATCGGCATTGGCGAGTTCAAGGACTCTTTAAGCATCAACGCCACAAACA
TCAAGCACTTCAAGAATTGTACCTCCATCAGCGGCGATTTACACATTCTCCCCGTGGCTTTTCGT
GGCGATTCCTTCACCCACACCCCCCCTCTGGACCCCCAAGAGCTGGACATTTTAAAAACCGTGA
AGGAGATCACCGGCTTTCTGCTGATCCAAGCTTGGCCCGAGAATCGTACCGACCTCCACGCCTT
CGAGAATTTAGAGATTATTCGTGGAAGGACCAAGCAGCACGGCCAGTTCTCTTTAGCCGTCGTG
TCTTTAAACATTACCAGCCTCGGTTTAAGGTCTTTAAAGGAGATTAGCGACGGCGACGTGATCA
TCTCCGGCAACAAGAACCTCTGCTACGCCAACACCATCAACTGGAAGAAGCTGTTCGGAACCA
GCGGCCAAAAGACCAAGATCATCAGCAATCGTGGAGAGAACTCTTGTAAGGCCACTGGTCAAG
TTTGCCACGCCCTCTGTAGCCCCGAAGGATGTTGGGGCCCCGAGCCTAGGGACTGTGTTAGCTG
CAGAAACGTGAGCAGAGGCAGAGAGTGTGTGGACAAATGCAATTTACTGGAAGGAGAGCCTA
GGGAGTTCGTGGAGAACAGCGAATGTATCCAGTGCCACCCCGAGTGTTTACCTCAAGCCATGA
ACATCACTTGTACCGGAAGGGGCCCCGATAACTGCATCCAATGCGCCCACTACATCGACGGAC
CCCACTGCGTGAAAACTTGTCCCGCCGGAGTGATGGGAGAGAATAACACTTTAGTGTGGAAGT
ACGCCGACGCTGGCCACGTCTGCCATCTGTGCCACCCCAACTGTACCTACGGCTGCACTGGTCC
CGGTTTAGAGGGATGTCCTACCAACGGCCCCAAGATCCCCTCCATCGCCACCGGAATGGTGGGC
GCTCTGTTATTACTGCTGGTGGTGGCTCTGGGCATCGGTTTATTCATG
FHVH1: CAR protein sequence, 757aa (SEQ ID NO: 27): 
MALPVTALLLPLALLLHAARPEVQLLESGGGLVQPGGSLRLSCAASGFTFSHSAMGWVRQAPGKG
LEWVSSIYARGGYTYYADSVKGRFTISRDNSKNTLYLQMNSLRAEDTAVYYCARGYHLEYMVSQD
VWGQGTLVTVSSFVPVFLPAKPTTTPAPRPPTPAPTIASQPLSLRPEACRPAAGGAVHTRGLDFACDI
YIWAPLAGTCGVLLLSLVITLYCNHRNKRGRKKLLYIFKQPFMRPVQTTQEEDGCSCRFPEEEEGGC
ELRVKFSRSADAPAYQQGQNQLYNELNLGRREEYDVLDKRRGRDPEMGGKPRRKNPQEGLYNEL
QKDKMAEAYSEIGMKGERRRGKGHDGLYQGLSTATKDTYDALHMQALPPRGSGEGRGSLLTCGD
VEENPGPMLLLVTSLLLCELPHPAFLLIPRKVCNGIGIGEFKDSLSINATNIKHFKNCTSISGDLHILPV
AFRGDSFTHTPPLDPQELDILKTVKEITGFLLIQAWPENRTDLHAFENLEIIRGRTKQHGQFSLAVVSL
NITSLGLRSLKEISDGDVIISGNKNLCYANTINWKKLFGTSGQKTKIISNRGENSCKATGQVCHALCS
PEGCWGPEPRDCVSCRNVSRGRECVDKCNLLEGEPREFVENSECIQCHPECLPQAMNITCTGRGPDN
CIQCAHYIDGPHCVKTCPAGVMGENNTLVWKYADAGHVCHLCHPNCTYGCTGPGLEGCPTNGPKI

PSIATGMVGALLLLLVVALGIGLFM
FHVH3 CAR nucleic acid sequence, 2262bp (SEQ ID NO: 28): 
ATGGCCCTGCCTGTGACAGCTCTGCTCCTCCCTCTGGCCCTGCTGCTCCATGCCGCCAGACCCCA
GGTGCAGCTGGTGCAGTCTGGGGCTGAGGTGAAGAAGCCTGGGTCCTCGGTGAAGGTCTCCTG
CAAGGCTTCTGGAGGCACCTTCAGCAACTATGCTATCAGCTGGGTGCGACAGGCCCCTGGACAA
GGGCTTGAGTGGATGGGATGGATCAGCGCCTACAATGGTGACACAAAATATGCACAGAGGCTCC
AGGGCAGAGTCACCATGACCACAGACACATCCACGAGCACAGCCTACATGGAGCTGAGGAACC
TAAGATCTGACGACACGGCCGTGTATTACTGTGCGCGCTACGAATCTATGTCTGGTCAGGATATCT
GGGGTCAAGGTACTCTGGTGACCGTCTCCTCATTCGTGCCCGTGTTCCTGCCCGCCAAACCTACT
ACTACCCCTGCACCTAGGCCTCCCACCCCAGCCCCAACAATCGCCAGCCAGCCTCTGTCTCTGCG
GCCCGAAGCCTGTAGACCTGCTGCCGGCGGAGCCGTGCACACCAGAGGCCTGGACTTCGCCTG
CGACATCTACATCTGGGCCCCTCTGGCCGGCACCTGTGGCGTGCTGCTGCTGAGCCTGGTGATCA
CCCTGTACTGCAACCACCGGAACAAACGGGGCAGAAAGAAACTCCTGTATATATTCAAACAACC
ATTTATGAGACCAGTACAAACTACTCAAGAGGAAGATGGCTGTAGCTGCCGATTTCCAGAAGAA
GAAGAAGGAGGATGTGAACTGAGAGTGAAGTTCAGCAGATCCGCCGACGCCCCTGCCTACCAG
CAGGGACAGAACCAGCTGTACAACGAGCTGAACCTGGGCAGACGGGAAGAGTACGACGTGCTG
GACAAGCGGAGAGGCCGGGACCCCGAGATGGGCGGAAAGCCCAGACGGAAGAACCCCCAGGA
AGGCCTGTATAACGAACTGCAGAAAGACAAGATGGCCGAGGCCTACAGCGAGATCGGCATGAA
GGGCGAGCGGAGGCGCGGCAAGGGCCACGATGGCCTGTACCAGGGCCTGAGCACCGCCACCAA
GGACACCTACGACGCCCTGCACATGCAGGCCCTGCCCCCCAGAGGATCCGGAGAGGGAAGGGG
CAGCTTATTAACATGTGGCGATGTGGAAGAGAACCCCGGTCCCATGCTGCTGCTCGTGACCTCTT
TACTGTTATGTGAGCTGCCCCACCCCGCTTTTTTACTGATCCCTCGTAAGGTGTGTAACGGAATCG
GCATTGGCGAGTTCAAGGACTCTTTAAGCATCAACGCCACAAACATCAAGCACTTCAAGAATTG
TACCTCCATCAGCGGCGATTTACACATTCTCCCCGTGGCTTTTCGTGGCGATTCCTTCACCCACAC
CCCCCCTCTGGACCCCCAAGAGCTGGACATTTTAAAAACCGTGAAGGAGATCACCGGCTTTCTG
CTGATCCAAGCTTGGCCCGAGAATCGTACCGACCTCCACGCCTTCGAGAATTTAGAGATTATTCG
TGGAAGGACCAAGCAGCACGGCCAGTTCTCTTTAGCCGTCGTGTCTTTAAACATTACCAGCCTCG
GTTTAAGGTCTTTAAAGGAGATTAGCGACGGCGACGTGATCATCTCCGGCAACAAGAACCTCTG
CTACGCCAACACCATCAACTGGAAGAAGCTGTTCGGAACCAGCGGCCAAAAGACCAAGATCAT
CAGCAATCGTGGAGAGAACTCTTGTAAGGCCACTGGTCAAGTTTGCCACGCCCTCTGTAGCCCC
GAAGGATGTTGGGGCCCCGAGCCTAGGGACTGTGTTAGCTGCAGAAACGTGAGCAGAGGCAGA
GAGTGTGTGGACAAATGCAATTTACTGGAAGGAGAGCCTAGGGAGTTCGTGGAGAACAGCGAA
TGTATCCAGTGCCACCCCGAGTGTTTACCTCAAGCCATGAACATCACTTGTACCGGAAGGGGCCC
CGATAACTGCATCCAATGCGCCCACTACATCGACGGACCCCACTGCGTGAAAACTTGTCCCGCCG

GAGTGATGGGAGAGAATAACACTTTAGTGTGGAAGTACGCCGACGCTGGCCACGTCTGCCATCT
GTGCCACCCCAACTGTACCTACGGCTGCACTGGTCCCGGTTTAGAGGGATGTCCTACCAACGGCC
CCAAGATCCCCTCCATCGCCACCGGAATGGTGGGCGCTCTGTTATTACTGCTGGTGGTGGCTCTG
GGCATCGGTTTATTCATG
FHVH3 CAR protein sequence, 754 aa (SEQ ID NO: 29): 
MALPVTALLLPLALLLHAARPQVQLVQSGAEVKKPGSSVKVSCKASGGTFSNYAISWVRQAPGQGL
EWMGWISAYNGDTKYAQRLQGRVTMTTDTSTSTAYMELRNLRSDDTAVYYCARYESMSGQDIWG
QGTLVTVSSFVPVFLPAKPTTTPAPRPPTPAPTIASQPLSLRPEACRPAAGGAVHTRGLDFACDIYIWAP
LAGTCGVLLLSLVITLYCNHRNKRGRKKLLYIFKQPFMRPVQTTQEEDGCSCRFPEEEEGGCELRVKF
SRSADAPAYQQGQNQLYNELNLGRREEYDVLDKRRGRDPEMGGKPRRKNPQEGLYNELQKDKMA
EAYSEIGMKGERRRGKGHDGLYQGLSTATKDTYDALHMQALPPRGSGEGRGSLLTCGDVEENPGP
MLLLVTSLLLCELPHPAFLLIPRKVCNGIGIGEFKDSLSINATNIKHFKNCTSISGDLHILPVAFRGDSFT
HTPPLDPQELDILKTVKEITGFLLIQAWPENRTDLHAFENLEIIRGRTKQHGQFSLAVVSLNITSLGLRS
LKEISDGDVIISGNKNLCYANTINWKKLFGTSGQKTKIISNRGENSCKATGQVCHALCSPEGCWGPEP
RDCVSCRNVSRGRECVDKCNLLEGEPREFVENSECIQCHPECLPQAMNITCTGRGPDNCIQCAHYID
GPHCVKTCPAGVMGENNTLVWKYADAGHVCHLCHPNCTYGCTGPGLEGCPTNGPKIPSIATGMVG
ALLLLLVVALGIGLFM
FHVH3VH1 CAR nucleic acid sequence, 2262bp (SEQ ID NO: 30): 
ATGGCCCTACCTGTGACAGCCCTACTGTTACCCCTGGCCCTCCTTCTGCATGCTGCTAGACCTCAG
GTGCAGCTGGTGCAGTCTGGGGCTGAGGTGAAGAAGCCTGGGTCCTCGGTGAAGGTCTCCTGC
AAGGCTTCTGGAGGCACCTTCAGCAACTATGCTATCAGCTGGGTGCGACAGGCCCCTGGACAAG
GGCTTGAGTGGATGGGATGGATCAGCGCCTACAATGGTGACACAAAATATGCACAGAGGCTCCA
GGGCAGAGTCACCATGACCACAGACACATCCACGAGCACAGCCTACATGGAGCTGAGGAACCT
AAGATCTGACGACACGGCCGTGTATTACTGTGCGCGCTACGAATCTATGTCTGGTCAGGATATCTG
GGGTCAAGGTACTCTGGTGACCGTCTCCTCAGGGGGGGGGGGCTCTGGGGGGGGTGGCTCAGG
TGGCGGTGGCTCTGAAGTTCAGCTGCTGGAAAGCGGTGGTGGTCTGGTTCAGCCTGGTGGTAGC
CTGCGTCTGAGCTGTGCAGCAAGCGGTTTTACCTTTAGCCATAGCGCCATGGGTTGGGTTCGTCA
GGCACCTGGTAAAGGTCTGGAATGGGTTAGCAGCATCTATGCCCGCGGCGGCTATACCTATTATGC
AGATAGCGTTAAAGGTCGTTTTACCATTAGCCGTGATAACAGCAAAAATACCCTGTACCTGCAGA
TGAATAGTCTGCGTGCAGAGGATACCGCAGTGTATTATTGTGCGCGCGGTTACCATCTGGAATACA
TGGTTTCTCAGGATGTTTGGGGTCAAGGTACTCTGGTGACCGTCTCCTCATTTGTGCCTGTATTTC
TGCCTGCCAAGCCCACCACAACACCTGCCCCTAGACCACCCACCCCTGCCCCCACCATTGCTTCT
CAGCCCCTTAGCTTAAGACCTGAAGCCTGTAGACCTGCTGCTGGGGGGGCTGTGCACACAAGAG
 
GCCTGGACTTTGCCTGTGACATCTACATCTGGGCCCCCCTGGCTGGCACCTGTGGGGTGCTGCTG
CTGAGCCTGGTGATCACCCTGTACTGCAACCACAGAAACAAGAGAGGCAGAAAGAAGCTGCTG
TACATCTTCAAGCAGCCCTTCATGAGACCTGTGCAGACCACCCAAGAGGAGGATGGCTGCAGCT
GCAGATTCCCTGAGGAGGAGGAGGGGGGCTGTGAGCTGAGAGTGAAGTTCAGCAGATCTGCTG
ATGCCCCTGCCTATCAGCAAGGGCAGAATCAGCTGTACAATGAGCTGAATCTGGGCAGAAGAGA
GGAGTATGATGTGCTGGACAAGAGAAGAGGCAGAGACCCTGAGATGGGGGGCAAGCCTAGAAG
AAAGAACCCCCAAGAGGGCCTGTATAATGAGCTGCAGAAGGACAAGATGGCTGAGGCCTACTCT
GAGATTGGCATGAAGGGGGAGAGAAGAAGAGGCAAGGGCCATGATGGCCTGTACCAAGGCCTG
AGCACAGCCACCAAGGACACCTATGATGCCCTACACATGCAAGCTCTGCCTCCTAGAGGCTCTG
GGGAAGGAAGGGGCAGCCTACTGACCTGTGGGGATGTGGAGGAGAACCCTGGCCCCATGTTGC
TATTAGTAACCAGCCTGCTGCTGTGTGAGCTGCCCCACCCTGCCTTCCTGTTAATCCCACGAAAG
GTATGTAATGGCATTGGCATTGGGGAGTTTAAGGACAGCCTGAGCATCAATGCCACCAACATCAA
GCACTTCAAGAACTGCACAAGCATCAGTGGGGACTTGCACATCCTGCCTGTGGCCTTCAGAGGG
GACAGCTTCACCCACACCCCCCCCCTGGACCCCCAAGAGCTGGACATCCTGAAGACAGTGAAG
GAGATCACTGGCTTCTTGCTGATCCAAGCCTGGCCTGAGAACAGAACAGACCTGCATGCCTTTG
AGAACCTGGAGATCATCAGAGGCAGAACCAAGCAGCATGGGCAGTTCAGCCTGGCTGTGGTGA
GCCTGAACATCACAAGCCTGGGCCTGAGAAGCTTAAAGGAGATCTCTGATGGGGATGTGATCAT
CTCTGGCAACAAGAACCTGTGCTATGCCAACACCATCAACTGGAAGAAGCTGTTTGGCACCTCT
GGGCAGAAGACCAAGATCATCAGCAACAGAGGGGAGAACTCCTGTAAGGCCACTGGCCAAGTG
TGTCATGCCCTATGCAGCCCTGAGGGGTGCTGGGGCCCTGAGCCTAGAGACTGTGTGAGCTGCA
GAAATGTGAGCAGAGGCAGAGAGTGTGTGGACAAGTGCAACCTGCTGGAGGGGGAGCCTAGA
GAGTTTGTGGAGAACTCTGAGTGTATTCAGTGTCATCCTGAGTGCCTGCCCCAAGCCATGAACAT
CACCTGCACTGGCAGAGGCCCTGACAACTGCATTCAGTGTGCCCACTACATTGATGGCCCCCACT
GTGTGAAGACCTGCCCTGCTGGGGTGATGGGGGAGAACAACACCCTGGTGTGGAAGTATGCTG
ATGCTGGCCATGTGTGTCACCTGTGCCATCCCAACTGCACCTATGGCTGCACTGGCCCTGGCCTG
GAGGGCTGCCCCACCAATGGTCCCAAGATTCCTAGCATTGCCACTGGCATGGTGGGGGCCCTGC
TCCTACTTCTGGTGGTTGCCCTGGGCATTGGCCTGTTCATG
FHVH3VH1, CAR protein sequence, 890 aa (SEQ ID NO: 31): 
MALPVTALLLPLALLLHAARPQVQLVQSGAEVKKPGSSVKVSCKASGGTFSNYAISWVRQAPGQGL
EWMGWISAYNGDTKYAQRLQGRVTMTTDTSTSTAYMELRNLRSDDTAVYYCARYESMSGQDIWG
QGTLVTVSSGGGGSGGGGSGGGGS
EVQLLESGGGLVQPGGSLRLSCAASGFTFSHSAMGWVRQAPGKGLEWVSSIYARGGYTYYADSVK
GRFTISRDNSKNTLYLQMNSLRAEDTAVYYCARGYHLEYMVSQDVWGQGTLVTVSSFVPVFLPAKP
TTTPAPRPPTPAPTIASQPLSLRPEACRPAAGGAVHTRGLDFACDIYIWAPLAGTCGVLLLSLVITLYCN
 
HRNKRGRKKLLYIFKQPFMRPVQTTQEEDGCSCRFPEEEEGGCELRVKFSRSADAPAYQQGQNQLY
NELNLGRREEYDVLDKRRGRDPEMGGKPRRKNPQEGLYNELQKDKMAEAYSEIGMKGERRRGKG
HDGLYQGLSTATKDTYDALHMQALPPRGSGEGRGSLLTCGDVEENPGPMLLLVTSLLLCELPHPAFL
LIPRKVCNGIGIGEFKDSLSINATNIKHFKNCTSISGDLHILPVAFRGDSFTHTPPLDPQELDILKTVKEI
TGFLLIQAWPENRTDLHAFENLEIIRGRTKQHGQFSLAVVSLNITSLGLRSLKEISDGDVIISGNKNLCY
ANTINWKKLFGTSGQKTKIISNRGENSCKATGQVCHALCSPEGCWGPEPRDCVSCRNVSRGRECVD
KCNLLEGEPREFVENSECIQCHPECLPQAMNITCTGRGPDNCIQCAHYIDGPHCVKTCPAGVMGENN
TLVWKYADAGHVCHLCHPNCTYGCTGPGLEGCPTNGPKIPSIATGMVGALLLLLVVALGIGLFM
FHVH1: sdAb nucleic acid sequence, 363bp (SEQ ID NO: 32) 
GAAGTTCAGCTGCTGGAAAGCGGTGGTGGTCTGGTTCAGCCTGGTGGTAGCCTGCGTCTGAGCT
GTGCAGCAAGCGGTTTTACCTTTAGCCATAGCGCCATGGGTTGGGTTCGTCAGGCACCTGGTAAA
GGTCTGGAATGGGTTAGCAGCATCTATGCCCGCGGCGGCTATACCTATTATGCAGATAGCGTTAAA
GGTCGTTTTACCATTAGCCGTGATAACAGCAAAAATACCCTGTACCTGCAGATGAATAGTCTGCGT
GCAGAGGATACCGCAGTGTATTATTGTGCGCGCGGTTACCATCTGGAATACATGGTTTCTCAGGAT
GTTTGGGGTCAAGGTACTCTGGTGACCGTCTCCTCA
FHVH1: sdAb protein sequence, 121 aa (SEQ ID NO: 33) 
EVQLLESGGGLVQPGGSLRLSCAASGFTFSHSAMGWVRQAPGKGLEWVSSIYARGGYTYYADSVK
GRFTISRDNSKNTLYLQMNSLRAEDTAVYYCARGYHLEYMVSQDVWGQGTLVTVSS
FHVH3: sdAb nucleic acid sequence, 354bp (SEQ ID NO: 34) 
CAGGTGCAGCTGGTGCAGTCTGGGGCTGAGGTGAAGAAGCCTGGGTCCTCGGTGAAGGTCTCC
TGCAAGGCTTCTGGAGGCACCTTCAGCAACTATGCTATCAGCTGGGTGCGACAGGCCCCTGGAC
AAGGGCTTGAGTGGATGGGATGGATCAGCGCCTACAATGGTGACACAAAATATGCACAGAGGCT
CCAGGGCAGAGTCACCATGACCACAGACACATCCACGAGCACAGCCTACATGGAGCTGAGGAA
CCTAAGATCTGACGACACGGCCGTGTATTACTGTGCGCGCTACGAATCTATGTCTGGTCAGGATAT
CTGGGGTCAAGGTACTCTGGTGACCGTCTCCTCA
FHVH3: sdAb protein sequence, 118 aa (SEQ ID NO:35) 
QVQLVQSGAEVKKPGSSVKVSCKASGGTFSNYAISWVRQAPGQGLEWMGWISAYNGDTKYAQRL
QGRVTMTTDTSTSTAYMELRNLRSDDTAVYYCARYESMSGQDIWGQGTLVTVSS
FHVH3VH1 sdAb nucleic acid sequence, 762 bp (SEQ ID NO: 36): 
CAGGTGCAGCTGGTGCAGTCTGGGGCTGAGGTGAAGAAGCCTGGGTCCTCGGTGAAGGTCTCC
TGCAAGGCTTCTGGAGGCACCTTCAGCAACTATGCTATCAGCTGGGTGCGACAGGCCCCTGGAC
AAGGGCTTGAGTGGATGGGATGGATCAGCGCCTACAATGGTGACACAAAATATGCACAGAGGCT
 
CCAGGGCAGAGTCACCATGACCACAGACACATCCACGAGCACAGCCTACATGGAGCTGAGGAA
CCTAAGATCTGACGACACGGCCGTGTATTACTGTGCGCGCTACGAATCTATGTCTGGTCAGGATAT
CTGGGGTCAAGGTACTCTGGTGACCGTCTCCTCAGGGGGGGGGGGCTCTGGGGGGGGTGGCTC
AGGTGGCGGTGGCTCTGAAGTTCAGCTGCTGGAAAGCGGTGGTGGTCTGGTTCAGCCTGGTGGT
AGCCTGCGTCTGAGCTGTGCAGCAAGCGGTTTTACCTTTAGCCATAGCGCCATGGGTTGGGTTCG
TCAGGCACCTGGTAAAGGTCTGGAATGGGTTAGCAGCATCTATGCCCGCGGCGGCTATACCTATT
ATGCAGATAGCGTTAAAGGTCGTTTTACCATTAGCCGTGATAACAGCAAAAATACCCTGTACCTG
CAGATGAATAGTCTGCGTGCAGAGGATACCGCAGTGTATTATTGTGCGCGCGGTTACCATCTGGA
ATACATGGTTTCTCAGGATGTTTGGGGTCAAGGTACTCTGGTGACCGTCTCCTCA
FHVH3VH1 sdAb protein sequence, 254 aa (SEQ ID NO: 37): 
QVQLVQSGAEVKKPGSSVKVSCKASGGTFSNYAISWVRQAPGQGLEWMGWISAYNGDTKYAQRL
QGRVTMTTDTSTSTAYMELRNLRSDDTAVYYCARYESMSGQDIWGQGTLVTVSSGGGGSGGGGSG
GGGSEVQLLESGGGLVQPGGSLRLSCAASGFTFSHSAMGWVRQAPGKGLEWVSSIYARGGYTYYA
DSVKGRFTISRDNSKNTLYLQMNSLRAEDTAVYYCARGYHLEYMVSQDVWGQGTLVTVSS
CDR sequence of FHVH1:
   The amino acid sequence of HCDR1 is GFTFSHSA (SEQ ID NO:38);
   The amino acid sequence of HCDR2 is IYARGGYT (SEQ ID NO:39);
   The amino acid sequence of HCDR3 is ARGYHLEYMVSQDV (SEQ ID NO:40);
CDR sequence of FHVH3:
   The amino acid sequence of HCDR1 is GGTFSNYA (SEQ ID NO:41);
   The amino acid sequence of HCDR2 is ISAYNGDT (SEQ ID NO:42);
   The amino acid sequence of HCDR3 is ARYESMSGQDI (SEQ ID NO:43);
   FHVH1VH3 CAR nucleic acid sequence (SEQ ID NO:44) 
ATGGCCCTGCCTGTGACAGCTCTGCTCCTCCCTCTGGCCCTGCTGCTCCATGCCGCCAGACCCGA
AGTTCAGCTGCTGGAAAGCGGTGGTCTGGTTCAGCCTGGTGGTAGCCTGCGTCTGAGCTGT
GCAGCAAGCGGTTTTACCTTTAGCCATAGCGCCATGGGTTGGGTTCGTCAGGCACCTGGTAAAGG
TCTGGAATGGGTTAGCAGCATCTATGCCCGCGGCGGCTATACCTATTATGCAGATAGCGTTAAAGG
TCGTTTTACCATTAGCCGTGATAACAGCAAAAATACCCTGTACCTGCAGATGAATAGTCTGCGTGC
AGAGGATACCGCAGTGTATTATTGTGCGCGCGGTTACCATCTGGAATACATGGTTTCTCAGGATGT
TTGGGGTCAAGGTACTCTGGTGACCGTCTCCTCAGGGGGGGGGGGCTCTGGGGGGGGTGGCTC
AGGTGGCGGTGGCTCTCAGGTGCAGCTGGTGCAGTCTGGGGCTGAGGTGAAGAAGCCTGGGTC
CTCGGTGAAGGTCTCCTGCAAGGCTTCTGGAGGCACCTTCAGCAACTATGCTATCAGCTGGGTGC
GACAGGCCCCTGGACAAGGGCTTGAGTGGATGGGATGGATCAGCGCCTACAATGGTGACACAA
 
AATATGCACAGAGGCTCCAGGGCAGAGTCACCATGACCACAGACACATCCACGAGCACAGCCTA
CATGGAGCTGAGGAACCTAAGATCTGACGACACGGCCGTGTATTACTGTGCGCGCTACGAATCTA
TGTCTGGTCAGGATATCTGGGGTCAAGGTACTCTGGTGACCGTCTCCTCATTCGTGCCCGTGTTC
CTGCCCGCCAAACCTACTACTACCCCTGCACCTAGGCCTCCCACCCCAGCCCCAACAATCGCCAG
CCAGCCTCTGTCTCTGCGGCCCGAAGCCTGTAGACCTGCTGCCGGCGGAGCCGTGCACACCAGA
GGCCTGGACTTCGCCTGCGACATCTACATCTGGGCCCCTCTGGCCGGCACCTGTGGCGTGCTGCT
GCTGAGCCTGGTGATCACCCTGTACTGCAACCACCGGAACAAACGGGGCAGAAAGAAACTCCT
GTATATATTCAAACAACCATTTATGAGACCAGTACAAACTACTCAAGAGGAAGATGGCTGTAGCT
GCCGATTTCCAGAAGAAGAAGAAGGAGGATGTGAACTGAGAGTGAAGTTCAGCAGATCCGCCG
ACGCCCCTGCCTACCAGCAGGGACAGAACCAGCTGTACAACGAGCTGAACCTGGGCAGACGGG
AAGAGTACGACGTGCTGGACAAGCGGAGAGGCCGGGACCCCGAGATGGGCGGAAAGCCCAGA
CGGAAGAACCCCCAGGAAGGCCTGTATAACGAACTGCAGAAAGACAAGATGGCCGAGGCCTAC
AGCGAGATCGGCATGAAGGGCGAGCGGAGGCGCGGCAAGGGCCACGATGGCCTGTACCAGGGC
CTGAGCACCGCCACCAAGGACACCTACGACGCCCTGCACATGCAGGCCCTGCCCCCCAGAGGAT
CCGGAGAGGGAAGGGGCAGCTTATTAACATGTGGCGATGTGGAAGAGAACCCCGGTCCCATGCT
GCTGCTCGTGACCTCTTTACTGTTATGTGAGCTGCCCCACCCCGCTTTTTTACTGATCCCTCGTAA
GGTGTGTAACGGAATCGGCATTGGCGAGTTCAAGGACTCTTTAAGCATCAACGCCACAAACATC
AAGCACTTCAAGAATTGTACCTCCATCAGCGGCGATTTACACATTCTCCCCGTGGCTTTTCGTGG
CGATTCCTTCACCCACACCCCCCCTCTGGACCCCCAAGAGCTGGACATTTTAAAAACCGTGAAG
GAGATCACCGGCTTTCTGCTGATCCAAGCTTGGCCCGAGAATCGTACCGACCTCCACGCCTTCGA
GAATTTAGAGATTATTCGTGGAAGGACCAAGCAGCACGGCCAGTTCTCTTTAGCCGTCGTGTCTT
TAAACATTACCAGCCTCGGTTTAAGGTCTTTAAAGGAGATTAGCGACGGCGACGTGATCATCTCC
GGCAACAAGAACCTCTGCTACGCCAACACCATCAACTGGAAGAAGCTGTTCGGAACCAGCGGC
CAAAAGACCAAGATCATCAGCAATCGTGGAGAGAACTCTTGTAAGGCCACTGGTCAAGTTTGCC
ACGCCCTCTGTAGCCCCGAAGGATGTTGGGGCCCCGAGCCTAGGGACTGTGTTAGCTGCAGAAA
CGTGAGCAGAGGCAGAGAGTGTGTGGACAAATGCAATTTACTGGAAGGAGAGCCTAGGGAGTT
CGTGGAGAACAGCGAATGTATCCAGTGCCACCCCGAGTGTTTACCTCAAGCCATGAACATCACTT
GTACCGGAAGGGGCCCCGATAACTGCATCCAATGCGCCCACTACATCGACGGACCCCACTGCGT
GAAAACTTGTCCCGCCGGAGTGATGGGAGAGAATAACACTTTAGTGTGGAAGTACGCCGACGCT
GGCCACGTCTGCCATCTGTGCCACCCCAACTGTACCTACGGCTGCACTGGTCCCGGTTTAGAGGG
ATGTCCTACCAACGGCCCCAAGATCCCCTCCATCGCCACCGGAATGGTGGGCGCTCTGTTATTAC
TGCTGGTGGTGGCTCTGGGCATCGGTTTATTCATG
   FHVH1VH3 CAR amino acid sequence (SEQ ID NO:45) 
MALPVTALLLPLALLLHAARPEVQLLESGGGLVQPGGSLRLSCAASGFTFSHSAMGWVRQAPGKG
LEWVSSIYARGGYTYYADSVKGRFTISRDNSKNTLYLQMNSLRAEDTAVYYCARGYHLEYMVSQD
VWGQGTLVTVSSGGGGSGGGGSGGGGSQVQLVQSGAEVKKPGSSVKVSCKASGGTFSNYAISWV
RQAPGQGLEWMGWISAYNGDTKYAQRLQGRVTMTTDTSTSTAYMELRNLRSDDTAVYYCARYES
MSGQDIWGQGTLVTVSSFVPVFLPAKPTTTPAPRPPTPAPTIASQPLSLRPEACRPAAGGAVHTRGLD
FACDIYIWAPLAGTCGVLLLSLVITLYCNHRNKRGRKKLLYIFKQPFMRPVQTTQEEDGCSCRFPEE
EEGGCELRVKFSRSADAPAYQQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPRGSGEGRGSL
LTCGDVEENPGPMLLLVTSLLLCELPHPAFLLIPRKVCNGIGIGEFKDSLSINATNIKHFKNCTSISGDL
HILPVAFRGDSFTHTPPLDPQELDILKTVKEITGFLLIQAWPENRTDLHAFENLEIIRGRTKQHGQFSL
AVVSLNITSLGLRSLKEISDGDVIISGNKNLCYANTINWKKLFGTSGQKTKIISNRGENSCKATGQVC
HALCSPEGCWGPEPRDCVSCRNVSRGRECVDKCNLLEGEPREFVENSECIQCHPECLPQAMNITCTG
RGPDNCIQCAHYIDGPHCVKTCPAGVMGENNTLVWKYADAGHVCHLCHPNCTYGCTGPGLEGCP
TNGPKIPSIATGMVGALLLLLVVALGIGLFM
   FHVH1VH3 sdAb nucleic acid sequence (SEQ ID NO:46) 
GAAGTTCAGCTGCTGGAAAGCGGTGGTGGTCTGGTTCAGCCTGGTGGTAGCCTGCGTCTGAGCT
GTGCAGCAAGCGGTTTTACCTTTAGCCATAGCGCCATGGGTTGGGTTCGTCAGGCACCTGGTAA
AGGTCTGGAATGGGTTAGCAGCATCTATGCCCGCGGCGGCTATACCTATTATGCAGATAGCGTT
AAAGGTCGTTTTACCATTAGCCGTGATAACAGCAAAAATACCCTGTACCTGCAGATGAATAGTC
TGCGTGCAGAGGATACCGCAGTGTATTATTGTGCGCGCGGTTACCATCTGGAATACATGGTTTC
TCAGGATGTTTGGGGTCAAGGTACTCTGGTGACCGTCTCCTCAGGGGGGGGGGGCTCTGGGGG
GGGTGGCTCAGGTGGCGGTGGCTCTCAGGTGCAGCTGGTGCAGTCTGGGGCTGAGGTGAAGAA
GCCTGGGTCCTCGGTGAAGGTCTCCTGCAAGGCTTCTGGAGGCACCTTCAGCAACTATGCTATC
AGCTGGGTGCGACAGGCCCCTGGACAAGGGCTTGAGTGGATGGGATGGATCAGCGCCTACAAT
GGTGACACAAAATATGCACAGAGGCTCCAGGGCAGAGTCACCATGACCACAGACACATCCACG
AGCACAGCCTACATGGAGCTGAGGAACCTAAGATCTGACGACACGGCCGTGTATTACTGTGCG
CGCTACGAATCTATGTCTGGTCAGGATATCTGGGGTCAAGGTACTCTGGTGACCGTCTCCTCA
   FHVH1VH3 sdAb amino acid sequence (SEQ ID NO:47) 
EVQLLESGGGLVQPGGSLRLSCAASGFTFSHSAMGWVRQAPGKGLEWVSSIYARGGYTYYADSVK
GRFTISRDNSKNTLYLQMNSLRAEDTAVYYCARGYHLEYMVSQDVWGQGTLVTVSSGGGGSGGG
GSGGGGSQVQLVQSGAEVKKPGSSVKVSCKASGGTFSNYAISWVRQAPGQGLEWMGWISAYNGD
TKYAQRLQGRVTMTTDTSTSTAYMELRNLRSDDTAVYYCARYESMSGQDIWGQGTLVTVSS
   FHVH2 CAR nucleic acid sequence (SEQ ID NO:48) 
ATGGCCCTGCCTGTGACAGCTCTGCTCCTCCCTCTGGCCCTGCTGCTCCATGCCGCCAGACCCCA
GGTGCAGCTGGTGGAGTCTGGGGGAGGCTTGGTACAGCCTGGAGGGTCCCTGAGACTCTCCTGT
GCAGCCTCTGGATTCACCTTCAGTAGTTATGAAATGAACTGGGTCCGCCAGGCTCCAGGGAAGG
GGCTGGAGTGGGTTTCATACATTAGTAGTAGTGGTAGTACCATATACTACGCAGACTCTGTGAAG
GGCCGATTCACCATCTCCAGAGACAACGCCAAGAACTCACTGTATCTGCAAATGAACAGTCTGA
GAGCCGAGGACACGGCTGTTTATTACTGTGCGAGAGTCGCCCAGAGAGAAGGGGACGTCTGGG
GCAAAGGGACCACGGTCACCGTCTCCTCATTCGTGCCCGTGTTCCTGCCCGCCAAACCTACTACT
ACCCCTGCACCTAGGCCTCCCACCCCAGCCCCAACAATCGCCAGCCAGCCTCTGTCTCTGCGGC
CCGAAGCCTGTAGACCTGCTGCCGGCGGAGCCGTGCACACCAGAGGCCTGGACTTCGCCTGCG
ACATCTACATCTGGGCCCCTCTGGCCGGCACCTGTGGCGTGCTGCTGCTGAGCCTGGTGATCACC
CTGTACTGCAACCACCGGAACAAACGGGGCAGAAAGAAACTCCTGTATATATTCAAACAACCAT
TTATGAGACCAGTACAAACTACTCAAGAGGAAGATGGCTGTAGCTGCCGATTTCCAGAAGAAGA
AGAAGGAGGATGTGAACTGAGAGTGAAGTTCAGCAGATCCGCCGACGCCCCTGCCTACCAGCA
GGGACAGAACCAGCTGTACAACGAGCTGAACCTGGGCAGACGGGAAGAGTACGACGTGCTGGA
CAAGCGGAGAGGCCGGGACCCCGAGATGGGCGGAAAGCCCAGACGGAAGAACCCCCAGGAAG
GCCTGTATAACGAACTGCAGAAAGACAAGATGGCCGAGGCCTACAGCGAGATCGGCATGAAGG
GCGAGCGGAGGCGCGGCAAGGGCCACGATGGCCTGTACCAGGGCCTGAGCACCGCCACCAAGG
ACACCTACGACGCCCTGCACATGCAGGCCCTGCCCCCCAGAGGATCCGGAGAGGGAAGGGGCA
GCTTATTAACATGTGGCGATGTGGAAGAGAACCCCGGTCCCATGCTGCTGCTCGTGACCTCTTTA
CTGTTATGTGAGCTGCCCCACCCCGCTTTTTTACTGATCCCTCGTAAGGTGTGTAACGGAATCGGC
ATTGGCGAGTTCAAGGACTCTTTAAGCATCAACGCCACAAACATCAAGCACTTCAAGAATTGTAC
CTCCATCAGCGGCGATTTACACATTCTCCCCGTGGCTTTTCGTGGCGATTCCTTCACCCACACCCC
CCCTCTGGACCCCCAAGAGCTGGACATTTTAAAAACCGTGAAGGAGATCACCGGCTTTCTGCTG
ATCCAAGCTTGGCCCGAGAATCGTACCGACCTCCACGCCTTCGAGAATTTAGAGATTATTCGTGG
AAGGACCAAGCAGCACGGCCAGTTCTCTTTAGCCGTCGTGTCTTTAAACATTACCAGCCTCGGTT
TAAGGTCTTTAAAGGAGATTAGCGACGGCGACGTGATCATCTCCGGCAACAAGAACCTCTGCTA
CGCCAACACCATCAACTGGAAGAAGCTGTTCGGAACCAGCGGCCAAAAGACCAAGATCATCAG
CAATCGTGGAGAGAACTCTTGTAAGGCCACTGGTCAAGTTTGCCACGCCCTCTGTAGCCCCGAA
GGATGTTGGGGCCCCGAGCCTAGGGACTGTGTTAGCTGCAGAAACGTGAGCAGAGGCAGAGAG
TGTGTGGACAAATGCAATTTACTGGAAGGAGAGCCTAGGGAGTTCGTGGAGAACAGCGAATGTA
TCCAGTGCCACCCCGAGTGTTTACCTCAAGCCATGAACATCACTTGTACCGGAAGGGGCCCCGAT
AACTGCATCCAATGCGCCCACTACATCGACGGACCCCACTGCGTGAAAACTTGTCCCGCCGGAG
TGATGGGAGAGAATAACACTTTAGTGTGGAAGTACGCCGACGCTGGCCACGTCTGCCATCTGTG
CCACCCCAACTGTACCTACGGCTGCACTGGTCCCGGTTTAGAGGGATGTCCTACCAACGGCCCCA

AGATCCCCTCCATCGCCACCGGAATGGTGGGCGCTCTGTTATTACTGCTGGTGGTGGCTCTGGGC
ATCGGTTTATTCATG
   FHVH2 CAR protein sequence (SEQ ID NO:49) 
MALPVTALLLPLALLLHAARPQVQLVESGGGLVQPGGSLRLSCAASGFTFSSYEMNWVRQAPGKG
LEWVSYISSSGSTIYYADSVKGRFTISRDNAKNSLYLQMNSLRAEDTAVYYCARVAQREGDVWGK
GTTVTVSSFVPVFLPAKPTTTPAPRPPTPAPTIASQPLSLRPEACRPAAGGAVHTRGLDFACDIYIWAP
LAGTCGVLLLSLVITLYCNHRNKRGRKKLLYIFKQPFMRPVQTTQEEDGCSCRFPEEEEGGCELRVK
FSRSADAPAYQQGQNQLYNELNLGRREEYDVLDKRRGRDPEMGGKPRRKNPQEGLYNELQKDKM
AEAYSEIGMKGERRRGKGHDGLYQGLSTATKDTYDALHMQALPPRGSGEGRGSLLTCGDVEENPG
PMLLLVTSLLLCELPHPAFLLIPRKVCNGIGIGEFKDSLSINATNIKHFKNCTSISGDLHILPVAFRGDS
FTHTPPLDPQELDILKTVKEITGFLLIQAWPENRTDLHAFENLEIIRGRTKQHGQFSLAVVSLNITSLG
LRSLKEISDGDVIISGNKNLCYANTINWKKLFGTSGQKTKIISNRGENSCKATGQVCHALCSPEGCW
GPEPRDCVSCRNVSRGRECVDKCNLLEGEPREFVENSECIQCHPECLPQAMNITCTGRGPDNCIQCA
HYIDGPHCVKTCPAGVMGENNTLVWKYADAGHVCHLCHPNCTYGCTGPGLEGCPTNGPKIPSIAT
GMVGALLLLLVVALGIGLFM
   FHVH4 CAR nucleic acid sequence (SEQ ID NO:50) 
ATGGCCCTGCCTGTGACAGCTCTGCTCCTCCCTCTGGCCCTGCTGCTCCATGCCGCCAGACCCCA
GGTCCAGCTGGTGCAGTCTGGGGCTGAGGTGAAAAAGCCCGGGGAGTCTCTGAGGATCTCCTG
TAAGGCTTCTGGATACAGTTTTAGCAACCATTGGATCGGCTGGGTGCGCCAGAAGCCCGGGAA
AGGCCTGGAGTGGATGGGGAGCGTCTATCCTGGTGACTCCGATACCAGATACAGCCCGTCCTTC
CAAGGCCACGTCACTGTCTCAGCCGACAAGTCCATGAATACCGCCTACCTGCAGTGGAGCAGC
CTGAAGACCTCGGACACCGCCATGTATTATTGTGCGAGAGGTGGGACTATTGACGGTGACTACG
GGGGGAGGCAAGACTTCTGGGGCCAGGGAACCATGGTCACCGTCTCTTCATTCGTGCCCGTGTT
CCTGCCCGCCAAACCTACTACTACCCCTGCACCTAGGCCTCCCACCCCAGCCCCAACAATCGCC
AGCCAGCCTCTGTCTCTGCGGCCCGAAGCCTGTAGACCTGCTGCCGGCGGAGCCGTGCACACCA
GAGGCCTGGACTTCGCCTGCGACATCTACATCTGGGCCCCTCTGGCCGGCACCTGTGGCGTGCT
GCTGCTGAGCCTGGTGATCACCCTGTACTGCAACCACCGGAACAAACGGGGCAGAAAGAAACT
CCTGTATATATTCAAACAACCATTTATGAGACCAGTACAAACTACTCAAGAGGAAGATGGCTGT
AGCTGCCGATTTCCAGAAGAAGAAGAAGGAGGATGTGAACTGAGAGTGAAGTTCAGCAGATCC
GCCGACGCCCCTGCCTACCAGCAGGGACAGAACCAGCTGTACAACGAGCTGAACCTGGGCAGA
CGGGAAGAGTACGACGTGCTGGACAAGCGGAGAGGCCGGGACCCCGAGATGGGCGGAAAGCC
CAGACGGAAGAACCCCCAGGAAGGCCTGTATAACGAACTGCAGAAAGACAAGATGGCCGAGG
CCTACAGCGAGATCGGCATGAAGGGCGAGCGGAGGCGCGGCAAGGGCCACGATGGCCTGTAC
CAGGGCCTGAGCACCGCCACCAAGGACACCTACGACGCCCTGCACATGCAGGCCCTGCCCCCC
 
AGAGGATCCGGAGAGGGAAGGGGCAGCTTATTAACATGTGGCGATGTGGAAGAGAACCCCGG
TCCCATGCTGCTGCTCGTGACCTCTTTACTGTTATGTGAGCTGCCCCACCCCGCTTTTTTACTGAT
CCCTCGTAAGGTGTGTAACGGAATCGGCATTGGCGAGTTCAAGGACTCTTTAAGCATCAACGCC
ACAAACATCAAGCACTTCAAGAATTGTACCTCCATCAGCGGCGATTTACACATTCTCCCCGTGG
CTTTTCGTGGCGATTCCTTCACCCACACCCCCCCTCTGGACCCCCAAGAGCTGGACATTTTAAAA
ACCGTGAAGGAGATCACCGGCTTTCTGCTGATCCAAGCTTGGCCCGAGAATCGTACCGACCTCC
ACGCCTTCGAGAATTTAGAGATTATTCGTGGAAGGACCAAGCAGCACGGCCAGTTCTCTTTAGC
CGTCGTGTCTTTAAACATTACCAGCCTCGGTTTAAGGTCTTTAAAGGAGATTAGCGACGGCGAC
GTGATCATCTCCGGCAACAAGAACCTCTGCTACGCCAACACCATCAACTGGAAGAAGCTGTTCG
GAACCAGCGGCCAAAAGACCAAGATCATCAGCAATCGTGGAGAGAACTCTTGTAAGGCCACTG
GTCAAGTTTGCCACGCCCTCTGTAGCCCCGAAGGATGTTGGGGCCCCGAGCCTAGGGACTGTGT
TAGCTGCAGAAACGTGAGCAGAGGCAGAGAGTGTGTGGACAAATGCAATTTACTGGAAGGAG
AGCCTAGGGAGTTCGTGGAGAACAGCGAATGTATCCAGTGCCACCCCGAGTGTTTACCTCAAG
CCATGAACATCACTTGTACCGGAAGGGGCCCCGATAACTGCATCCAATGCGCCCACTACATCGA
CGGACCCCACTGCGTGAAAACTTGTCCCGCCGGAGTGATGGGAGAGAATAACACTTTAGTGTG
GAAGTACGCCGACGCTGGCCACGTCTGCCATCTGTGCCACCCCAACTGTACCTACGGCTGCACT
GGTCCCGGTTTAGAGGGATGTCCTACCAACGGCCCCAAGATCCCCTCCATCGCCACCGGAATGG
TGGGCGCTCTGTTATTACTGCTGGTGGTGGCTCTGGGCATCGGTTTATTCATG
   FHVH4 CAR protein sequence (SEQ ID NO:51) 
MALPVTALLLPLALLLHAARPQVQLVQSGAEVKKPGESLRISCKASGYSFSNHWIGWVRQKPGKGL
EWMGSVYPGDSDTRYSPSFQGHVTVSADKSMNTAYLQWSSLKTSDTAMYYCARGGTIDGDYGGR
QDFWGQGTMVTVSSFVPVFLPAKPTTTPAPRPPTPAPTIASQPLSLRPEACRPAAGGAVHTRGLDFA
CDIYIWAPLAGTCGVLLLSLVITLYCNHRNKRGRKKLLYIFKQPFMRPVQTTQEEDGCSCRFPEEEE
GGCELRVKFSRSADAPAYQQGQNQLYNELNLGRREEYDVLDKRRGRDPEMGGKPRRKNPQEGLY
NELQKDKMAEAYSEIGMKGERRRGKGHDGLYQGLSTATKDTYDALHMQALPPRGSGEGRGSLLT
CGDVEENPGPMLLLVTSLLLCELPHPAFLLIPRKVCNGIGIGEFKDSLSINATNIKHFKNCTSISGDLHI
LPVAFRGDSFTHTPPLDPQELDILKTVKEITGFLLIQAWPENRTDLHAFENLEIIRGRTKQHGQFSLAV
VSLNITSLGLRSLKEISDGDVIISGNKNLCYANTINWKKLFGTSGQKTKIISNRGENSCKATGQVCHA
LCSPEGCWGPEPRDCVSCRNVSRGRECVDKCNLLEGEPREFVENSECIQCHPECLPQAMNITCTGRG
PDNCIQCAHYIDGPHCVKTCPAGVMGENNTLVWKYADAGHVCHLCHPNCTYGCTGPGLEGCPTN
GPKIPSIATGMVGALLLLLVVALGIGLFM
   FHVH2VH1 CAR nucleic acid sequence (SEQ ID NO:52) 
ATGGCCCTACCTGTGACAGCCCTACTGTTACCCCTGGCCCTCCTTCTGCATGCTGCTAGACCTCA
GGTGCAGCTGGTGGAGTCTGGGGGAGGCTTGGTACAGCCTGGAGGGTCCCTGAGACTCTCCTGT
GCAGCCTCTGGATTCACCTTCAGTAGTTATGAAATGAACTGGGTCCGCCAGGCTCCAGGGAAGG
GGCTGGAGTGGGTTTCATACATTAGTAGTAGTGGTAGTACCATATACTACGCAGACTCTGTGAA
GGGCCGATTCACCATCTCCAGAGACAACGCCAAGAACTCACTGTATCTGCAAATGAACAGTCT
GAGAGCCGAGGACACGGCTGTTTATTACTGTGCGAGAGTCGCCCAGAGAGAAGGGGACGTCTG
GGGCAAAGGGACCACGGTCACCGTCTCCTCAGGGGGGGGGGGCTCTGGGGGGGGTGGCTCAGG
TGGCGGTGGCTCTGAAGTTCAGCTGCTGGAAAGCGGTGGTGGTCTGGTTCAGCCTGGTGGTAGC
CTGCGTCTGAGCTGTGCAGCAAGCGGTTTTACCTTTAGCCATAGCGCCATGGGTTGGGTTCGTC
AGGCACCTGGTAAAGGTCTGGAATGGGTTAGCAGCATCTATGCCCGCGGCGGCTATACCTATTA
TGCAGATAGCGTTAAAGGTCGTTTTACCATTAGCCGTGATAACAGCAAAAATACCCTGTACCTG
CAGATGAATAGTCTGCGTGCAGAGGATACCGCAGTGTATTATTGTGCGCGCGGTTACCATCTGG
AATACATGGTTTCTCAGGATGTTTGGGGTCAAGGTACTCTGGTGACCGTCTCCTCATTTGTGCCT
GTATTTCTGCCTGCCAAGCCCACCACAACACCTGCCCCTAGACCACCCACCCCTGCCCCCACCA
TTGCTTCTCAGCCCCTTAGCTTAAGACCTGAAGCCTGTAGACCTGCTGCTGGGGGGGCTGTGCA
CACAAGAGGCCTGGACTTTGCCTGTGACATCTACATCTGGGCCCCCCTGGCTGGCACCTGTGGG
GTGCTGCTGCTGAGCCTGGTGATCACCCTGTACTGCAACCACAGAAACAAGAGAGGCAGAAAG
AAGCTGCTGTACATCTTCAAGCAGCCCTTCATGAGACCTGTGCAGACCACCCAAGAGGAGGAT
GGCTGCAGCTGCAGATTCCCTGAGGAGGAGGAGGGGGGCTGTGAGCTGAGAGTGAAGTTCAGC
AGATCTGCTGATGCCCCTGCCTATCAGCAAGGGCAGAATCAGCTGTACAATGAGCTGAATCTGG
GCAGAAGAGAGGAGTATGATGTGCTGGACAAGAGAAGAGGCAGAGACCCTGAGATGGGGGGC
AAGCCTAGAAGAAAGAACCCCCAAGAGGGCCTGTATAATGAGCTGCAGAAGGACAAGATGGC
TGAGGCCTACTCTGAGATTGGCATGAAGGGGGAGAGAAGAAGAGGCAAGGGCCATGATGGCC
TGTACCAAGGCCTGAGCACAGCCACCAAGGACACCTATGATGCCCTACACATGCAAGCTCTGC
CTCCTAGAGGCTCTGGGGAAGGAAGGGGCAGCCTACTGACCTGTGGGGATGTGGAGGAGAACC
CTGGCCCCATGTTGCTATTAGTAACCAGCCTGCTGCTGTGTGAGCTGCCCCACCCTGCCTTCCTG
TTAATCCCACGAAAGGTATGTAATGGCATTGGCATTGGGGAGTTTAAGGACAGCCTGAGCATC
AATGCCACCAACATCAAGCACTTCAAGAACTGCACAAGCATCAGTGGGGACTTGCACATCCTG
CCTGTGGCCTTCAGAGGGGACAGCTTCACCCACACCCCCCCCCTGGACCCCCAAGAGCTGGACA
TCCTGAAGACAGTGAAGGAGATCACTGGCTTCTTGCTGATCCAAGCCTGGCCTGAGAACAGAA
CAGACCTGCATGCCTTTGAGAACCTGGAGATCATCAGAGGCAGAACCAAGCAGCATGGGCAGT
TCAGCCTGGCTGTGGTGAGCCTGAACATCACAAGCCTGGGCCTGAGAAGCTTAAAGGAGATCT
CTGATGGGGATGTGATCATCTCTGGCAACAAGAACCTGTGCTATGCCAACACCATCAACTGGAA
GAAGCTGTTTGGCACCTCTGGGCAGAAGACCAAGATCATCAGCAACAGAGGGGAGAACTCCTG
TAAGGCCACTGGCCAAGTGTGTCATGCCCTATGCAGCCCTGAGGGGTGCTGGGGCCCTGAGCCT
 
AGAGACTGTGTGAGCTGCAGAAATGTGAGCAGAGGCAGAGAGTGTGTGGACAAGTGCAACCTG
CTGGAGGGGGAGCCTAGAGAGTTTGTGGAGAACTCTGAGTGTATTCAGTGTCATCCTGAGTGCC
TGCCCCAAGCCATGAACATCACCTGCACTGGCAGAGGCCCTGACAACTGCATTCAGTGTGCCCA
CTACATTGATGGCCCCCACTGTGTGAAGACCTGCCCTGCTGGGGTGATGGGGGAGAACAACAC
CCTGGTGTGGAAGTATGCTGATGCTGGCCATGTGTGTCACCTGTGCCATCCCAACTGCACCTAT
GGCTGCACTGGCCCTGGCCTGGAGGGCTGCCCCACCAATGGTCCCAAGATTCCTAGCATTGCCA
CTGGCATGGTGGGGGCCCTGCTCCTACTTCTGGTGGTTGCCCTGGGCATTGGCCTGTTCATG
   FHVH2VH1 CAR protein sequence (SEQ ID NO:53) 
MALPVTALLLPLALLLHAARPQVQLVESGGGLVQPGGSLRLSCAASGFTFSSYEMNWVRQAPGKG
LEWVSYISSSGSTIYYADSVKGRFTISRDNAKNSLYLQMNSLRAEDTAVYYCARVAQREGDVWGK
GTTVTVSSGGGGSGGGGSGGGGSEVQLLESGGGLVQPGGSLRLSCAASGFTFSHSAMGWVRQAPG
KGLEWVSSIYARGGYTYYADSVKGRFTISRDNSKNTLYLQMNSLRAEDTAVYYCARGYHLEYMVS
QDVWGQGTLVTVSSFVPVFLPAKPTTTPAPRPPTPAPTIASQPLSLRPEACRPAAGGAVHTRGLDFA
CDIYIWAPLAGTCGVLLLSLVITLYCNHRNKRGRKKLLYIFKQPFMRPVQTTQEEDGCSCRFPEEEE
GGCELRVKFSRSADAPAYQQGQNQLYNELNLGRREEYDVLDKRRGRDPEMGGKPRRKNPQEGLY
NELQKDKMAEAYSEIGMKGERRRGKGHDGLYQGLSTATKDTYDALHMQALPPRGSGEGRGSLLT
CGDVEENPGPMLLLVTSLLLCELPHPAFLLIPRKVCNGIGIGEFKDSLSINATNIKHFKNCTSISGDLHI
LPVAFRGDSFTHTPPLDPQELDILKTVKEITGFLLIQAWPENRTDLHAFENLEIIRGRTKQHGQFSLAV
VSLNITSLGLRSLKEISDGDVIISGNKNLCYANTINWKKLFGTSGQKTKIISNRGENSCKATGQVCHA
LCSPEGCWGPEPRDCVSCRNVSRGRECVDKCNLLEGEPREFVENSECIQCHPECLPQAMNITCTGRG
PDNCIQCAHYIDGPHCVKTCPAGVMGENNTLVWKYADAGHVCHLCHPNCTYGCTGPGLEGCPTN
GPKIPSIATGMVGALLLLLVVALGIGLFM
   FHVH4VH1 CAR nucleic acid sequence (SEQ ID NO:54) 
ATGGCCCTACCTGTGACAGCCCTACTGTTACCCCTGGCCCTCCTTCTGCATGCTGCTAGACCTCA
GGTCCAGCTGGTGCAGTCTGGGGCTGAGGTGAAAAAGCCCGGGGAGTCTCTGAGGATCTCCTG
TAAGGCTTCTGGATACAGTTTTAGCAACCATTGGATCGGCTGGGTGCGCCAGAAGCCCGGGAA
AGGCCTGGAGTGGATGGGGAGCGTCTATCCTGGTGACTCCGATACCAGATACAGCCCGTCCTTC
CAAGGCCACGTCACTGTCTCAGCCGACAAGTCCATGAATACCGCCTACCTGCAGTGGAGCAGC
CTGAAGACCTCGGACACCGCCATGTATTATTGTGCGAGAGGTGGGACTATTGACGGTGACTACG
GGGGGAGGCAAGACTTCTGGGGCCAGGGAACCATGGTCACCGTCTCTTCAGGGGGGGGGGGCT
CTGGGGGGGGTGGCTCAGGTGGCGGTGGCTCTGAAGTTCAGCTGCTGGAAAGCGGTGGTGGTC
TGGTTCAGCCTGGTGGTAGCCTGCGTCTGAGCTGTGCAGCAAGCGGTTTTACCTTTAGCCATAG
CGCCATGGGTTGGGTTCGTCAGGCACCTGGTAAAGGTCTGGAATGGGTTAGCAGCATCTATGCC
 
CGCGGCGGCTATACCTATTATGCAGATAGCGTTAAAGGTCGTTTTACCATTAGCCGTGATAACA
GCAAAAATACCCTGTACCTGCAGATGAATAGTCTGCGTGCAGAGGATACCGCAGTGTATTATTG
TGCGCGCGGTTACCATCTGGAATACATGGTTTCTCAGGATGTTTGGGGTCAAGGTACTCTGGTG
ACCGTCTCCTCATTTGTGCCTGTATTTCTGCCTGCCAAGCCCACCACAACACCTGCCCCTAGACC
ACCCACCCCTGCCCCCACCATTGCTTCTCAGCCCCTTAGCTTAAGACCTGAAGCCTGTAGACCT
GCTGCTGGGGGGGCTGTGCACACAAGAGGCCTGGACTTTGCCTGTGACATCTACATCTGGGCCC
CCCTGGCTGGCACCTGTGGGGTGCTGCTGCTGAGCCTGGTGATCACCCTGTACTGCAACCACAG
AAACAAGAGAGGCAGAAAGAAGCTGCTGTACATCTTCAAGCAGCCCTTCATGAGACCTGTGCA
GACCACCCAAGAGGAGGATGGCTGCAGCTGCAGATTCCCTGAGGAGGAGGAGGGGGGCTGTG
AGCTGAGAGTGAAGTTCAGCAGATCTGCTGATGCCCCTGCCTATCAGCAAGGGCAGAATCAGC
TGTACAATGAGCTGAATCTGGGCAGAAGAGAGGAGTATGATGTGCTGGACAAGAGAAGAGGC
AGAGACCCTGAGATGGGGGGCAAGCCTAGAAGAAAGAACCCCCAAGAGGGCCTGTATAATGA
GCTGCAGAAGGACAAGATGGCTGAGGCCTACTCTGAGATTGGCATGAAGGGGGAGAGAAGAA
GAGGCAAGGGCCATGATGGCCTGTACCAAGGCCTGAGCACAGCCACCAAGGACACCTATGATG
CCCTACACATGCAAGCTCTGCCTCCTAGAGGCTCTGGGGAAGGAAGGGGCAGCCTACTGACCT
GTGGGGATGTGGAGGAGAACCCTGGCCCCATGTTGCTATTAGTAACCAGCCTGCTGCTGTGTGA
GCTGCCCCACCCTGCCTTCCTGTTAATCCCACGAAAGGTATGTAATGGCATTGGCATTGGGGAG
TTTAAGGACAGCCTGAGCATCAATGCCACCAACATCAAGCACTTCAAGAACTGCACAAGCATC
AGTGGGGACTTGCACATCCTGCCTGTGGCCTTCAGAGGGGACAGCTTCACCCACACCCCCCCCC
TGGACCCCCAAGAGCTGGACATCCTGAAGACAGTGAAGGAGATCACTGGCTTCTTGCTGATCC
AAGCCTGGCCTGAGAACAGAACAGACCTGCATGCCTTTGAGAACCTGGAGATCATCAGAGGCA
GAACCAAGCAGCATGGGCAGTTCAGCCTGGCTGTGGTGAGCCTGAACATCACAAGCCTGGGCC
TGAGAAGCTTAAAGGAGATCTCTGATGGGGATGTGATCATCTCTGGCAACAAGAACCTGTGCT
ATGCCAACACCATCAACTGGAAGAAGCTGTTTGGCACCTCTGGGCAGAAGACCAAGATCATCA
GCAACAGAGGGGAGAACTCCTGTAAGGCCACTGGCCAAGTGTGTCATGCCCTATGCAGCCCTG
AGGGGTGCTGGGGCCCTGAGCCTAGAGACTGTGTGAGCTGCAGAAATGTGAGCAGAGGCAGAG
AGTGTGTGGACAAGTGCAACCTGCTGGAGGGGGAGCCTAGAGAGTTTGTGGAGAACTCTGAGT
GTATTCAGTGTCATCCTGAGTGCCTGCCCCAAGCCATGAACATCACCTGCACTGGCAGAGGCCC
TGACAACTGCATTCAGTGTGCCCACTACATTGATGGCCCCCACTGTGTGAAGACCTGCCCTGCT
GGGGTGATGGGGGAGAACAACACCCTGGTGTGGAAGTATGCTGATGCTGGCCATGTGTGTCAC
CTGTGCCATCCCAACTGCACCTATGGCTGCACTGGCCCTGGCCTGGAGGGCTGCCCCACCAATG
GTCCCAAGATTCCTAGCATTGCCACTGGCATGGTGGGGGCCCTGCTCCTACTTCTGGTGGTTGC
CCTGGGCATTGGCCTGTTCATG
   FHVH4VH1 CAR protein sequence (SEQ ID NO:55) 
MALPVTALLLPLALLLHAARPQVQLVQSGAEVKKPGESLRISCKASGYSFSNHWIGWVRQKPGKGL
EWMGSVYPGDSDTRYSPSFQGHVTVSADKSMNTAYLQWSSLKTSDTAMYYCARGGTIDGDYGGR
QDFWGQGTMVTVSSGGGGSGGGGSGGGGSEVQLLESGGGLVQPGGSLRLSCAASGFTFSHSAMG
WVRQAPGKGLEWVSSIYARGGYTYYADSVKGRFTISRDNSKNTLYLQMNSLRAEDTAVYYCARG
YHLEYMVSQDVWGQGTLVTVSSFVPVFLPAKPTTTPAPRPPTPAPTIASQPLSLRPEACRPAAGGAV
HTRGLDFACDIYIWAPLAGTCGVLLLSLVITLYCNHRNKRGRKKLLYIFKQPFMRPVQTTQEEDGCS
CRFPEEEEGGCELRVKFSRSADAPAYQQGQNQLYNELNLGRREEYDVLDKRRGRDPEMGGKPRRK
NPQEGLYNELQKDKMAEAYSEIGMKGERRRGKGHDGLYQGLSTATKDTYDALHMQALPPRGSGE
GRGSLLTCGDVEENPGPMLLLVTSLLLCELPHPAFLLIPRKVCNGIGIGEFKDSLSINATNIKHFKNCT
SISGDLHILPVAFRGDSFTHTPPLDPQELDILKTVKEITGFLLIQAWPENRTDLHAFENLEIIRGRTKQH
GQFSLAVVSLNITSLGLRSLKEISDGDVIISGNKNLCYANTINWKKLFGTSGQKTKIISNRGENSCKAT
GQVCHALCSPEGCWGPEPRDCVSCRNVSRGRECVDKCNLLEGEPREFVENSECIQCHPECLPQAMN
ITCTGRGPDNCIQCAHYIDGPHCVKTCPAGVMGENNTLVWKYADAGHVCHLCHPNCTYGCTGPGL
EGCPTNGPKIPSIATGMVGALLLLLVVALGIGLFM
   FHVH2 sdAb nucleic acid sequence (SEQ ID NO:56) 
CAGGTGCAGCTGGTGGAGTCTGGGGGAGGCTTGGTACAGCCTGGAGGGTCCCTGAGACTCTCC
TGTGCAGCCTCTGGATTCACCTTCAGTAGTTATGAAATGAACTGGGTCCGCCAGGCTCCAGGGA
AGGGGCTGGAGTGGGTTTCATACATTAGTAGTAGTGGTAGTACCATATACTACGCAGACTCTGT
GAAGGGCCGATTCACCATCTCCAGAGACAACGCCAAGAACTCACTGTATCTGCAAATGAACAG
TCTGAGAGCCGAGGACACGGCTGTTTATTACTGTGCGAGAGTCGCCCAGAGAGAAGGGGACGT
CTGGGGCAAAGGGACCACGGTCACCGTCTCCTCA
   FHVH2 sdAb protein sequence (SEQ ID NO:57) 
QVQLVESGGGLVQPGGSLRLSCAASGFTFSSYEMNWVRQAPGKGLEWVSYISSSGSTIYYADSVKG
RFTISRDNAKNSLYLQMNSLRAEDTAVYYCARVAQREGDVWGKGTTVTVSS
   FHVH4 sdAb nucleic acid sequence (SEQ ID NO:58) 
CAGGTCCAGCTGGTGCAGTCTGGGGCTGAGGTGAAAAAGCCCGGGGAGTCTCTGAGGATCTCC
TGTAAGGCTTCTGGATACAGTTTTAGCAACCATTGGATCGGCTGGGTGCGCCAGAAGCCCGGGA
AAGGCCTGGAGTGGATGGGGAGCGTCTATCCTGGTGACTCCGATACCAGATACAGCCCGTCCTT
CCAAGGCCACGTCACTGTCTCAGCCGACAAGTCCATGAATACCGCCTACCTGCAGTGGAGCAG
CCTGAAGACCTCGGACACCGCCATGTATTATTGTGCGAGAGGTGGGACTATTGACGGTGACTAC
GGGGGGAGGCAAGACTTCTGGGGCCAGGGAACCATGGTCACCGTCTCTTCA
   FHVH4 sdAb protein sequence (SEQ ID NO:59) 
QVQLVQSGAEVKKPGESLRISCKASGYSFSNHWIGWVRQKPGKGLEWMGSVYPGDSDTRYSPSFQ
GHVTVSADKSMNTAYLQWSSLKTSDTAMYYCARGGTIDGDYGGRQDFWGQGTMVTVSS
   FHVH2VH1 sdAb nucleic acid sequence (SEQ ID NO:60) 
CAGGTGCAGCTGGTGGAGTCTGGGGGAGGCTTGGTACAGCCTGGAGGGTCCCTGAGACTCTCC
TGTGCAGCCTCTGGATTCACCTTCAGTAGTTATGAAATGAACTGGGTCCGCCAGGCTCCAGGGA
AGGGGCTGGAGTGGGTTTCATACATTAGTAGTAGTGGTAGTACCATATACTACGCAGACTCTGT
GAAGGGCCGATTCACCATCTCCAGAGACAACGCCAAGAACTCACTGTATCTGCAAATGAACAG
TCTGAGAGCCGAGGACACGGCTGTTTATTACTGTGCGAGAGTCGCCCAGAGAGAAGGGGACGT
CTGGGGCAAAGGGACCACGGTCACCGTCTCCTCAGGGGGGGGGGGCTCTGGGGGGGGTGGCTC
AGGTGGCGGTGGCTCTGAAGTTCAGCTGCTGGAAAGCGGTGGTGGTCTGGTTCAGCCTGGTGGT
AGCCTGCGTCTGAGCTGTGCAGCAAGCGGTTTTACCTTTAGCCATAGCGCCATGGGTTGGGTTC
GTCAGGCACCTGGTAAAGGTCTGGAATGGGTTAGCAGCATCTATGCCCGCGGCGGCTATACCTA
TTATGCAGATAGCGTTAAAGGTCGTTTTACCATTAGCCGTGATAACAGCAAAAATACCCTGTAC
CTGCAGATGAATAGTCTGCGTGCAGAGGATACCGCAGTGTATTATTGTGCGCGCGGTTACCATC
TGGAATACATGGTTTCTCAGGATGTTTGGGGTCAAGGTACTCTGGTGACCGTCTCCTCA
   FHVH2VH1 sdAb protein sequence (SEQ ID NO:61) 
QVQLVESGGGLVQPGGSLRLSCAASGFTFSSYEMNWVRQAPGKGLEWVSYISSSGSTIYYADSVKG
RFTISRDNAKNSLYLQMNSLRAEDTAVYYCARVAQREGDVWGKGTTVTVSSGGGGSGGGGSGGG
GSEVQLLESGGGLVQPGGSLRLSCAASGFTFSHSAMGWVRQAPGKGLEWVSSIYARGGYTYYADS
VKGRFTISRDNSKNTLYLQMNSLRAEDTAVYYCARGYHLEYMVSQDVWGQGTLVTVSS
   FHVH4VH1 sdAb nucleic acid sequence (SEQ ID NO:62) 
CAGGTCCAGCTGGTGCAGTCTGGGGCTGAGGTGAAAAAGCCCGGGGAGTCTCTGAGGATCTCC
TGTAAGGCTTCTGGATACAGTTTTAGCAACCATTGGATCGGCTGGGTGCGCCAGAAGCCCGGGA
AAGGCCTGGAGTGGATGGGGAGCGTCTATCCTGGTGACTCCGATACCAGATACAGCCCGTCCTT
CCAAGGCCACGTCACTGTCTCAGCCGACAAGTCCATGAATACCGCCTACCTGCAGTGGAGCAG
CCTGAAGACCTCGGACACCGCCATGTATTATTGTGCGAGAGGTGGGACTATTGACGGTGACTACG
GGGGGAGGCAAGACTTCTGGGGCCAGGGAACCATGGTCACCGTCTCTTCAGGGGGGGGGGGC
TCTGGGGGGGGTGGCTCAGGTGGCGGTGGCTCTGAAGTTCAGCTGCTGGAAAGCGGTGGTGGT
CTGGTTCAGCCTGGTGGTAGCCTGCGTCTGAGCTGTGCAGCAAGCGGTTTTACCTTTAGCCATA
GCGCCATGGGTTGGGTTCGTCAGGCACCTGGTAAAGGTCTGGAATGGGTTAGCAGCATCTATGC
CCGCGGCGGCTATACCTATTATGCAGATAGCGTTAAAGGTCGTTTTACCATTAGCCGTGATAAC

AGCAAAAATACCCTGTACCTGCAGATGAATAGTCTGCGTGCAGAGGATACCGCAGTGTATTATT
GTGCGCGCGGTTACCATCTGGAATACATGGTTTCTCAGGATGTTTGGGGTCAAGGTACTCTGGT
GACCGTCTCCTCA
   FHVH4VH1 sdAb protein sequence (SEQ ID NO:63) 
QVQLVQSGAEVKKPGESLRISCKASGYSFSNHWIGWVRQKPGKGLEWMGSVYPGDSDTRYSPSFQ
GHVTVSADKSMNTAYLQWSSLKTSDTAMYYCARGGTIDGDYGGRQDFWGQGTMVTVSSGGGGS
GGGGSGGGGSEVQLLESGGGLVQPGGSLRLSCAASGFTFSHSAMGWVRQAPGKGLEWVSSIYARG
GYTYYADSVKGRFTISRDNSKNTLYLQMNSLRAEDTAVYYCARGYHLEYMVSQDVWGQGTLVTV
SS
   Amino acid sequence of FHVH2 HCDR1 (SEQ ID NO:64)
      GFTFSSYE
   Amino acid sequence of FHVH2 HCDR2 (SEQ ID NO:65)
      ISSSGSTI
   Amino acid sequence of FHVH2 HCDR3 (SEQ ID NO:66)
      ARVAQREGDV
   Amino acid sequence of FHVH4 HCDR1 (SEQ ID NO:67)
      GYSFSNHW
   Amino acid sequence of FHVH4 HCDR2 (SEQ ID NO:68)
      VYPGDSDT
   Amino acid sequence of FHVH4 HCDR3 (SEQ ID NO:69)
      ARGGTIDGDYGGRQDF
   CD5 KO sgRNA: GCTGTAGAACTCCACCACGC (SEQ ID NO:70)

## Claims

1. A chimeric antigen receptor (CAR), comprising a CD5 binding domain, a transmembrane domain, a co-stimulatory domain and an intracellular signaling domain, wherein the CD5 binding domain comprises one or more antibodies or fragments thereof specifically binding to CD5, wherein the antibody comprises a heavy chain complementarity determining region 1 (HCDR1), a heavy chain complementarity determining region 2 (HCDR2) and a heavy chain complementarity determining region 3 (HCDR3), and the amino acid sequences of the HCDR1, HCDR2, and HCDR3 are selected from any one of the following combinations:
(1) HCDR1 with an amino acid sequence as set forth in SEQ ID NO:38, HCDR2 withan amino acid sequence as set forth in SEQ ID NO:39, and HCDR3 with an amino acid sequence as set forth in SEQ ID NO:40;
(2) HCDR1 with an amino acid sequence as set forth in SEQ ID NO:41, HCDR2 with an amino acid sequence as set forth in SEQ ID NO:42, and HCDR3 with an amino acid sequence as set forth in SEQ ID NO:43;
(3) HCDR1 with an amino acid sequence as set forth in SEQ ID NO:64, HCDR2 with an amino acid sequence as set forth in SEQ ID NO:65, and HCDR3 with an amino acid sequence as set forth in SEQ ID NO:66;
(4) HCDR1 with an amino acid sequence as set forth in SEQ ID NO:67, HCDR2 with an amino acid sequence as set forth in SEQ ID NO:68, and HCDR3 with an amino acid sequence as set forth in SEQ ID NO:69.

2. The CAR according to claim 1, wherein the CD5 binding domain comprises at least two antibodies or fragments thereof that specifically bind to CD5, and the HCDR1, HCDR2, and HCDR3 comprised in the antibodies or fragments thereof are independently selected from any of the following combinations:
(1) HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40;
(2) HCDR1 with an amino acid sequence as set forth in SEQ ID NO:41, HCDR2 with an amino acid sequence as set forth in SEQ ID NO:42, and HCDR3 with an amino acid sequence as set forth in SEQ ID NO:43;
(3) HCDR1 with an amino acid sequence as set forth in SEQ ID NO:64, HCDR2 with an amino acid sequence as set forth in SEQ ID NO:65, and HCDR3 with an amino acid sequence as set forth in SEQ ID NO:66;
(4) HCDR1 with an amino acid sequence as set forth in SEQ ID NO:67, HCDR2 with an amino acid sequence as set forth in SEQ ID NO:68, and HCDR3 with an amino acid sequence as set forth in SEQ ID NO:69.

3. The CAR according to claim 2, wherein the CD5 binding domain comprises a first antibody or a fragment thereof and a second antibody or a fragment thereof that specifically bind to CD5, and the HCDR1, HCDR2, and HCDR3 comprised in the first antibody or fragment thereof and the second antibody or fragment thereof are selected from any of the following combinations:
(1) the first antibody or fragment thereof comprises HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40; the second antibody or fragment thereof comprises HCDR1 with an amino acid sequence as set forth in SEQ ID NO:41, HCDR2 with an amino acid sequence as set forth in SEQ ID NO:42, and HCDR3 with an amino acid sequence as set forth in SEQ ID NO:43;
(2) the first antibody or fragment thereof comprises HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40; the second antibody or fragment thereof comprises HCDR1 with an amino acid sequence as set forth in SEQ ID NO:64, HCDR2 with an amino acid sequence as set forth in SEQ ID NO:65, and HCDR3 with an amino acid sequence as set forth in SEQ ID NO:66;
(3) the first antibody or fragment thereof comprises HCDR1 with an amino acid sequence as set forth in SEQ ID NO:38, HCDR2 with an amino acid sequence as set forth in SEQ ID NO:39, and HCDR3 with an amino acid sequence as set forth in SEQ ID NO:40; the second antibody or fragment thereof comprises HCDR1 with an amino acid sequence as set forth in SEQ ID NO:67, HCDR2 with an amino acid sequence as set forth in SEQ ID NO:68, and HCDR3 with an amino acid sequence as set forth in SEQ ID NO:69.

4. The CAR according to claim 2 or 3, wherein the at least two antibodies or fragments thereof that specifically bind to CD5 are linked in tandem.

5. The CAR according to any one of claims 1-4, wherein the antibody comprises a heavy chain variable region VH, and the amino acid sequence of the heavy chain variable region is as set forth in SEQ ID NO: 33, 35, 37, 47, 57, 59, 61 or 63.

6. The CAR according to any one of claims 1-5, wherein the antibody is a single-domain antibody.

7. The CAR according to claim 6, wherein the CD5 binding domain comprises a plurality of single-domain antibodies, and the plurality of single-domain antibodies are linked by linker fragments.

8. The CAR according to claim 7, wherein the linker fragment comprises a linker fragment as set forth in SEQ ID NO:25.

9. The CAR according to any one of claims 1-8, wherein the antibody comprises an amino acid sequence as set forth in SEQ ID No: 33, 35, 37, 47, 57, 59, 61 or 63, or a functional variant thereof.

10. The CAR according to any one of claims 1-9, wherein the transmembrane domain comprises a polypeptide from a protein selected from: α, β or ζ chains of T cell receptors, CD28, CD3e, CD45, CD4, CD5, CD8α, CD9, CD16, CD22, CD33, CD37, CD64, CD80, CD86, CD134, CD137, and CD154.

11. The CAR according to any one of claims 1-10, wherein the transmembrane domain comprises an amino acid sequence as set forth in SEQ ID No: 6 or a functional variant thereof.

12. The CAR according to any one of claims 1-11, wherein the co-stimulatory domain comprises a polypeptide selected from the following proteins: CD28, 4-1BB, OX-40 and ICOS.

13. The CAR according to any one of claims 1-12, wherein the-co-stimulatory domain comprises an amino acid sequence as set forth in SEQ ID No: 8 or a functional variant thereof.

14. The CAR according to any one of claims 1-13, wherein the intracellular signaling domain comprises a signaling domain derived from CD3ζ.

15. The CAR according to any one of claims 1-14, wherein the intracellular signaling domain comprises an amino acid sequence as set forth in SEQ ID No: 10 or a functional variant thereof.

16. The CAR according to any one of claims 1-15, wherein the CAR further comprises a hinge region linking the CD5 binding domain and the transmembrane domain.

17. The CAR according to claim 16, wherein the hinge region comprises an amino acid sequence as set forth in SEQ ID No: 4 or a functional variant thereof.

18. The CAR according to any one of claims 1-17, wherein the CAR is further linked to a CD8α signal peptide.

19. The CAR according to claim 18, wherein the signal peptide comprises an amino acid sequence as set forth in SEQ ID No: 2 or a functional variant thereof.

20. The CAR according to any one of claims 1-19, wherein the CAR further comprises a cleaving peptide.

21. The CAR according to claim 20, wherein the cleaving peptide comprises an amino acid sequence derived from a T2A peptide.

22. The CAR according to any one of claims 20-21, wherein the cleaving peptide comprises an amino acid sequence as set forth in SEQ ID No: 12 or a functional variant thereof.

23. The CAR according to any one of claims 1-22, wherein the CAR is further linked to a truncated EGFR molecule (tEGFR) through the cleaving peptide and a CSF2RA signal peptide.

24. The CAR according to claim 23, wherein the CSF2RA signal peptide comprises an amino acid sequence as set forth in SEQ ID No: 14 or a functional variant thereof, and the truncated EGFR molecule comprises an amino acid sequence as set forth in SEQ ID No: 16 or a functional variant thereof.

25. The CAR according to any one of claims 1-24, comprising an amino acid sequence selected from SEQ ID No: 27, 29, 31, 45, 49, 51, 53 or 55 or a functional variant thereof.

26. An isolated nucleic acid molecule encoding the CAR according to any one of claims 1-25.

27. The isolated nucleic acid molecule encoding the CAR according to claim 26, comprising a nucleic acid sequence selected from SEQ ID No: 1, 3, 5, 7, 9, 11, 13, 15, 17-24, 26, 28, 30, 32, 34, 36, 44, 46, 48, 50, 52, 54, 56, 58, 60, or 62 or a functional variant thereof.

28. A vector comprising the nucleic acid molecule of any one of claims 26-27.

29. The vector according to claim 28, wherein the vector is selected from plasmid, retroviral vector and lentiviral vector.

30. An immune effector cell comprising the CAR according to any one of claims 1-25, the nucleic acid molecule according to any one of claims 26-27, or the vector according to any one of claims 28-29.

31. The cell according to claim 30, wherein CD5 is not expressed on the immune effector cell.

32. The cell according to any one of claims 30-31, wherein the immune effector cell is selected from T lymphocyte and natural killer (NK) cell.

33. A method for preparing an immune effector cell, comprising knocking out the CD5 gene of the immune effector cell, and introducing the nucleic acid molecule of claim 26 or 27 or the vector of claim 28 or 29 into the immune effector cell.

34. A pharmaceutical composition comprising the immune effector cell of any one of claims 30-32 and a pharmaceutically acceptable adjuvant.

35. Use of the CAR according to any one of claims 1-25, the nucleic acid molecule according to any one of claims 26-27, the vector according to any one of claims 28-29, or the immune effector cell according to any one of claims 30-32 in preparing a drug, wherein the drug is used for treating a disease or a condition associated with the expression of CD5.

36. A method for treating a disease or a condition associated with the expression of CD5, comprising administering a therapeutically effective amount of the immune effector cells according to any one of claims 30-32 or the pharmaceutical composition according to claim 34 to a patient in need thereof.

37. The method according to claim 36, further comprising further administering an anti-EGFR antibody to the patient in need thereof to inhibit the effect of the immune effector cells or the pharmaceutical composition; preferably, the anti-EGFR antibody is cetuximab.

38. The use according to claim 35 or the method according to any one of claims 36-37, wherein the disease or condition associated with the expression of CD5 is cancer or malignant tumor.

39. The use according to claim 35 or the method according to any one of claims 36-37, wherein the disease or condition associated with the expression of CD5 is T lymphoblastic lymphoma or mantle cell lymphoma.
